(19) Europäisches Patentamt
European Patent Office
Office européen des brevets

(11) EP 1 546 073 B1

(12) **EUROPÄISCHE PATENTSCHRIFT**

(45) Veröffentlichungstag und Bekanntmachung des Hinweises auf die Patenterteilung:
**11.01.2017 Patentblatt 2017/02**

(21) Anmeldenummer: **03769283.7**

(22) Anmeldetag: **18.09.2003**

(51) Int Cl.:
***C07C 7/05*** (2006.01) ***C07C 51/25*** (2006.01)
***C07C 45/35*** (2006.01) ***C07C 255/08*** (2006.01)
***C07C 253/24*** (2006.01) ***C07D 301/10*** (2006.01)
***C07C 5/48*** (2006.01) ***C07C 5/32*** (2006.01)
***C07C 11/06*** (2006.01) ***B01J 23/00*** (2006.01)
***B01J 23/887*** (2006.01)

(86) Internationale Anmeldenummer:
**PCT/EP2003/010375**

(87) Internationale Veröffentlichungsnummer:
**WO 2004/031107 (15.04.2004 Gazette 2004/16)**

# (54) VERFAHREN ZUR HERSTELLUNG VON WENIGSTENS EINEM PARTIELLEN OXIDATIONS- UND/ODER AMMOXIDATIONSPRODUKT DES PROPYLENS

METHOD FOR THE PRODUCTION OF AT LEAST ONE PARTIAL PROPYLENE OXIDATION AND/OR AMMOXIDATION PRODUCT

PROCEDE DE PRODUCTION D'AU MOINS UN PRODUIT D'OXYDATION ET/OU AMMOXYDATION PARTIELLE DU PROPYLENE

(84) Benannte Vertragsstaaten:
**AT BE BG CH CY CZ DE DK EE ES FI FR GB GR HU IE IT LI LU MC NL PT RO SE SI SK TR**

(30) Priorität: **27.09.2002 DE 10245585**
**01.10.2002 DE 10246119**

(43) Veröffentlichungstag der Anmeldung:
**29.06.2005 Patentblatt 2005/26**

(73) Patentinhaber: **BASF SE**
**67056 Ludwigshafen am Rhein (DE)**

(72) Erfinder:
- **HECHLER, Claus**
**67063 Ludwigshafen (DE)**
- **SCHINDLER, Götz-Peter**
**68219 Mannheim (DE)**
- **PETZOLDT, Jochen**
**68163 Mannheim (DE)**
- **ADAMI, Christoph**
**69469 Weinheim (DE)**
- **MACHHAMMER, Otto**
**68163 Mannheim (DE)**
- **MÜLLER-ENGEL, Klaus Joachim**
**76297 Stutensee (DE)**
- **MARTAN, Hans**
**67227 Frankenthal (DE)**

(56) Entgegenhaltungen:
**WO-A-00/20404 WO-A-01/96270**
**US-A- 5 268 497**

Bemerkungen:
Die Akte enthält technische Angaben, die nach dem Eingang der Anmeldung eingereicht wurden und die nicht in dieser Patentschrift enthalten sind.

EP 1 546 073 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Vorliegende Erfindung betrifft ein Verfahren zur Herstellung von wenigstens einem partiellen Oxidations- und/oder Ammoxidationsprodukt des Propylens, bei dem man

a) in einem ersten Schritt Roh-Propan im Beisein und/oder unter Ausschluss von Sauerstoff einer homogenen und/oder einer heterogen katalysierten Dehydrierung und/oder Oxidehydrierung unterwirft, wobei ein Propan und Propylen enthaltendes Gasgemisch 1 erzeugt wird,
und

b) aus im ersten Schritt gebildetem Gasgemisch 1 von den darin enthaltenen, von Propan und Propylen verschiedenen, Bestandteilen gegebenenfalls eine Teilmenge abtrennt und/oder in andere Verbindungen wandelt, wobei aus dem Gasgemisch 1 Jeweils ein Propan und Propylen sowie auch von Sauerstoff, Propan und Propylen verschiedene Verbindungen enthaltendes Gasgemisch 1' erzeugt wird, und in wenigstens einem weiteren Schritt

c) Gasgemisch 1 und/oder Gasgemisch 1' als Bestandteil eines Gasgemisches 2 einer heterogen katalysierten Gasphasen-Partialoxidation und/oder partiellen Gasphasen-Ammoxidation von im Gasgemisch 1 und/oder Gasgemisch 1' enthaltenem Propylen unterwirft.

Unter Roh-Propan soll in dieser Schrift ein Propan enthaltendes Gas verstanden werden, das neben Propan sowie gegebenenfalls Propylen noch wenigstens eine, häufig wenigstens zwei oder drei und vielfach wenigstens vier oder fünf von Propan und Propylen verschiedene chemische Verbindung enthält. Letzteres ist dann der Fall, wenn solche chemische Verbindungen chromatographisch, z.B. gaschromatographisch, in Roh-Propan nachweisbar sind.

Unter Oxidehydrierung von Propan soll in dieser Schrift eine Dehydrierung verstanden werden, die durch anwesenden Sauerstoff erzwungen und bei der intermediär kein freier Wasserstoff gebildet wird bzw. nachweisbar ist. Im Unterschied zur konventionellen Dehydrierung, die endotherm verläuft, ist die Wärmetönung der Oxidehydrierung exotherm. Die Oxidehydrierung von Propan kann unter Einwirkung erhöhter Temperatur sowohl homogen (d.h., ohne das Beisein eines z.B. festen Katalysators; vgl. z.B. US-A 3 798 283) als auch heterogen katalysiert (z.B. an festen Katalysatoren; vgl. DE-A 2058054 und DE-A 19530494) durchgeführt werden.

Das Gleiche gilt im wesentlichen für die konventionelle Dehydrierung, bei der der Dehydrierschritt ohne aktive Mitwirkung von Sauerstoff erfolgt (vgl. z.B. EP-A 731 077 und WO 01/96270). D.h., als primäres Nebenprodukt entsteht hier Wasserstoff und nicht Wasser wie im Fall der Oxidehydrierung.

Unter einer vollständigen Oxidation von Propylen wird in dieser Schrift verstanden, dass der im Propylen insgesamt enthaltene Kohlenstoff in Oxide des Kohlenstoffs (CO, $CO_2$) umgewandelt wird. Alle davon verschiedenen Umsetzungen des Propylens unter reaktiver Einwirkung von molekularem Sauerstoff werden in dieser Schrift mit dem Begriff der Partialoxidation subsummiert. Die zusätzliche reaktive Einwirkung von Ammoniak kennzeichnet die Ammoxidation.

Die in dieser Schrift bevorzugten partiellen Oxidations- und/oder Ammoxidationsprodukte des Propylens sind Acrolein, Acrylsäure,, Propylenoxid und Acrylnitril.

Partielle Oxidations- und/oder Ammoxidationsprodukte des Propylens sind wichtige Zwischenprodukte, z.B. zur Herstellung von Polymerisaten.

Die Durchführung solcher partiellen Oxidationen und/oder Ammoxidationen (durch in an sich bekannter Weise zu steuernde Wahl des Gehaltes an Ammoniak im Reaktionsgasgemisch kann die Reaktion im wesentlichen ausschließlich als partielle Oxidation, oder ausschließlich als partielle Ammoxidation, oder als Überlagerung beider Reaktionen gestaltet werden) ist an sich bekannt. Es handelt sich dabei um heterogen katalysierte Gasphasen-Reaktionen an festen, in der Regel oxidischen, Katalysatoren.

Beispielhaft zitiert seien die DE-A 2 351 151 (Beispiel für die Umsetzung von Propylen zu Acrolein und/oder Acrylsäure sowie von Propylen zu Acrylnitril) und die EP-A 372 972 (Beispiel für die Umsetzung von Propylen zu Propylenoxid).

Als Oxidationsmittel wird normalerweise molekularer Sauerstoff verwendet, der z.B. in reiner Form oder im Gemisch mit sich bezüglich der Partialoxidation-/ammoxidation im wesentlichen inert verhaltenden Gasen (z.B. als Luft) dem Reaktionsgasgemisch zugesetzt werden kann. Häufig sind die Reaktanden im Reaktionsgasgemisch auch aus Gründen der Wärmeabfuhr und aus Gründen der sicheren Reaktionsführung durch wenigstens ein Inertgas (z.B. $N_2$, $H_2O$, CO, $CO_2$, gesättigte, z.B. $C_1$-$C_5$-,Kohlenwasserstoffe (z.B. gemäß DE-A 1924431 und EP-A 293224), He und/oder Ar etc.) verdünnt. Die DE-AS 2251364 empfiehlt u.a. Butan als inertes Verdünnungsgas mitzuverwenden. Die Ammoxidation unterscheidet sich dabei, wie bereits erwähnt, durch ein zusätzliches Beisein von Ammoniak.

Als Ausgangspropylen wird im Unterschied zu Labor und Technikumsversuchen großtechnisch üblicherweise kein chemisch reines, sondern ein Verunreinigungen aufweisendes rohes Propylen verwendet, das aber einer vergleichsweise hohe Reinheit aufweist (z.B. "polymer grade" oder "chemical grade"; vgl. DE-A 10131297).

Die Gewinnung von solchermaßen vergleichsweise reinem Roh-Propylen ist relativ aufwendig und kostspielig. Sie geht normalerweise von rohen paraffinischen Kohlenwasserstoffen aus und beinhaltet in der Regel wenigstens eine Reinigungsstufe in welcher nicht umgesetzter paraffinischer Kohlenwasserstoff von gebildetem Propylen mittels physikalischer Verfahren abgetrennt wird (vgl. z.B. DE-A 3 521 458)). Üblicherweise umfasst die wenigstens eine Reinigungsstufe auch eine Trennung von von Propylen verschiedenen Olefinen sowie von von Propylen verschiedenen anderen Nebenprodukten, einschließlich der im rohen paraffinischen Kohlenwasserstoff bereits enthaltenen Nebenkomponenten.

Die vorgenannten Abtrennungen sind in aller Regel investitionsintensiv und in Folge der Ähnlichkeit von olefinischen/paraffinischen Kohlenwasserstoffen sehr energieintensiv. Sie werden daher üblicherweise nur im Verbund mit Raffineriecrackern und Steamcrackern angewendet und rechnen sich nur deshalb, weil die überwiegende Menge des so gewonnenen Roh-Propylen für nachfolgende Polymerisationen (z.B. Herstellung von Polypropylen) einerseits in großen Mengen benötigt wird (Stichwort: "economy of scale") und dabei andererseits eine hohe Wertschöpfung erfährt.

Der von diesen Roh-Propylenen in Partialoxidationen und/oder -ammoxidationen fließende Anteil ist von eher untergeordneter Bedeutung und läuft als Nebenbedarfsstrom quasi mit, weshalb solchermaßen erzeugtes Roh-Propylen auch für Partialoxidationen und/oder -ammoxidationen noch einen akzeptablen Rohstoffpreis aufweist.

Dieser Rohstoffpreis ließe sich nur dadurch merklich senken, wenn wenigstens auf einen Teil der angesprochenen Trennungen oder auf deren Gesamtmenge verzichtet werden könnte.

Als Problemlösung schlägt die EP-B 938463 vor, in einem ersten Schritt z.B. rohes Propan im Beisein von Sauerstoff heterogen katalysiert partiell zu dehydrieren, wobei ein erstes, Propylen und Propan enthaltendes Gasgemisch erzeugt wird, das als solches, d.h., ohne Zwischenbehandlung, als Bestandteil eines zweiten Gasgemisches einer heterogen katalysierten Gasphasen-Partialoxidation von im ersten Gasgemisch enthaltenem Propylen zu Acrolein und/oder Acrylsäure unterworfen wird.

Zur Frage der Reinheit des zu verwendenden rohen Propans führt die EP-B 938463 in Spalte 3, Zeilen 40 ff u.a. aus: "The purity of the starting material alkane is not particularly limited". "Further, the starting material alkane may be a mixture of various alkanes. Typically the feed will comprise at least 30 mole percent, preferably at least 50 mole percent and more perferably at least 80 mole percent propane. The source of the alkane, e.g. propane feed, for use in the process of the present invention is not critical."

Darüber hinaus lehrt die EP-B 938463 in Spalte 3, Zeilen 17ff: "Hence, after recovery of the acrolein, the noncondensed gases containing propane may be recycled without significant, additional purification steps."

Die Lehre der EP-A 117146 entspricht im wesentlichen der EP-B 938463, sieht man davon ab, dass die EP-A 117146 empfiehlt, die heterogen katalysierte Dehydrierung des Propans unter Ausschluss von Sauerstoff durchzuführen.

Ferner lehrt die EP-A 117146 auf Seite 11, Zeilen 14ff den vorgenannten Recyclestrom betreffend: "Since the light and heavy hydrocarbon by - products, such as methane, ethane, ethylene, butane, and butenes boil at temperatures significantly different from those of acrolein or the $C_3$ hydrocarbons, they may be separated by destillation. Alternatevely, streams containing the by - products in concentrated amounts may be purged." Die Möglichkeit eines solchen purge-Stromes an Recyclegas sieht auch die EP-B 938463 in Spalte 11, Zeile 10.

Die Notwendigkeit einer Abtrennung der vorgenannten Nebenkomponenten vorab der Partialoxidation und/oder -ammoxidation sehen beide Schriften nicht.

Die Lehre der WO 01/96270 schließt sich an die in der EP-B 938463 und an die in der EP-A 117 146 gegebene Lehre an. So führt sie auf Seite 4, Zeile 10ff aus: "Selbstredend kann das Beschickungsgasgemisch der Oxidationsstufe B bei der erfindungsgemäßen Verfahrensweise neben den bereits genannten Bestandteilen zusätzlich noch andere Bestandteile wie z.B. CO, $CO_2$, $H_2O$, Edelgase wie He und/oder Ar, Wasserstoff, Methan, Ethylen, Butane, Butene, Butine, Pentane, Propin, Allene und/oder Acrolein enthalten."

Ferner lehrt die WO 01/96270 auf Seite 15, Zeilen 26ff bezüglich des für den Dehydrierschritt zu verwendenden rohen Propans: "Erfindungsgemäß wesentlich ist, dass das in der Stufe A verwendete Propan kein reines Propan sein muss. Vielmehr kann das verwendete Propan bis zu 50 Vol.-% anderer Gase wie z.B. Ethan, Methan, Ethylen, Butane, Butene, Propin, Acethylen, $H_2S$, $SO_2$, Pentane etc. enthalten."

Zwar empfiehlt die WO 01/96270 auch, aus dem im Dehydrierschritt gebildeten, Propan und Propylen enthaltenden, ersten Gasgemisch vor dessen Weiterverwendung für die Partialoxidation des darin enthaltenen Propylen wenigstens eine Teilmenge des im ersten Gasgemisch enthaltenen Wasserstoff und im Rahmen dieser Abtrennung gegebenenfalls auch andere, gegebenenfalls im wesentlichen alle, von Propan und Propylen verschiedenen Bestandteile abzutrennen, und in der EP-B 731077 wird eine quantitative Abtrennung aller von Propan, Propylen und gegebenenfalls molekularem Sauerstoff verschiedenen Bestandteile aus einem solchen ersten Gasgemisch vor seiner Weiterverwendung sogar als besonders bevorzugt betrachtet, doch mindert einerseits jedes diesbezüglich anzuwendende Trennverfahren die Wirtschaftlichkeit des Gesamtverfahrens und erweisen sich andererseits manche der in den vorgenannten Schriften als für eine solche quantitative Trennung empfohlenen Verfahren als wenig geeignet. Letzteres trifft z.B. auf das in der WO 01/96270 auf Seite 16 unten empfohlene Absorptions/Desorptions-Verfahren zu, das sich bei näherer

Überprüfung als wenig geeignet erwies, um z.B. $C_4$-Kohlenwasserstoffe von $C_3$-Kohlenwasserstoffen zu trennen.

Die Notwendigkeit, beim Rohstoffwechsel von Roh-Propylen zu Roh-Propan nach dem Dehydrier- und/oder Oxidehydrierschritt oder bereits vorab dieses Schrittes bestimmte Nebenkomponenten abzutrennen, um in dem wenigstens einen Oxidations- und/oder Ammoxidationsschritt neben der Bildung des gewünschten Zielprodukts die Bildung von Nebenprodukten zu mindern, sieht der zitierte Stand der Technik nicht. Er ist sich zwar der sich mit dem Rohstoffwechsel verändernden Nebenproduktbildung bewusst (gemäß WO 01/96270 bedingt z.B. das Beisein von Propan in der partiellen Oxidation eine erhöhte Bildung an Propionaldehyd und/oder Propionsäure; der Vorteil einer Nichtabtrennung des Propan vom in der Dehydrierung gebildeten Propylen wird jedoch höher bewertet), sieht diese jedoch insofern als nicht besonders kritisch, als eine Vorabtrennung in der Regel aufwendiger ist, als die Trennung von Zielprodukt und Nebenprodukt. Dies auch vor dem Hintergrund, dass in jedem Fall eine Zielproduktabtrennung von gebildeten Nebenprodukten durchgeführt werden muss.

Diese Auffassung wird ganz offensichtlich auch von jenen geteilt, die wie die EP-A 1192987 oder die DE-A 10122027, oder die EP-A 608838, oder die EP-A 529853, oder die DE-A 10051419, oder die DE-A 10119933 empfehlen, das Eingangs dieser Schrift definierte Verfahren in einer einzigen Reaktionszone (meist wird es an wenigstens zwei Reaktionszonen durchgeführt) an einer Katalysatorbeschickung durchzuführen, deren aktive Oxidmasse wenigstens ein Multimetalloxid ist, das die Elemente Mo, V und Te und/oder Sb enthält. Grundlage dieser Verfahrensweise ist, dass die relevante aktive Oxidmasse einerseits sowohl die Oxidehydrierung des Propans zu Propylen (vgl. z.B. EP-B 938463, Spalte 4, Zeile 37ff) und andererseits auch die Partialoxidation- und/oder -ammoxidation von Propylen zu katalysieren vermag. Selbstredend wird bei einer solchen Verfahrensweise das Gasgemisch 1 als solches für den nachfolgenden wenigstens einen partiellen Oxidations- und/ oder Ammoxidationsschritt eingesetzt.

Nichtsdestotrotz empfiehlt z.B. die EP-A 1192987 auf Seite 9, Zeile 26ff: "Similarly, there is no limitation on the source of the alkane. It may be purchased, per se, or in admixture with an alkene and/or other impurities. Moreover, the alkane, regardless of source, and the alkene, regardless of source, may be blended as desired." In völliger Entsprechung lehrt die DE-A 10122027 auf Seite 3, Zeilen 35/36: "An das für das erfindungsgemäße Verfahren zu verwendende Propan werden keine besonders hohen Ansprüche in Bezug auf seine Reinheit gestellt." Die WO 0196271 betrachtet ebenfalls Roh-Propane unterschiedlichster Reinheit als anwendbar.

Nachteilig an den vorstehenden Empfehlungen des Standes der Technik ist, dass sie nicht hinterfragen, welche in kostengünstigem Roh-Propan gegebenenfalls bereits enthaltenen Bestandteile oder sich im Verlauf des ersten Schrittes aus solchen Bestandteilen bildende und dann im ersten Gasgemisch enthaltene chemische Verbindungen (die in den normalerweise eingesetzten Roh-Propylenen nicht oder nur in Spuren enthalten sind, so dass ihre negativen Auswirkungen bisher unbemerkt blieben) bezüglich der nachfolgenden heterogen katalysierten Partialoxidation und/oder -ammoxidation als Katalysatorgifte wirken, indem sie deren Aktivität und/oder Selektivität hinsichtlich der angestrebten Partialoxidation und/oder -ammoxidation des Propylens mindern.

Als Ergebnis eingehender und sorgfältiger Untersuchungen wurde nun gefunden, dass $C_4$-Kohlenwasserstoffe (chemische Verbindungen, die aus vier Kohlenstoffatomen und Wasserstoff bestehen) im allgemeinen und unter diesen vor allem die Gruppe der olefinischen Vertreter (Buten-1, trans-Buten-2, cis-Buten-2 und iso-Buten) und unter diesen wiederum insbesondere das Buten-1 solche Katalysatorgifte bilden. Aber auch die gesättigten Vertreter und die anderen ungesättigten Vertreter wirken nachteilig.

Gerade $C_4$-Kohlenwasserstoffe (z.B. n-Butan, iso-Butan, trans-Buten-2, cis-Buten-2, iso-Buten, Butadien 1,3, Butadien 1,2, 1-Butin und/oder 2-Butin) bilden aber ubiquitäre Begleiter des Propan und sind daher üblicherweise in signifikanten Mengen in kostengünstigen Roh-Propanen enthalten. Diese Aussage trifft in ganz besonderem Ausmaß auf die gesättigten $C_4$-Kohlenwasserstoffe zu, aus denen jedoch unter den Bedingungen einer partiellen Dehydrierung und/oder Oxidehydrierung von Propan unweigerlich wenigstens teilweise die olefinischen $C_4$-Kohlenwasserstoffe, vor allem das besonders störende Buten-1, gebildet werden.

Die Aufgabe der vorliegenden Erfindung bestand daher darin, im Unterschied zu den Verfahren des Standes der Technik ein wie eingangs beschriebenes Verfahren zur Herstellung von wenigstens einem partiellen Oxidations- und/oder Ammoxidationsprodukt des Propylens zur Verfügung zu stellen, das den vorgenannten Sachverhalten Rechnung trägt.

Demgemäß wurde ein Verfahren zur Herstellung von wenigstens einem partiellen Oxidations- und/oder Ammoxidationsprodukt des Propylens, bei dem man

a) in einem ersten Schritt Roh-Propan im Beisein und/oder unter Ausschluss von Sauerstoff einer homogenen und/oder heterogen katalysierten Dehydrierung und/oder Oxidehydrierung unterwirft, wobei ein Propan und Propylen enthaltendes Gasgemisch 1 erhalten wird,
und

b) aus im ersten Schritt gebildetem Gasgemisch 1, von den darin enthaltenen, von Propan und Propylen verschiedenen, Bestandteilen gegebenenfalls eine Teilmenge abtrennt und/oder in andere Verbindungen wandelt, wobei

aus dem Gasgemisch 1 jeweils ein Propan und Propylen sowie auch von Sauerstoff, Propan und Propylen verschiedene Verbindungen enthaltendes Gasgemisch 1' erzeugt wird, und in wenigstens einem weiteren Schritt Geänderte Beschreibungsseiten - Reinschrift -

c) Gasgemisch 1 und/oder Gasgemisch 1' als Bestandteil eines Gasgemisches 2 einer heterogen katalysierten Gasphasen-Partialoxidation und/oder partiellen Gasphasen-Ammoxidation von im Gasgemisch 1 und/oder Gasgemisch 1' enthaltenem Propylen unterwirft,

gefunden,
das dadurch gekennzeichnet ist,
dass der Gehalt des Gasgemisches 2 an Buten-1 ≤ 1 Vol-% beträgt, das erfindungsgemäße Verfahren in einer Reaktionszone an einer Katalysatorbeschickung durchgeführt wird, deren Aktivmasse wenigstens eine Multimetalloxidmasse ist, die die Elemente Mo, V, wenigstens eines der beiden Elemente Te und Sb, und wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthält.

Weiterhin wurde ein Verfahren zur Herstellung von wenigstens einem partiellen Oxidations- und/oder Ammoxidationsprodukt des Propylens, bei dem man

a) in einem ersten Schritt Roh- Propan im Beisein und/oder unter Ausschluß von Sauerstoff einer homogenen und/oder einer heterogen katalysierten Dehydrierung und/oder Oxidhydrierung unterwirft, wobei ein Propan und Propylen enthaltendes Gasgemisch 1 erhalten wird,
und

b) aus im ersten Schritt gebildetem Gasgemisch 1, von den darin enthaltenen, von Propan und Propylen verschiedenen, Bestandteilen gegebenenfalls eine Teilmenge abtrennt und/oder in andere Verbindungen wandelt, wobei aus dem Gasgemisch 1 jeweils ein Propan und Propylen sowie auch von Sauerstoff, Propan und Propylen verschiedene Verbindungen enthaltendes Gasgemisch 1' erzeugt wird, und in wenigstens einem weiteren Schritt

c) Gasgemisch 1 und/oder Gasgemisch 1' als Bestandteil eines Gasgemisches 2 einer heterogen katalysierten Gasphasen Partialoxidation und/oder partiellen Gasphasen-Ammoxidation von im Gasgemisch 1 und/oder Gasgemisch 1' enthaltenem Propylen unterwirft,

gefunden,
das dadurch gekennzeichnet ist, daß
der Gehalt des Gasgemisches 2 an Buten-1 ≤ 1 Vol.-% beträgt, wobei der erste Schritt in einer getrennten Reaktionszone durchgeführt wird und in der heterogen katalysierten Gasphasen-Partialoxidation ein Katalysator mitverwendet wird, dessen Aktivmasse die Elemente Mo und V enthält.

Die Menge der auf dem Weg zum Gasgemisch 1' aus dem Gemisch 1 abgetrennten und/oder in andere Verbindungen gewandelten, von Propan und Propylen verschiedenen, Bestandteile kann, bezogen auf die im Gasgemisch 1 enthaltene Menge dieser Bestandteile, beim erfindungsgemäßen Verfahren bis zu 5 Gew.-%, oder bis zu 10 Gew.-%, oder bis zu 20 Gew.-%, oder bis zu 30 oder 40 Gew.-%, oder bis zu 60 Gew.-%, oder bis zu 70 oder 80 Gew.-%, oder bis zu 85 oder 90 Gew.-%, oder bis zu 94 Gew.-%, oder bis zu 96 oder 98 Gew.-%, oder bis zu 99 Gew.-% oder mehr betragen.

Erfindungsgemäß bevorzugt beträgt der Gesamtgehalt des Gasgemisches 2 an Buten-1 ≤0,9 Vol.-%, oder ≤0,75 Vol.-%, oder ≤0,6 Vol.-%, bzw. ≤0,5 Vol.-%, oder ≤0,4 Vol.-%, besonders bevorzugt ≤0,3 Vol.-%, ganz besonders bevorzugt ≤0,2 Vol.-% und noch besser ≤0,1 Vol.-%bzw. ≤0,05 Vol.-%, oder ≤0,03 Vol.-% bzw. ≤0,01 Vol.-%. Bei Gasgemischen 2, die überhaupt kein Buten-1 mehr enthalten, tritt dessen negative Wirkung selbstverständlich überhaupt nicht mehr in Erscheinung. Im Rahmen einer Gesamtwirtschaftlichkeitsbetrachtung kann es jedoch vertretbar sein, eine gewisse mindernde Wirkung des Buten-1 im Gasgemisch 2 in Kauf zu nehmen, und dessen Gehalt im Gasgemisch 2 bei Werten von >0, 001 Vol.-%, oder ≥0,003 Vol.-%, oder ≥ 0,006 Vol.-%, oder in extremen Fällen ≥ 0,009 Vol.-% zu belassen.

Die vorgenannten Grenzwerte gelten im Rahmen des erfindungsgemäßen Verfahrens bevorzugt nicht nur für die Menge an im Gasgemisch 2 enthaltenem Buten-1, sondern, unabhängig voneinander, gleichzeitig auch für jeden anderen möglichen Vertreter innerhalb der Butene (d.h. , für trans-Buten-2, für cis-Buten-2 und für iso-Buten), und ganz besonders bevorzugt gleichzeitig auch für die Gesamtmenge an Butenen im Gasgemisch 2. D.h., erfindungsgemäß geeignete Gasgemische 2 sind z.B. solche, für die gilt:

- Gehalt an Buten-1 ≤1 Vol.-% und Gesamtgehalt an Butenen ≤1 Vol.-%; oder

- Gehalt an Buten-1 ≤0,5 Vol.-% und Gesamtgehalt an Butenen ≤1 Vol.-%; oder
- Gehalt an Buten-1 ≤0,3 Vol.-% und Gesamtgehalt an Butenen ≤1 Vol.-%; oder
- Gehalt an Buten-1 ≤0,5 Vol.-% und Gesamtgehalt an Butenen ≤0,75 Vol.-%; oder
- Gehalt an Buten-1 ≤0,75 Vol.-% und Gesamtgehalt an Butenen ≤1 Vol.-%; oder
- Gehalt an Buten-1 ≤0,4 Vol.-% und Gesamtgehalt an Butenen ≤1 Vol.-%; oder
- Gehalt an Buten-1 ≤0,2 Vol.-% und Gesamtgehalt an Butenen ≤1 Vol.-%; oder
- Gehalt an Buten-1 ≤0,5 Vol.-% und Gesamtgehalt an Butenen ≤0, 5Vol.-%; usw.

Um die vorgenannten Grenzwerte einzuhalten, bietet das erfindungsgemäße Verfahren im wesentlichen zwei Möglichkeiten, von denen entweder nur eine oder beide zur Anwendung gelangen können.

Zum einen kann.von einem Roh-Propan ausgegangen werden, das entweder überhaupt keine $C_4$-Kohlenwasserstoffe mehr enthält, oder das $C_4$-Kohlenwasserstoffe nur noch in solchen Mengen enthält, dass die erfindungsgemäß einzuhaltenden Grenzen für die Gehalte an Buten-1, (Gesamt-)Butenen sowie (Gesamt-)$C_4$-Kohlenwasserstoffen im Gasgemisch 2 erfüllt werden. Welcher Gehalt an welchen der möglichen $C_4$-Kohlenwasserstoffe im Roh-Propan dabei noch mit dem erfindungsgemäßen Verfahren verträglich ist, hängt u.a. von den für den ersten Schritt des Verfahrens angewandten spezifischen Randbedingungen ab und kann vom Fachmann, an diese spezifischen Randbedingungen jeweils angepasst, in wenigen Vorversuchen ermittelt werden.

Eine gegebenenfalls erforderliche Abtrennung von $C_4$-Kohlenwasserstoffen aus käuflich erworbenem Roh-Propan kann in an sich bekannter Weise z.B. rektifikativ vorgenommen werden. Selbstredend kommen auch alle anderen Trennverfahren wie z.B. Adsorption/Desorption (z.B. Druckwechseladsorption), Extraktion und/oder Absorption/Desorption zusätzlich in Betracht.

Ergänzend und/oder alternativ können aus dem Gasgemisch 1 vorab seiner erfindungsgemäßen Weiterverwendung als Gasgemisch 1' $C_4$-Kohlenwasserstoffe im allgemeinen und Buten-1 bzw. Butene im besonderen soweit abgetrennt werden, bis die erfindungsgemäß einzuhaltenden Grenzen erreicht bzw. unterschritten sind. Diese Maßnahme ist z.B. dann angebracht, wenn im Verlauf des Oxidehydrier- bzw. Dehydrierschrittes z.B. durch Disproportionierung und/oder Metathese die störenden $C_4$-Kohlenwasserstoffe z.B. aus Propan erst gebildet werden. Die Wahrscheinlichkeit dafür ist u.a. dann erhöht, wenn für den ersten Schritt des erfindungsgemäßen Verfahrens (im Fall einer katalytischen Dehydrierung) die Teilmengenkreisgasfahrweise der DE-A 10211275 angewendet wird. Als Trennverfahren können dabei z.B. die in der DE-A 10131297 beschriebene Kombination aus Absorption und Desorption bzw. Strippen (bevorzugt als Druckabsorption), Druckwechseladsorption, rektifikative und/oder extraktive Verfahren angewendet werden. Im Fall einer Strippung ist darauf zu achten, dass über das verwendete Strippgas keine $C_4$-Kohlenwasserstoffe eingeführt werden.

Wird das (z.B. aus dem Produktgasgemisch einer katalytischen Dehydrierung absorptiv entnommene) im Absorptionsmittel angereichert enthaltene Propan und Propen mittels Luft freigestrippt, kann eine der erfindungsgemäßen Verfahrenskonstellationen vorliegen, bei der das durch das Freistrippen erzeugte Gasgemisch 1' bei geschickter Wahl der Strippgasmenge unmittelbar für die partielle Gasphasen-Partialoxidation und/oder die partielle Gasphasen-Ammoxidation verwendbar und damit mit dem Gasgemisch 2 identisch ist. In der Regel wird in diesem Fall der Gehalt an von Propan, Propen und Sauerstoff verschiedenen Komponenten im Gasgemisch 1' 35 bis 55 Vol.-% betragen. Im Fall der erfindungsgemäßen Herstellung von Acrolein und/oder Acrylsäure ist eine solche Verfahrensweise bevorzugt.

Selbstredend kann im Rahmen der angesprochenen Abtrennungen von $C_4$-Kohlenwasserstoffen auch eine Abtrennung anderer, von Propan und Propylen verschiedenen Bestandteilen einhergehen. Natürlich können auch Teilmengen an Propan und/oder Propylen jeweils mitabgetrennt werden.

Erfindungsgemäß zweckmäßig wird man die angesprochenen Trennverfahren gezielt auf eine Abtrennung der $C_4$-Kohlenwasserstoffe richten, um den insgesamt erforderlichen Trennaufwand und damit die Minderung der Wirtschaftlichkeit zu begrenzen.

D.h. im Normalfall wird ein Gasgemisch 1' beim erfindungsgemäßen Verfahren noch wenigstens ≥0,1 Vol.-%, häufig ≥0,2 Vol,-%, oder ≥0,3 Vol.-% bzw. ≥0,4 Vol.-%, oder ≥0,5 Vol.-%, vielfach ≥0,6 Vol.-%, oder ≥0,8 Vol.-% bzw. ≥1 Vol.-%, oft ≥2 Vol.-%, oder ≥3 Vol.-% bzw. ≥5 Vol.-%, durchaus üblich ≥10 Vol.-%, oder ≥15 Vol.-% bzw. ≥20 Vol.-%, oder ≥25 Vol.-%, oder ≥30 Vol.-%, oder ≥35 Vol.-% an von Propan und Propylen sowie Sauerstoff verschiedenen Bestandteilen enthalten.

Im Regelfall wird der Anteil an von Propan und Propylen sowie Sauerstoff verschiedenen Bestandteilen im Gasgemisch 1' beim erfindungsgemäßen Verfahren jedoch ≤80 Vol.-%, bzw. ≤70 Vol.-%, oder ≤60 Vol.-%, oder ≤50

Vol.-%, oder ≤40 Vol.-% betragen.

Erfindungsgemäß von Vorteil ist es, wenn die bisher angesprochenen Abtrennungen so durchgeführt werden, dass das Gasgemisch 2 nicht nur die erfindungsgemäß geeigneten Buten-1- sowie gegebenenfalls sonstigen Butenisomeren- und gegebenenfalls Butengesamtgehalte erfüllt, sondern gleichzeitig einen Gesamtgehalt von $C_4$-Kohlenwasserstoffen aufweist, der ≤3 Vol.-%, oder ≤2,5 Vol.-%, bevorzugt ≤2 Vol.-% und ganz besonders bevorzugt ≤0,5 Vol.-%, oder ≤0,3 Vol.-% bzw. ≤0,1 Vol.-% beträgt. Dies rührt daher, dass sich ein Beisein von $C_4$-Kohlenwasserstoffen (insbesondere n-Butan und/oder iso-Butan) bei der partiellen Oxidation und/oder Ammoxidation des Propylens ganz generell als nachteilig herausgestellt hat.

Bei Gasgemischen 2, die überhaupt keine $C_4$-Kohlenwasserstoffe mehr enthalten, tritt deren negative Wirkung selbstverständlich überhaupt nicht mehr in Erscheinung. Im Rahmen einer Gesamtwitschaftlichkeitsbetrachung kann es jedoch vertretbar sein, eine gewisse mindernde Wirkung der $C_4$-Kohlenwasserstoffe im Gasgemisch 2 in Kauf zu nehmen, und deren Gesamtgehalt im Gasgemisch 2 bei Werten von ≥ 0,05 Vol.-%, oder ≥ 0,07 Vol.-%, oder ≥ 0,09 Vol.-%, oder ≥ 0,1 Vol.-% bzw. in extremen Fällen ≥ 0,2 Vol.-% zu belassen.

D.h., die Ziele der erfindungsgemäßen Verfahrensweise werden insbesondere dann erreicht, wenn für das Gasgemisch 2 wenigstens eine für den Buten-1-Gehalt gesetzte erfindungsgemäße Grenze und gleichzeitig eine für den Gesamtgehalt an $C_4$-Kohlenwasserstoffen in dieser Schrift gesetzte Grenze erfüllt ist.

Mit anderen Worten sind erfindungsgemäß geeignete Gasgemische 2 z.B. solche, für die gleichzeitig erfüllt ist

- Gehalt an Buten-1 ≤ 1 Vol.-% und Gesamtgehalt an $C_4$-Kohlenwasserstoffen ≤ 3 Vol-%; oder
- Gehalt an Buten-1 ≤ 1 Vol.-% und Gesamtgehalt an $C_4$-Kohlenwasserstoffen ≤ 2 Vol-%; oder
- Gehalt an Buten-1 ≤ 0,5 Vol.-% und Gesamtgehalt an $C_4$-Kohlenwasserstoffen ≤ 3 Vol-%; oder
- Gehalt an Buten-1 ≤ 0,5 Vol.-% und Gesamtgehalt an $C_4$-Kohlenwasserstoffen ≤ 2 Vol-%; oder
- Gehalt an Buten-1 ≤ 0,75 Vol.-% und Gesamtgehalt an $C_4$-Kohlenwasserstoffen ≤ 3 Vol-%; oder
- Gehalt an Buten-1 ≤ 0,75 Vol.-% und Gesamtgehalt an $C_4$-Kohlenwasserstoffen ≤ 2 Vol-%; oder
- Gehalt an Buten-1 ≤ 0,4 Vol.-% und Gesamtgehalt an $C_4$-Kohlenwasserstoffen ≤ 3 Vol-%; oder
- Gehalt an Buten-1 ≤ 0,4 Vol.-% und Gesamtgehalt an $C_4$-Kohlenwasserstoffen ≤ 2 Vol-%; oder
- Gehalt an Buten-1 ≤ 0,4 Vol.-% und Gesamtgehalt an $C_4$-Kohlenwasserstoffen ≤ 1 Vol-%; oder
- Gehalt an Buten-1 ≤ 0,3 Vol.-% und Gesamtgehalt an $C_4$-Kohlenwasserstoffen ≤ 3 Vol-%; oder
- Gehalt an Buten-1 ≤ 0,3 Vol.-% und Gesamtgehalt an $C_4$-Kohlenwasserstoffen ≤ 2 Vol-%; oder
- Gehalt an Buten-1 ≤ 0,3 Vol.-% und Gesamtgehalt an $C_4$-Kohlenwasserstoffen ≤ 1 Vol-%; usw..

Besonders günstig sind für das erfindungsgemäße Verfahren Gasgemische 2, für die gleichzeitig zusätzlich wenigstens eine in dieser Schrift gesetzte Grenze für den Gesamtgehalt an Butenen im Gasgemisch 2 erfüllt ist.

Mit anderen Worten sind erfindungsgemäß geeignete Gasgemische 2 insbesondere solche, für die gleichzeitig erfüllt ist

- Gehalt an Buten-1 ≤ 1 Vol.-% und Gesamtgehalt an Butenen ≤ 1 Vol.-% und Gesamtgehalt an $C_4$-Kohlenwasserstoffen ≤ 3 Vol-%; oder
- Gehalt an Buten-1 ≤ 0,75 Vol.-% und Gesamtgehalt an Butenen ≤ 1 Vol.-% und Gesamtgehalt an $C_4$-Kohlenwasserstoffen ≤ 3 Vol-%; oder
- Gehalt an Buten-1 ≤ 0,5 Vol.-% und Gesamtgehalt an Butenen ≤ 1 Vol.-% und Gesamtgehalt an $C_4$-Kohlenwasserstoffen ≤ 3 Vol-%; oder
- Gehalt an Buten-1 ≤ 0,3 Vol.-% und Gesamtgehalt an Butenen ≤ 1 Vol.-% und Gesamtgehalt an $C_4$-Kohlenwasserstoffen ≤ 3 Vol-%; oder
- Gehalt an Buten-1 ≤ 0,5 Vol.-% und Gesamtgehalt an Butenen ≤ 0,75 Vol.-% und Gesamtgehalt an $C_4$-Kohlenwasserstoffen ≤ 3 Vol-%; oder
- Gehalt an Buten-1 ≤ 0,5 Vol.-% und Gesamtgehalt an Butenen ≤ 0,5 Vol.-% und Gesamtgehalt an $C_4$-Kohlenwasserstoffen ≤ 3 Vol- % ; oder
- Gehalt an Buten-1 ≤ 0,5 Vol.-% und Gesamtgehalt an Butenen ≤ 0,5 Vol.-% und Gesamtgehalt an $C_4$-Kohlenwasserstoffen ≤ 2 Vol-%; usw..

Erfindungsgemäß besonders günstig sind dabei Verfahren, bei denen nicht nur die vorgenannten Kombinationen von Buten-1-Gehalt und Gesamtgehalt an $C_4$-Kohlenwasserstoffen sowie gegebenenfalls Gesamtgehalt an Butenen im Gasgemisch 2 erfüllt sind, sondern gleichzeitig ein Gasgemisch 1' verwendet wird, das noch wenigstens ≥ 0,1 Vol.-%, oder ≥ 0,2 Vol.-%, oder ≥ 0,3 Vol.-%, oder ≥ 0,4 Vol.-%, oder ≥ 0,5 Vol.-%, oder ≥ 0,6 Vol.-%, oder ≥ 0,8 Vol.-%, oder ≥ 1 Vol.-%, oder ≥ 2 Vol.-%, oder ≥ 3 Vol.-%, oder ≥ 5 Vol.-%, oder ≥ 10 Vol.-%, oder ≥ 15 Vol.-%, oder ≥ 20 Vol.-%, oder ≥ 25 Vol.-%, oder ≥ 30 Vol.-%, (meist aber ≤ 80 Vol.-%, oder ≤ 70 Vol.-%, oder ≤ 60 Vol.-%, oder ≤ 50 Vol.-%) an von Propan und Propylen sowie Sauerstoff verschiedenen Bestandteilen enthalten.

Die im Rahmen dieser Erfindung durchgeführten Studien ergaben ferner, daß es für das erfindungsgemäße Verfahren im Sinne einer Vermeidung von unerwünschter Vollverbrennung an Propylen bei der Partialoxidation und/oder -ammoxidation generell günstig ist, wenn der Propangehalt im Gasgemisch 2 ein vergleichsweise begrenzter ist. Erfindungsgemäß bevorzugt beträgt der Propangehalt im Gasgemisch 2 ≤ 60 Vol.-%, oder ≤ 50 Vol.-%. Besonders günstig sind Propangehalte im Gasgemisch 2 von 20 bis 40 Vol.-%, z.B. etwa 30 Vol.-%.

Läßt man einen für die Nitrilerzeugung gegebenenfalls mitzuverwendenden Ammoniakgehalt außer acht, (d.h., berücksichtigt ihn auch nicht in der Bezugsbasis für die Vol.-%), sind für das erfindungsgemäße Verfahren in der Regel geeignete Gasgemische 2 daher solche, die einerseits die erfindungsgemäßen Grenzen bezüglich ihres Gehalts an 1-Buten, bevorzugt zusätzlich an Gesamtbuten und besonders bevorzugt zusätzlich an Gesamt-$C_4$-Kohlenwasserstoff erfüllen und andererseits die nachfolgenden Gehalte aufweisen:

7 bis 15 Vol.-% $O_2$
5 bis 10 Vol.-% Propylen,
15 bis 40 Vol.-% Propan, häufig 25 bis 35 Vol.-%,
25 bis 60 Vol.-% Stickstoff, häufig 40 bis 60 Vol.-%,
1 bis 5 Vol.-% Summe aus CO, $CO_2$ und $H_2O$ und
0 bis 5 Vol.-% sonstige Bestandteile.

Die vorstehende Aussage trifft insbesondere dann zu, wenn das Gasgemisch 2 für eine heterogen katalysierte Partialoxidation des im Gasgemisch 2 enthaltenen Propylen zur Herstellung von Acrolein und/oder Acrylsäure verwendet wird.

Im übrigen kommen für alle vom erfindungsgemäßen Verfahren umfaßten heterogen katalysierten Partialoxidationen und/oder -ammoxidationen des Propylens als Gasgemische 2 vor allem solche in Betracht, die, läßt man einen gegebenenfalls enthaltenen $NH_3$-Gehalt für die Nitrilbildung wiederum unberücksichtigt (auch in der Bezugsbasis), in folgendem Zusammensetzungsraster liegen:

| | |
|---|---|
| $H_2O$ | ≤ 60 Vol.-%, meist ≤ 20 Vol.-%, in der Regel 0 bis 5 Vol.-%; |
| $N_2$ | ≤ 80 Vol.-%, meist ≤ 70 Vol.-%, in der Regel 40 bis 60 Vol.-%; |
| $O_2$ | bis zu 20 Vol.-%, meist 2 bis 20 Vol.-%, in der Regel 5 bis 15 Vol.-%; |
| CO | ≤ 2 Vol.-%, meist ≤ 1 Vol.-%, in der Regel 0 bis 0,5 Vol.-%; |
| $CO_2$ | ≤ 5 Vol.-%, meist ≤ 3 Vol.-%, in der Regel 0 bis 2 Vol.-%; |
| Ethan | ≤ 10 Vol.-%, meist ≤ 5 Vol.-%, in der Regel 0 bis 2 Vol.-%; |
| Ethylen | ≤ 5 Vol.-%, meist ≤ 2 Vol.-%, in der Regel 0 bis 0,5 Vol.-%; |
| Methan | ≤ 5 Vol.-%, meist ≤ 2 Vol.-%, in der Regel 0 bis 0,2 Vol.-%; |
| Propan | Vol.-%, meist 10 bis 50 Vol.-%, in der Regel 20 bis 40 Vol.-%; |
| Cyclopropan | Vol.-%, meist ≤ 0,05 Vol.-%, in der Regel 0 bis 150 Vol.ppm; |
| Propin | ≤ 0,1 Vol.-%, meist ≤ 0,05 Vol.-%, in der Regel 0 bis 150 Vol.ppm; |
| Propadien | ≤ 0,1 Vol.-%, meist ≤ 0,05 Vol.-%, in der Regel 0 bis 150 Vol.ppm; |
| Propylen | 0, ≤30 Vol.-%, meist ≥ 2, ≤ 20 Vol.-%, in der Regel 5 bis 10 Vol.%; |
| $H_2$ | ≤ 30 Vol.-%, meist ≤ 20 Vol.-%, in der Regel 0 bis 10 Vol.-%; |
| iso-Butan | ≤ 3 Vol.-%, bevorzugt ≤ 2 Vol.-%, häufig 0,1 bis 1 Vol.-%; |
| n-Butan | ≤ 3 Vol.-%, bevorzugt ≤ 2 Vol.-%, häufig 0,1 bis 1 vol.-%; |
| trans-Buten-2 | ≤ 1 Vol.-%, bevorzugt ≤ 0,5 Vol.-%, häufig ≥ 0,003 Vol.-%, ≤ 0,1 Vol.-%; |
| cis-Buten-2 | ≤ 1 Vol.-%, bevorzugt ≤ 0,5 Vol.-%, häufig ≤ 0,003 Vol.-%, ≤ 0,1 Vol.-%; |
| Buten-1 | ≤ 1 Vol.-%, bevorzugt ≤ 0,5 Vol.-%, häufig ≥ 0,003 Vol.-%, ≤ 0,1 Vol.-%; |
| iso-Buten | ≤ 1 Vol.-%, bevorzugt ≤ 0,5 Vol.-%, häufig ≥ 0,003 Vol.-%, ≤ 0,1 Vol.-%; |
| Butadien 1,3 | ≤ 1 Vol.-%, bevorzugt ≤ 0,5 Vol.-%, häufig ≥ 0,003 Vol.-%, ≤ 0,1 Vol.-%; |
| Butadien 1,2 | ≤ 1 Vol.-%, bevorzugt ≤ 0,5 Vol.-%, häufig ≥ 0 bis 0,1 Vol.-%; |
| 1-Butin | ≤ 0,5 Vol.-%, bevorzugt ≤ 0,3 Vol.-%, häufig 0 bis 0,1 Vol.-%; und |
| 2-Butin | ≤ 0,5 Vol.-%, bevorzugt ≤ 0,3 Vol.-%, häufig 0 bis 0,1 Vol.-%. |

Erfindungsgemäß geeignete Gasgemische 2 sind auch solche, die nicht nur die vorgenannten Spezifikationen, sondern gleichzeitig auch noch die folgenden Spezifikationen erfüllen:

sonstige ungesättigte $C_4$-Kohlenwasserstoffe insgesamt ≤ 0,5 Vol.-%, bevorzugt ≤ 0,3 Vol.-%, häufig 0 bis 0,1 Vol.-%;

$C_5$-Kohlenwasserstoffe insgesamt ≤ 0,1 Vol.-%, meist ≤ 0,05 Vol.-%, in der Regel 0 bis 300 Vol. ppm;

$C_6$-bis $C_8$-Kohlenwasserstoffe insgesamt ≤ 200 Vol.ppm, meist ≤ 150 Vol.ppm, in der Regel 0 bis 30 Vol.ppm;

Aceton ≤ 100 Vol.ppm;

$C_1$-bis $C_4$-Alkohole ≤ 100 Vol.ppm;

$C_2$-bis $C_4$-Aldehyde ≤ 100 Vol.ppm;

Acetylen ≤ 10 Vol.ppm;

Carbonylgruppen enthaltende Verbindungen insgesamt (berechnet als $Ni(CO)_4$) ≤ 100 Vol.ppm;

ionogenes Chlor ≤ 1 mg/kg, in der Regel 0 bis 0,2 mg/kg;

Cl enthaltende Verbindungen insgesamt und ausgedruckt als Cl ≤ 1 mg/kg, in der Regel 0 bis 0,2 mg/kg;

F enthaltende Verbindungen insgesamt und ausgedrückt als F ≤ 1 mg/kg, in der Regel 0 bis 0,2 mg/kg; und

S enthaltende Verbindungen insgesamt und ausgedrückt als S ≤ 10 mg/kg, häufig 0 bis 1 mg/kg, in der Regel 0 bis 0,1 mg/kg;

mit der Maßgabe, daß in allen vorgenannten Fällen der Gesamtgehalt aller Butene bevorzugt ≤ 1 Vol.-% (besonders bevorzugt ≤ 0,75 Vol.-% und ganz besonders bevorzugt ≤ 0,5 Vol.-%) und besonders bevorzugt gleichzeitig der Gesamtgehalt an $C_4$-Kohlenwasserstoffen ≤ 3 Vol.-% (bevorzugt ≤ 2 Vol.-% und besonders bevorzugt ≤ 1 Vol.-%) beträgt.

Nicht spezifizierte Verbindungen (Bestandteile) sind in den erfindungsgemäßen Gasgemischen 2 bevorzugt nicht enthalten, d.h., nicht nachweisbar.

Derartige Gasgemische 2 sind im Rahmen des erfindungsgemäßen Verfahrens, insbesondere bei Anwendung der erwähnten Trennverfahren zur Überführung von Gasgemischen 1 in erfindungsgerechte Gasgemische 1', in aller Regel auch dann erhältlich, wenn man im ersten Schritt Roh-Propane verwendet, die > 100 Gew.-ppm, oder ≥ 150 Gew.-ppm, oder ≥ 200 Gew.-ppm $C_4$-Kohlenwasserstoffe, oder z.B. bis zu 6 Vol.-% (z.B. 0,1 Vol.-% oder 0,5 Vol.-% bis 6 Vol.-%) derselben enthalten, insbesondere wenn sie die folgende Spezifikation erfüllen:

Gehalt an Propan ≥ 90 Vol.-%, meist ≥ 93 Vol.-%, in der Regel ≥ 95 Vol.-%;

Gehalt an Propan und Propylen ≤ 99,75 Vol.-%, oder ≤ 99,5 Vol.-%, meist ≤ 99 Vol.-% oder ≤ 98 Vol.-%, in der Regel ≤ 97 Vol.-%;

Gesamtgehalt an $C_4$-Kohlenwasserstoffen ≤ 6 Vol.-%, meist ≤ 5 Vol.-%, in der Regel ≤ 4 Vol.-%; häufig aber ≥ 0,5 Vol.-%, oder ≥ 1 Vol.-%, manchmal ≥ 2 Vol.-%, oder teilweise ≥ 3 Vol.-%;

Gehalt an Buten-1 ≤ 0,5 Vol.-%, meist ≤ 0,3 Vol.-%, in der Regel ≤ 0,1 Vol.-%; häufig aber ≥ 5 Vol.ppm, manchmal ≥ 10 Vol.ppm, oder teilweise ≥ 20 Vol.ppm;

Gesamtgehalt an Butenen ≤ 0,5 Vol.-%, meist ≤ 0,3 Vol.-%, in der Regel ≤ 0,1 Vol.-%; häufig aber ≥ 10 Vol.ppm, manchmal ≥ 20 Vol.ppm, oder teilweise ≥ 30 Vol.ppm;

Gehalt an Ethan ≤ 10 Vol.-%, meist ≤ 5 Vol.-%, in der Regel 0 bis 2 Vol.%;

Gehalt an Ethylen ≤ 5 Vol.-%, meist ≤ 2 Vol.-%, in der Regel 0 bis 0,5 Vol.%;

Gehalt an Methan ≤ 5 Vol.-%, meist ≤ 2 Vol.-%, in der Regel 0 bis 0,2 Vol.%;

Gehalt an Cyclopropan ≤ 0,1 Vol.-%;

Gehalt an Propylen ≤ 10 Vol.-%, meist ≤ 5 Vol.-%, in der Regel ≤ 2 Vol.-%;

Gesamtgehalt an von Propan und Propylen verschiedenen $C_3$-Kohlenwasserstoffen ≤ 0,3 Vol.-%;

Gesamtgehalt an $C_5$-Kohlenwasserstoffen ≤ 0,3 Vol.-%; und

Gesamtgehalt an $C_6$- bis $C_8$-Kohlenwasserstoffen ≤ 600 Vol.ppm.

Erfindungsgemäß geeignete Roh-Propane sind auch solche, die nicht nur die vorgenannten Spezifikationen, sondern gleichzeitig auch noch die folgenden Spezifikationen erfüllen:

Gesamtgehalt an Sauerstoff enthaltenden organischen Verbindungen ≤ 300 Vol.ppm;

Gehalt an Acetylen ≤ 30 Vol.ppm;

Gehalt an inogenem Chlor ≤ 1 mg/kg;

Gesamtgehalt an Cl enthaltenden Verbindungen und ausgedrückt als Cl ≤ 1 mg/kg;

Gesamtgehalt an F enthaltenden Verbindungen und ausgedrückt als F ≤ 1 mg/kg;

Gesamtgehalt an S enthaltenden Verbindungen und ausgedrückt als S ≤ 10 mg/kg (bei katalytischen Dehydrierungen kann es zweckmäßig sein, wenn das Reaktionsgasgemisch, bezogen auf darin enthaltenes Propan, 1 bis 1000 Vol.-ppm, bevorzugt 1 bis 100 Vol.-ppm, an Schwefel enthaltenden Verbindungen (z.B. $H_2S$ und/oder Dimethylsulfid) enthält, da diese einerseits Stahlbestandteile (des Reaktors) wie Ni, Cr und Fe zu passivieren (was ein unerwünschtes Cracken des Propans mindert) und andererseits die verwendeten Katalysatoren zu aktivieren vermögen (vgl. "Catalytic dehydrogenation of lower alkanes, Resasco, Daniel E.; Haller, Gary L., University Oklahoma, USA, Catalysis (1994), 11, 379-411");

mit der Maßgabe, dass der Gehalt an Buten-1 bevorzugt ≤ 0,1 Vol.-%, und besonders bevorzugt gleichzeitig der Gesamtgehalt an Butenen ≤ 0,1 Vol.-% und ganz besonders bevorzugt gleichzeitig der Gesamtgehalt an $C_4$-Kohlenwasserstoffen ≤ 3 Vol.-%, oder ≤ 2,5 Vol.-%, oder ≤ 2 Vol.-% beträgt.

Die unter der Maßgabe definierten Spezifikationen des Roh-Propan eignen sich für das erfindungsgemäße Verfahren in der Regel dann, wenn das Gasgemisch 1 als solches als Bestandteil eines Gasgemisches 2 einer heterogen katalysierten Gasphasen-Partialoxidation und/oder einer partiellen Gasphasen-Ammoxidation des im Gasgemisch 1 enthaltenen Propylen unterworfen wird. Dabei wirkt es sich vorteilhaft aus, dass im ersten Schritt des erfindungsgemäßen Verfahrens ein limitierter Oxidehydrier- und/oder Dehydrierumsatz insgesamt erfindungsgemäß günstig ist. In der Regel liegt dieser Umsatz, bei jedem einzelnen enthaltenen gesättigten Kohlenwasserstoff, bei Werten ≥ 5 mol.-%, aber ≤ 30 mol.-%, häufig ≤ 25 mol.-% und vielfach ≤ 20 mol.-%.

Normalerweise enthalten die vorstehend spezifizierten, (aber auch alle anderen) für das erfindungsgemäße Verfahren geeigneten, Roh-Propane wenigstens 0,25 Vol.-%, oder wenigstens 0,5 Vol.-%, oder wenigstens 1 Vol.-%, häufig wenigstens 1,5 Vol.-% oder wenigstens 2 Vol.-% und vielfach wenigstens 2,5 Vol.-% oder wenigstens 3 Vol.-% an von Propan und Propylen verschiedenen Bestandteilen (jedoch häufig ≤ 10 Vol.-%, meist ≤ 7 Vol.-% und in der Regel ≤ 5 Vol.-% dieser Bestandteile). Diese Fremdgehalte gelten meist auch für andere für das erfindungsgemäße Verfahren geeignete RohPropane, z.B. solche, die frei von $C_4$-Kohlenwasserstoffen sind. Diese können aber auch ≥ 0,1 Vol.-%, oder ≥ 0,5 Vol.-%, häufig bis zu 6 Vol.-% an $C_4$-Kohlenwasserstoffen enthalten (z.B. 0,1 oder 0,5 Vol.-% bis 6 Vol.-%). Außerdem und gleichzeitig können sie ≥ 5 Vol.ppm, häufig bis zu 0,5 Vol.-% an Butenen (z.B. 5 Vol.ppm bis 0,5 Vol.-%) enthalten. Ferner können sie außerdem gleichzeitig ≥ 5 Vol.ppm, häufig bis zu 0,5 Vol.-% an Buten-1 (z.B. 5 Vol.ppm bis 0,5 Vol.-%) enthalten.

Erfindungsgemäß besonders geeignete Roh-Propane sind auch solche, die nicht nur die vorgenannten Spezifikationen, sondern gleichzeitig auch noch die folgenden Spezifikationen erfüllen:

$$Ag \leq 1\ \mu g/kg;$$

Al $\leq$ 10 μg/kg;

As $\leq$ 1 μg/kg;

Au $\leq$ 1 μg/kg;

Ba $\leq$ 1 μg/kg;

Be $\leq$ 1 μg/kg;

Bi $\leq$ 1 μg/kg;

Ca $\leq$ 2 μg/kg;

Cd $\leq$ 1 μg/kg;

Co $\leq$ 1 μg/kg;

Cr $\leq$ 1 μg/kg;

Cu $\leq$ 1 μg/kg;

Fe $\leq$ 10 μg/kg;

Ga $\leq$ 1 μg/kg;

Ge $\leq$ 1 μg/kg;

Hg $\leq$ 1 μg/kg;

In $\leq$ 1 μg/kg;

Ir $\leq$ 1 μg/kg;

K $\leq$ 1 μg/kg;

Li $\leq$ 1 µg/kg;

Mg $\leq$ 1 µg/kg;

Mn $\leq$ 1 µg/kg;

Mo $\leq$ 1 µg/kg;

Na $\leq$ 1 µg/kg;

Nb $\leq$ 1 µg/kg;

Ni $\leq$ 1 µg/kg;

Pb $\leq$ 1 µg/kg;

Pd $\leq$ 1 µg/kg;

Pt $\leq$ 1 µg/kg;

Rh $\leq$ 1 µg/kg;

Sb $\leq$ 1 µg/kg;

Sn $\leq$ 1 µg/kg;

Sr $\leq$ 1 µg/kg;

Ta $\leq$ 1 µg/kg;

Ti $\leq$ 1 µg/kg;

Tl $\leq$ 1 µg/kg;

V $\leq$ 1 µg/kg;

Zn $\leq$ 1 µg/kg;

und

$$Zr \leq 1\ \mu g/kg.$$

Erfindungsgemäß ganz besonders geeignete Roh-Propane sind solche, die nicht nur gleichzeitig die vorgenannten Spezifikationen, sondern gleichzeitig auch noch die folgenden Spezifikationen erfüllen.

$$\text{Massendichte bei } 20^oC = 500 \pm 2{,}0\ kg/m^3;$$

$$\text{Dampfdruck bei } 20^oC = 7{,}6 \pm 0{,}2\ bar;$$

$$\text{Wasser} \leq 10\ mg/kg;$$

$$\text{Verdampfungsrückstand} \leq 2\ mg/kg.$$

Die angegebenen Spezifikationen beziehen sich auf Bestimmungen mittels Gaschromatographie und mittels Atomabsorptionsspektroskopie. Der Verdampfungsrückstand bezieht sich auf gravimetrische Bestimmung. Er besteht in der Regel aus hochsiedenden Kohlenwasserstoffen (z.B. green oil).

Nicht spezifizierte Bestandteile sind in den erfindungsgemäß besonders geeigneten Roh-Propanen bevorzugt nicht enthalten, d.h., nicht nachweisbar.

Von besonderer Bedeutung ist die erfindungsgemäße Verfahrensweise dann, wenn sie in Kreisfahrweise angewandt wird.

In diesem Fall wird aus dem Produktgasgemisch der Gasphasen-Partialoxidation und/oder -ammoxidation nach einem der bekannten Trennverfahren das gewünschte Zielprodukt abgetrennt, und wenigstens in diesem Produktgasgemisch enthaltenes nicht umgesetztes Propan, in der Regel gemeinsam mit darin enthaltenem nicht umgesetztem Propylen, in den Oxidehydrier- und/oder Dehydrierschritt und/oder in die Gasphasen-Partialoxidation und/oder -ammoxidation rückgeführt. Üblicherweise erfolgt diese Rückführung (Kreisführung) des Propans und Propylens als Bestandteil des nach der Zielproduktabtrennung verbleibenden Restgases, ohne dass selbiges zwischenbehandelt wird, oder, wenn es zwischenbehandelt wird (z.B. Abtrennung von darin enthaltenem CO, $CO_2$, $H_2$ und/oder $O_2$ vor Rückführung), wird diese Zwischenbehandlung nur mit begrenztem Aufwand betrieben. D.h., selbst wenn das eingesetzte Roh-Propan nur geringe Anteile an Buten-1, sonstigen Butenen und/oder sonstigen $C_4$-Kohlenwasserstoffen enthält (z.B. Gesamtgehalt an $C_4$-Kohlenwasserstoffen $\geq$ 0,01 Vol.-%, evtl. bis 6 Vol.-%), können sich diese im Rahmen einer Kreisgasfahrweise im Gasgemisch 2 aufpegeln und die erfindungsgemäßen Grenzwerte überschreiten, wenn nicht besondere Maßnahmen ergriffen werden. Diese Maßnahmen können z.B. darin bestehen, dass aus dem nach der Zielproduktabtrennung verbleibenden Restgas die $C_4$-Kohlenwasserstoffe rektifikativ und/oder durch Absorption/Desorption und/oder Strippung und/oder durch Adsorption/Desorption und/oder durch Kondensation und/oder durch Membranverfahren spezifisch abgetrennt werden und erst das danach verbleibende, Propan und Propylen enthaltende Restgas im Kreis gefahren wird.

Die EP-A 938463 erachtete einen solchen Trennschritt als nicht erforderlich, obwohl sie empfiehlt, das Gasgemisch 1 als solches für die Partialoxidation einzusetzen, und für den ersten Schritt Roh-Propane von im wesentlichen beliebiger Reinheit einzusetzen.

Alternativ zu einer Kreisgasfahrweise können die erwähnten Restgase zur Vermeidung von unerwünschten Aufpegelungen von $C_4$-Kohlenwasserstoffen auch anderen Verwendungszwecken zugeführt werden. Beispielsweise können sie samt des darin enthaltenen Propans und Propylens für Zwecke der Stromerzeugung verbrannt und/oder zur Herstellung von Synthesegas u.ä. verwendet werden.

Im übrigen kann das erfindungsgemäße Verfahren wie die verschiedenen im Stand der Technik beschriebenen Grundvarianten durchgeführt werden.

D.h., in der einfachsten Variante werden alle Schritte des erfindungsgemäßen Verfahrens in einer einzigen Reaktionszone und an einer in selbiger befindlichen Katalysatorbeschickung durchgeführt, wie es z.B. in den Schriften EP-A 608838, EP-A 529853, DE-A 19835247, EP-A 895809, JP-A 7-232071, JP-A 11-169716, EP-A 1192987, JP-A 10-57813, JP-A 2000-37623, JP-A 10-36311, WO 00/29105, EP-A 767164, DE-A 10029338, JP-A 8-57319, JP-A

10-28862, JP-A 11-43314, JP-A 11-574719, WO 00/29106, JP-A 10-330343, JP-A 11-285637, JP-A 310539, JP-A 11-42434, JP-A 11-343261, JP-A 3423262, WO 99/03825, JP-A 7-53448, JP-A 2000-51693, JP-A 11-263745, DE-A 10046672, DE-A 10118814, DE-A 10119933, JP-A 2000/143244, EP-A 318295, EP-A 603836, DE-A 19832033, DE-A 19836359, EP-A 962253, DE-A 10119933, DE-A 10051419, DE-A 10046672, DE-A 10033121, DE-A 101 459 58, DE-A 10122027, EP-A 1193240 und der in diesen Schriften zitierten Literatur gelehrt wird.

Bei der zu verwendenden Aktivmasse der Katalysatorbeschickung handelt es sich im wesentlichen um Multimetalloxidmassen, die die Elemente Mo, V, wenigstens eines der beiden Elemente Te und Sb, und wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthalten.

Bevorzugt enthält die Kombination dabei aus der letzten Elementgruppe die Elemente Nb, Ta, W und/oder Ti und besonders bevorzugt das Element Nb.

Bevorzugt enthalten die relevanten Multimetalloxidaktivmassen die vorgenannte Elementkombination in der Stöchiometrie I

$$Mo_1V_bM^1_cM^2_d \qquad (I),$$

mit

$M^1$ = Te und/oder Sb,
$M^2$ = wenigstens eines der Elemente aus der Gruppe umfassen Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,
b = 0,01 bis 1,
c = > 0 bis 1, und
d = > 0 bis 1.

Erfindungsgemäß bevorzugt ist $M^1$ = Te und $M^2$ = Nb, Ta, W und/oder Ti. Vorzugsweise ist $M^2$ = Nb.

Der stöchiometrische Koeffizient b beträgt mit Vorteil 0,1 bis 0.6. In entsprechender Weise beläuft sich der Vorzugsbereich für den stöchiometrischen Koeffizienten c auf 0,01 bis 1 bzw. auf 0,05 bis 0,4 und günstige Werte für d betragen 0,01 bis 1 bzw. 0,1 bis 0,6.

Erfindungsgemäß besonders günstig ist es, wenn die stöchiometrischen Koeffizienten b, c und d simultan in den vorgenannten Vorzugsbereichen liegen.

Das Vorgenannte gilt insbesondere dann, wenn die Aktivmasse der Katalysatorbeschickung hinsichtlich ihrer von Sauerstoff verschiedenen Elemente aus einer vorgenannten Elementkombination besteht.

Dies sind dann insbesondere die Multimetalloxidaktivmassen der allgemeinen Stöchiometrie II

$$Mo_1V_bM^1_cM^2_dO_n \qquad (II),$$

wobei die Variablen die bezüglich der Stöchiometrie I angeführte Bedeutung aufweisen und n = eine Zahl ist, die durch die Wertigkeit und Häufigkeit der von Sauerstoff verschiedenen Elemente in (II) bestimmt wird.

Ferner werden bei der Einzonenfahrweise für das erfindungsgemäße Verfahren bevorzugt solche Multimetalloxidaktivmassen eingesetzt,die einerseits entweder eine der vorgenannten Elementkombinationen enthalten oder, bezüglich der von Sauerstoff verschiedenen Elemente, aus ihr bestehen und gleichzeitig ein Röntgendiffraktogramm aufweisen, das Beugungsreflexe h und i zeigt, deren Scheitelpunkte bei den Beugungswinkeln (2Θ) 22,2 ± 0,5° (h) und 27,3 ± 0,5° (i) liegen (alle in dieser Schrift auf ein Röntgendiffraktogramm bezogenen Angaben beziehen sich auf ein unter Anwendung von Cu-Kα-Strahlung als Röntgenstrahlung erzeugtes Röntgendiffraktogramm (Siemens-Diffraktometer Theta-Theta D-5000, Röhrenspannung: 40 kV, Röhrenstrom : 40mA" Aperturblende V20 (variabel), Streustrahlblende V20 (variabel), Sekundärmonochromatorblende (0,1 mm), Detektorblende (0,6 mm), Meßintervall (2Θ) : 0,02°, Meßzeit je Schritt : 2,4s, Detektor : Scintillationszählrohr).

Die Halbwertsbreite dieser Beugungsreflexe kann dabei sehr klein oder auch sehr ausgeprägt sein.

Besonders bevorzugt sind für das erfindungsgemässe Verfahren diejenigen der vorgenannten Multimetalloxidaktivmassen, deren Röntgendiffraktogramm zusätzlich zu den Beugungsreflexen h und i einen Beugungsreflexes k aufweist, dessen Scheitelpunkt bei 28,2 ± 0,5° (k) liegt.

Unter den letzeren sind wiederum jede erfindungsgemäß bevorzugt, bei denen der Beugungsreflex h innerhalb des Röntgendiffraktogramms der intensitätsstärkste ist, sowie eine Halbwertsbreite von höchstens 0,5° aufweist, und ganz besonders bevorzugt sind jene, bei denen die Halbwertsbreite des Beugungsreflexes i und des Beugungsreflexes k gleichzeitig jeweils ≤ 1° beträgt und die Intensität $P_k$ des Beugungsreflexes k und die Intensität $P_i$ des Beugungsreflexes i die Beziehung 0,2 ≤ R ≤ 0,85, besser 0,3 ≤ R ≤ 0,85, bevorzugt 0,4 ≤ R ≤ 0,85, besonders bevorzugt 0,65 ≤ R ≤ 0,85, noch mehr bevorzugt 0,67 ≤ R ≤ 0,75 und ganz besonders bevorzugt R = 0,70 bis 0,75 bzw. R = 0,72 erfüllen, in der R

das durch die Formel

$$R = P_i/(P_i+P_k)$$

definierte Intensitätsverhältnis ist. Bevorzugt weisen die vorgenannten Röntgendiffraktogramme keinen Beugungsreflex auf, dessen Maximum bei 2 ⊖ = 50 ± 0,3° liegt.

Die Definition der Intensität eines Beugungsreflexes im Röntgendiffraktogramm bezieht sich in dieser Schrift auf die in der DE-A 19835247, der DE-A 10122027, sowie die in der DE-A 10051419 und DE-A 10046672 niedergelegte Definition. Das gleiche gilt für die Definition der Halbwertsbreite.

Neben den Beugungsreflexen h, i und k enthalten die vorgenannten Röntgendiffraktogramme von erfindungsgemäß vorteilhaft einzusetzenden Multimetalloxidaktivmassen noch weitere Beugungsreflexe, deren Scheitepunkte bei den nachfolgenden Beugungswinkeln (2 ⊖) liegen:

9,0 ± 0,4° (1)
6,7 ± 0,4° (o) und
7, 9 ± 0,4° (p).

Günstig ist es ferner, wenn das Röntgendiffraktogramm zusätzlich einen Beugungsreflex enthält, dessen Scheitelpunkt beim Beugungswinkel (2 ⊖) = 45,2 ± 0,4° (q) liegt.

Häufig enthält das Röntgendiffraktogramm auch noch die Reflexe 29,2 ± 0,4° (m) und 35,4 ± 0,4° (n).

Es ist ferner günstig, wenn die in den Formeln I und II definierten Elementkombinationen als reine i-Phase vorliegen. Enthält die katalytisch aktive Oxidmasse auch noch k-Phase, enthält ihr Röntgendiffraktogramm neben den oben genannten noch weitere Beugungsreflexe, deren Scheitelpunkte bei den nachfolgenden Beugungswinkeln (2 ⊖) liegen: 36,2 ± 0,4° und 50 ± 0,4° (die Begriffe i- und k-Phase werden in dieser Schrift wie in der DE-A 10122027 und DE-A 10119933 festgelegt verwendet).

Ordnet man dem Beugungsreflex h die Intensität 100 zu, ist es erfindungsgemäß günstig, wenn die Beugungsreflexe i, 1, m, n, o, p, q in der gleichen Intensitätsskala die nachfolgenden Intensitäten aufweisen:

i: 5 bis 95, häufig 5 bis 80, teilweise 10 bis 60;
1: 1 bis 30;
m: 1 bis 40;
n: 1 bis 40;
o: 1 bis 30;
p: 1 bis 30 und
q: 5 bis 60.

Enthält das Röntgendiffraktogramm von den vorgenannten zusätzlichen Beugungsreflexen, ist die Halbwertsbreite derselben in der Regel ≤ 1°.

Die spezifische Oberfläche von erfindungsgemäß zu verwendenden Multimetalloxidaktivmassen der allgemeinen Formel II oder von Multimetalloxidaktivmassen, die Elementkombinationen der allgemeinen Formel I enthalten beträgt vielfach 1 bis 30 $m^2/g$ (BET-Oberfläche, Stickstoff), vor allem dann, wenn ihr Röntgendiffraktogramm wie beschrieben gestaltet ist.

Die Herstellung der beschriebenen Multimetalloxidaktivmassen findet sich im im Zusammenhang mit diesen zitierten Stand der Technik. Dazu zählen insbesondere die DE-A 10122027, die DE-A 10119933, DE-A 10033121, die EP-A 1192987, die DE-A 10029338, die JP-A 2000-143244, die EP-A 962253, die EP-A 895809, die DE-A 19835247, die WO 00/29105, die WO 00/29106, die EP-A 529853 und die EP-A 608838 (in allen Ausführungsbeispielen der beiden letztgenannten Schriften ist als Trocknungsmethode die Sprühtrocknung anzuwenden; z.B. mit einer Eingangstemperatur von 300 bis 350°C und einer Ausgangstemperatur von 100 bis 150°C; Gegenstrom oder Gleichstrom).

Die beschriebenen Multimetalloxidativmassen können als solche (d.h. in Pulverform) oder zu geeigneten Geometrien geformt (vgl. z.B. die Schalenkatalysatoren der DE-A 10051419 sowie die geometrischen Varianten der DE-A 10122027) für die Einzonengestaltung des erfindungsgemäßen Verfahrens eingesetzt werden. Sie eignen sich insbesondere zur Herstellung von Acrolein und/oder Acrylsäure sowie zur Herstellung von Acrylnitril.

Basis für die Einzonenfahrweise ist, daß die zu verwendenden Katalysatoren alle Schritte des erfindungsgemäßen Verfahrens zu katalysieren in der Lage sind.

Sie kann sowohl in einem Katalysatorfestbett oder in einem Katalysatorwirbelbett bzw. Fließbett (Wanderbett) durchgeführt werden. Entsprechende Verfahrensbeschreibungen finden sich in den Schriften des Standes der Technik.

Wird das erfindungsgemäße Verfahren z.B. zur Herstellung von Acrylsäure in der Einzonenfahrweise als Festbettreaktion ausgeführt, erfolgt die Durchführung in zweckmäßiger Weise in einem Rohrbündelreaktor, dessen Kontaktrohre mit dem Katalysator beschickt sind. Um die Kontaktrohre wird im Normalfall als Wärmeträger eine Flüssigkeit, in der Regel eine Salzschmelze geführt. Alternativ kann auch ein Thermoplattenreaktor eingesetzt werden, wobei sich die Katalysatorbeschickung als ebene Anordnung zwischen Kühlplatten befindet.

Das Reaktionsgasgemisch wird in den Kontaktrohren über den Reaktor betrachtet entweder im Gleichstrom oder im Gegenstrom zum Salzbad geführt. Das Salzbad selbst kann relativ zu den Kontaktrohren eine reine Parallelströmung ausführen. Selbstverständlich kann dieser aber auch eine Querströmung überlagert sein. Insgesamt kann das Salzbad um die Kontaktrohre auch eine mäanderförmige Strömung ausführen, die nur über den Reaktor betrachtet im Gleichstrom oder im Gegenstrom zum Reaktionsgasgemisch geführt ist. Für das erfindungsgemäße Verfahren geeignete Rohrbündelreaktoren offenbaren z.B. die Schriften EP-A 700714 und EP-A 700893.

Die verschiedenen möglichen Zusammensetzungen des Reaktionsgasausgangsgemisches für die Einzonenvariante des erfindungsgemäßen Verfahrens können dem im Zusammenhang mit dieser Verfahrensvariante zitierten Stand der Technik entnommen werden.

Für die Herstellung von Acrylsäure bewegt sich die Zusammensetzung des Reaktionsgasausangsgemisches in typischer Weise innerhalb des folgenden Rahmens (molare Verhältnisse):

Propan: Sauerstoff : $H_2O$: sonstige Bestandteile (vor allem inerte Verdünnungsgase) =
1 : (0,1-10) : (>0-50) : (>0-50).

Vorzugsweise beträgt das vorgenannte Verhältnis
1 : (0,5-5) : (1-30) : (1-30).

Die vorgenannten Bereiche gelten insbesondere dann, wenn als sonstige Bestandteile überwiegend molekularer Stickstoff eingesetzt wird. Die Reaktionstemperatur beträgt typisch 250 bis 550°C (die Bedingungen für die Ammoxidation sind vergleichbar, sieht man davon ab, daß das Reaktionsgasgemisch zusätzlich Ammoniak umfaßt (vgl. z.B. EP-A 929853).

Die Belastung einer Festbettkatalysatorbeschickung mit Propan kann bei der Einzonenvariante des erfindungsgemäßen Verfahrens z.B. 10 bis 500 Nl/l (Festbett) ·h betragen. Die Belastung mit Reaktionsgasausgangsgemisch liegt häufig im Bereich von 100 bis 10000 Nl/l·h, vielfach im Bereich 500 bis 5000 Nl/l·h.

Aus dem resultierenden Produktgasgemisch kann das Zielprodukt, z.B. Acrylsäure, in an sich bekannter, wie z.B. in der DE-A 10122027 beschriebener, Weise abgetrennt werden. D.h., aus dem Produktgasgemisch kann die enthaltene Acrylsäure z.B. durch die Absorption mit einem hochsiedenden inerten hydrophoben organischen Lösungsmittel (z.B. einem Gemisch aus Diphenylether und Diphenyl das gegebenenfalls noch Zusätze wie Dimethylphthalat enthalten kann) aufgenommen werden. Das dabei resultierende Gemisch aus Absorbens und Acrylsäure kann anschließend in an sich bekannter Weise rektifikativ, extraktiv und/oder kristallisativ bis zur Reinacrylsäure aufgearbeitet werden. Alternativ kann die Grundabtrennung der Acrylsäure aus dem Produktgasgemisch auch durch fraktionierende Kondensation erfolgen, wie es z.B. in der DE-A 10053086, DE-A 19627847, DE-A 19740253, DE-A 19740252, DE-A 19606877 und DE-A 19740253 beschrieben ist. Das dabei resultierende Acrylsäurekondensat kann dann z.B. durch fraktionierte Kristallisation (z.B. Suspensionskristallisation und/oder Schichtkristallisation) weitergereinigt werden.

Das bei der Grundabtrennung der Acrylsäure verbleibende Restgasgemisch enthält insbesondere nicht umgesetztes Propan und gegebenenfalls nicht umgesetztes Propylen.

Je nach dem Gehalt dieses Restgasgemisches an Buten-1, an Gesamtbutenen und an Gesamt-$C_4$-Kohlenwasserstoffen und verwendeter Sauerstoffquelle (ob reiner Sauerstoff, ein Sauerstoff enthaltendes Inertgas oder Luft), kann das Restgasgemisch als solches rückgeführt werden. Gegebenenfalls wird man es auch in zwei Teile identischer Zusammensetzung aufteilen und nur die eine Teilmenge rückführen und die andere Teilmenge purgen (z.B. seiner Verbrennung oder einer anderweitigen Verwertung (z.B. Synthesegasherstellung zuführen)). Letzteres könnte selbstredend auch mit der Gesamtmenge des Restgasgemisches erfolgen.

Bei erhöhten Anteilen der erfindungsgemäß unerwünschten $C_4$-Komponenten und/oder erhöhten Anteilen sonstiger unerwünschter Komponenten im Restgasgemisch kann das im Restgasgemisch enthaltene Propan und gegebenenfalls Propen, z.B. durch fraktionierte Druckrektifikation abgetrennt (der Trennfaktor kann entsprechend gewählt werden) und anschließend in das erfindungsgemäße Verfahren rückgeführt und mit dem Roh-Propan und sonstigen Bestandteilen des Reaktionsgasausgangsgemischs vereinigt werden. Gegebenenfalls ist es jedoch unter erfindungsgemäßen Gesichtspunkten bereits ausreichend, das Restgas in einer Extraktionsvorrichtung mit einem vorzugsweise $C_3$-Kohlenwasserstoffe absorbierenden hydrophoben organischen Lösungsmittel in Kontakt zu bringen (z.B. durch selbiges durchleiten). Durch nachfolgende Desorption und/oder Strippung mit Luft (die als Sauerstoffquelle sowieso benötigt würde) kann das absorbierte Propan und gegebenenfalls Propen wieder freigesetzt und in das erfindungsgmeäße Verfahren rückgeführt werden. Selbstverständlich könnte die Acrylsäure aber auch nach der in der DE-A 10059122

beschriebenen Verfahrensweise aus dem Produktgemisch abgetrennt werden. Die für die Einzonenfahrweise empfohlenen Multimetalloxidaktivmassen können selbstredend auch in mit feinteiligen, z.B. kolloidalen, Materialien wie Silicumdioxid, Titandioxid, Aluminiumoxid, Zirkonoxid und Nioboxid verdünnter Form im erfindungsgemäßen Verfahren eingesetzt werden.

Das Verdünnungsmassenvehrältnis kann dabei bis zu 9 (Verdünner) : 1 (Aktivmasse) betragen. D.h., mögliche Verdünnungsmassenverhältnisse betragen z.B. 6 (Verdünner) : 1 (Aktivmasse) und 3 (Verdünner) : 1 (Aktivmasse). Die Einarbeitung der Verdünner kann gemäß der DE-A 10122027 dabei vor oder nach der Calcination erfolgen. Selbstverständlich können für die erfindungsgemäße Einzonenfahrweise aber auch andere Katalysatorsysteme eingesetzt werden, wie sie z.B. die JP-A 3-170445 beschreibt.

Wird das erfindungsgemäße Verfahren in einer Reaktionszone verwirklicht, liegt einer der Fälle vor, bei denen das Gasgemisch 1 und das Gasgemisch 2 identisch sind. Vom erfindungsgemäßen Verfahren wird dabei insbesondere dann Gebrauch gemacht, wenn im Produktgasgemisch des erfindungsgemäßen Verfahrens die erfindungsgemäßen Gehaltsgrenzen für Buten-1 sowie anderer C4-Kohlenwasserstoffe überschritten sind.

Erfindungsgemäß bevorzugt wird das erfindungsgemäße Verfahren in mehr als einer Reaktionszone verwirklicht, wie es z.B. die EP-A 938463, die EP-A 117146, die DE-A 3313573, die GB-A 2118939, die US-A 3161670, die WO 01/96270, die EP-A 731077, die DE-A 19837520, die DE-A 19837517, die DE-A 19837519, die DE-A 19837518, die DE-A 19837520, die DE-A 10131297 und die DE-A 10211275 beschreiben.

Mehr als eine Reaktionszone meint dabei in erster Linie, daß wenigstens ein Schritt des erfindungsgemäßen Verfahrens unter Bedingungen durchgeführt wird, die wenigstens teilweise unabhängig von jenen des wenigstens einen anderen Schrittes innerhalb des erfindungsgemäßen Verfahrens gewählt werden können, oder, allerdings nur in zweiter Linie, daß innerhalb ein und desselben Schritts längs des Rektionspfads wenigstens teilweise voneinander unabhängige Reaktionsbedingungen verwirklicht werden (letzteres ist z.B. dann der Fall, wenn für einen Schritt sogenannte Mehrzonenfahrweisen (mit voneinander unabhängig einstellbaren Temperaturzonen) angewendet werden, wie es z.B. DE-A 19948241, die DE-A 19927624, die DE-A 19910508, die DE-A 19910506 und die DE-A 19948248 beschreiben). D.h., umfaßt das erfindungsgemäße Verfahren z.B. zwei Schritte, so könnte für den ersten Schritt z.B. ein anderer Katalysator bzw. eine andere Katalysatorbeschickung eingesetzt werden als für den zweiten Schritt. Oder man könnte so vorgehen, daß bei Verwendung identischer Katalysatoren bzw. Katalysatorbeschickungen für beide Schritte die Reaktionstemperaturen für die beiden Schritte unabhängig voneinander gewählt und eingestellt werden können. Natürlich kann auch beides überlagert angewendet werden.

Der Vorteil der Mehrzonenfahrweise liegt darin begründet, dass sie prinzipiell eine verbesserte Anpassung der Reaktionsbedingungen an die Erfordernisse der einzelnen Schritte des erfindungsgemäßen Verfahrens ermöglichen.

Dieser Vorteil ist von der heterogen katalysierten Gasphasen-Partialoxidation von Propylen zu Acrylsäure mit molekularem Sauerstoff wohlbekannt.

Sie verläuft längs der Reaktionskoordinate prinzipiell in zwei längs der Reakionskoordinate aufeinanderfolgenden Schritten ab, von denen der erste zum Acrolein und der zweite vom Acrolein zur Acrylsäure führt.

Dieser Reaktionsablauf eröffnet in an sich bekannter Weise die Möglichkeit, die erfindungsgemäße Partialoxidation des im Gasgemisch 2 enthaltenen Propylen in zwei hintereinander angeordneten Oxidationszonen auszuführen, wobei in jeder der beiden Oxidationszonen der zu verwendende oxidische Katalysator in optimierender Weise angepaßt werden kann (diese Anpassungsmöglichkeit gestattet es auch, die Partialoxidation des Propylens auf der Höhe des Acroleins zu stoppen und das Acrolein zu isolieren). So wird für die erste Oxidationszone (Propylen $\rightarrow$ Acrolein) in der Regel ein Katalysator auf der Basis von die Elementkombination Mo-Bi-Fe enthaltenden Multimetalloxiden bevorzugt, während für die zweite Oxidationszone (Acrolein $\rightarrow$ Acrylsäure) normalerweise Katalysatoren auf der Basis von die Elementkombination Mo-V enthaltenden Multimetalloxiden bevorzugt werden (z.B. auch jene, die in dieser Schrift für die Einzonenfahrweise empfohlen wurden). Prinzipiell können aber diese beiden Reatkionsschritte in einer einzigen Reaktionszone und an einem einzigen Katalysator durchgeführt werden.

Ganz generell wird man beim erfindungsgemäßen Verfahren in zweckmäßiger Weise den ersten Schritt in einer getrennten Reaktionszone duchführen.

Im Fall einer Oxidehydrierung des Propan kann diese als homogene und/oder heterogen katalysierte Oxidehydrierung von Propan zu Propylen mit molekularem Sauerstoff in der Gasphase durchgeführt werden. Als Quelle des molekularen Sauerstoff kann dabei Luft, reiner molekularer Sauerstoff oder an molekularem Sauerstoff angereicherte Luft eingesetzt werden.

Gestaltet man die Reaktionszone als eine homogene Oxidehydrierung, so läßt sich diese prinzipiell so durchführen, wie es z.B. in den Schriften US-A 3,798,283, CN-A 1 105 352, Applied Catalysis, 70(2)1991, S. 175-187, Catalysis Today 13, 1992, S. 673-678 und in der Anmeldung DE-A 19 622 331 beschrieben ist. Eine zweckmäßige Sauerstoffquelle ist Luft. Die Temperatur der homogenen Oxidehydrierung wird zweckmäßigerweise im Bereich von 300 bis 700°C, vorzugsweise im Bereich von 400 bis 600°C, besonders bevorzugt im Bereich von 400 bis 500°C liegend gewählt. Der Arbeitsdruck kann 0,5 bis 100 bar, insbesondere 1 bis 10 bar betragen. Die Verweilzeit liegt üblicherweise bei 0,1 beziehungsweise 0,5 bis 20 Sekunden, vorzugsweise bei 0,1 beziehungsweise 0,5 bis 5 Sekunden.

Als Reaktor kann zum Beispiel ein Rohrofen oder ein Rohrbündelreaktor verwendet werden, wie zum Beispiel ein Gegenstrom-Rohrofen mit Rauchgas als Wärmeträger oder ein Rohrbündelreaktor mit Salzschmelze als Wärmeträger. Das Propan zu Sauerstoff-Verhältnis im Ausgangsgemisch beträgt vorzugsweise 0,5:1 bis 40:1, insbesondere zwischen 1:1 bis 6:1, stärker bevorzugt zwischen 2:1 bis 5:1. Das Ausgangsgemisch kann auch weitere, bevorzugt inerte (unter inerten Bestandteilen sollen in dieser Schrift ganz generell vorzugsweise solche Bestandteile verstanden werden, die sich beim relevanten Reaktionsschritt zu weniger als 5 mol-%, bevorzugt zu weniger als 3 mol.-% und besonders bevorzugt zu weniger als 1 mol.-% umsetzen; ganz besonders bevorzugt setzten sie sich überhaupt nicht um), Bestandteile, wie Wasser, Kohlendioxid, Kohlenmonoxid, Stickstoff, Edelgase, andere Kohlenwasserstoffe (z.B. im Roh-Propan enthaltene Nebenbestandteile), und/oder Propylen etc. umfassen, wobei es sich hierbei auch um zurückgeführte (Kreisgas) Bestandteile handeln kann.

Gestaltet man die Propandehydrierung als eine heterogen katalysierte Oxidehydrierung, so läßt sich diese prinzipiell durchführen wie beschrieben zum Beispiel in den Schriften US-A 4 788 371, CN-A 1073893, Catalysis Letters 23 (1994), 103-106, W. Zhang, Gaodeng Xuexiao Huaxue Xuebao, 14 (1993) 566, Z. Huang, Shiyou Huagong, 21 (1992) 592, WO 97/36849, DE-A 197 53 817, US-A 3 862 256, US-A 3 887 631, DE-A 195 30 454, US-A 4 341 664, J. of Catalysis 167, 560-569 (1997), J. of Catalysis 167, 550-559 (1997), Topics in Catalysis 3 (1996) 265-275, US-A 5 086 032, Catalysis Letters 10 (1991), 181-192, Ind. Eng. Chem. Res. 1996, 35, 14-18, US-A 4 255 284, Applied Catalysis A: General, 100 (1993), 111-130, J. of Catalysis 148, 56-67 (1994), V. Cortes Corberän und S. Vic Bellön (Ed.), New Developments in Selective Oxidation II, 1994, Elsevier Science B.V., S. 305-313, 3rd World Congress on Oxidation Catalysis, R.K. Grasselli, S.T. Oyama, A.M. Gaffney and J.E. Lyons (Ed.), 1997, Elsevier Science B.V., S. 375ff oder in DE-A 19837520, DE-A 19837517, DE-A 19837519 und DE-A 19837518. Dabei kann als Sauerstoffquelle auch Luft eingesetzt werden. Häufig weist jedoch die Sauerstoffquelle hier zu mindestens 90 mol-% an molekularem Sauerstoff, und vielfach wenigsten 95 mol-% Sauerstoff auf.

Die für die heterogen katalysierte Oxidehydrierung geeigneten Katalysatoren unterliegen keinen besonderen Beschränkungen. Es eignen sich alle dem Fachmann auf diesem Gebiet bekannten Oxidehydrierungskatalysatoren, die in der Lage sind, Propan zu Propylen zu oxidieren. Insbesondere können alle in den zuvor genannten Schriften genannten Oxidehydrierungskatalysatoren eingesetzt werden. Geeignete Katalysatoren sind beispielsweise Oxidehydrierungskatalysatoren, die MoVNb-Oxide oder Vanadylpyrophosphat, gegebenenfalls mit Promotor, umfassen. Ein Beispiel für einen günstigen Oxidehydrierkatalysator ist ein Katalysator, wie er auch für die Einzonenfahrweise empfohlen wurde, der ein Mischmetalloxid mit Mo, V, Te, O und X als wesentliche Bestandteile enthält, wobei X mindestens ein Element ist, ausgewählt aus Niob, Tantal, Wolfram, Titan, Aluminium, Zirkonium, Chrom, Mangan, Gallium, Eisen, Ruthenium, Kobalt, Rhodium, Nickel, Palladium, Platin, Antimon, Bismut, Bor, Indium, Silizium, Lanthan, Natrium, Lithium, Kalium, Magnesium, Silber, Gold und Cer (vgl. dazu auch EP-A 938463 und EP-A 167109). Weiterhin besonders geeignete Oxidehydrierungskatalysatoren sind die Multimetalloxidmassen beziehungsweise -katalysatoren A der DE-A-197 53 817 und die Katalysatoren der DE-A 19838312, wobei die in der erstgenannten Schrift als bevorzugt genannten Multimetalloxidmassen beziehungsweise -katalysatoren A ganz besonders günstig sind. Das heißt, als Aktivmassen kommen insbesondere Multimetalloxidmassen der allgemeinen Formel III

$$M^1_aMo_{1-b}M^2_bO_x \qquad (III),$$

wobei

$M^1$ = Co, Ni, Mg, Zn, Mn und/oder Cu,
$M^2$ = W, V, Te, Nb, P, Cr, Fe, Sb, Ce, Sn und/oder La,
a = 0,5-1,5
b = 0-0,5

sowie
x = eine Zahl, die durch die Wertigkeit und Häufigkeit der von Sauerstoff verschiedenen Elemente in (III) bestimmt wird, in Betracht.

Prinzipiell können geeignete Aktivmassen (III) in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 450 bis 1000°C calciniert. Als Quellen für die elementaren Konstituenten der Multimetalloxid-Aktivmassen (III) kommen solche Verbindungen in Betracht, bei denen es sich bereits um Oxide handelt und/oder solche Verbindungen, die durch Erhitzen, wenigstens in Anwesenheit von Sauerstoff, in Oxide überführbar sind. Hierbei handelt es sich vom allem um Halogenide, Nitrate, Formiate, Oxalate, Citrate, Acetate, Carbonate, Amminkomplexsalze, Ammoniumsalze und/oder Hydroxide. Das innige Vermischen der Ausgangsverbindungen zur Herstellung der Multimetalloxidmassen (III) kann in trockener Form, zum Beispiel als feinteiliges Pulver, oder in nasser Form, zum Beispiel mit Wasser als Lösungsmittel, erfolgen. Die Multimetalloxidmassen (III) können sowohl in Pulverform als auch zu bestimmten Katalysatorgeometrien geformt, eingesetzt werden, wobei die Formgebung vor oder nach der abschließenden Calcinierung erfolgen kann. Es können Vollkatalysatoren eingesetzt werden. Die Formgebung einer pulverförmigen Aktivmasse beziehungsweise Vorläufermasse kann aber auch durch Aufbringung auf vorgeformte inerte Katalysatorträger erfolgen. Als Trägermaterialien können dabei übliche, poröse oder unporöse Aluminiumoxide, Siliciumdioxid, Thoriumdioxid, Zirkondioxid, Siliciumcarbid oder Silikate verwendet werden, wobei die Trägerkörper regelmäßig oder unregelmäßig geformt sein können.

Für die heterogen katalysierte Oxidehydrierung des Propans liegt die Reaktionstemperatur vorzugsweise im Bereich von 200 bis 600°C, insbesondere im Bereich von 250 bis 500°C, stärker bevorzugt im Bereich von 350 bis 440°C. Der Arbeitsdruck liegt vorzugsweise im Bereich von 50 bis 1000 kPa (0,5 bis 10 bar), insbesondere von 100 bis 1000 kPa (1 bis 10 bar), stärker bevorzugt von 100 bis 500 KPa (1 bis 5 bar). Arbeitsdrucke oberhalb von 100 kPa (1 bar), zum Beispiel von 150 bis 1000 kPa (1,5 bis 10 bar), haben sich als besonders vorteilhaft erwiesen. In der Regel erfolgt die heterogen katalysierte Oxidehydrierung des Propans an einem Katalysatorfestbett. Letzteres wird zweckmäßigerweise in den Rohren eines Rohrbündelreaktors aufgeschüttet, wie sie zum Beispiel in der EP-A-0 700 893 und in der EP-A-0 700 714 sowie der in diesen Schriften zitierten Literatur beschrieben sind. Die mittlere Verweilzeit des Reaktionsgasgemisches in der Katalysatorschüttung liegt zweckmäßigerweise bei 0,5 bis 20 Sekunden. Das Verhältnis von Propan zu Sauerstoff variiert mit dem gewünschten Umsatz und der Selektivität des Katalysators, zweckmäßigerweise liegt es im Bereich von 0,5:1 bis 40:1, insbesondere von 1:1 bis 6:1, stärker bevorzugt von 2:1 bis 5:1. In der Regel nimmt die Propylenselektivität mit steigendem Propanumsatz ab. Vorzugsweise wird deshalb die Propan zu Propylen-Reaktion so durchgeführt, daß relativ niedrige Umsätze mit Propan bei hohen Selektivitäten zu Propylen erreicht werden. Besonders bevorzugt liegt der Umsatz an Propan im Bereich von 5 bis 40 mol-%, häufig im Bereich von 10 bis 30 mol-%. Hierbei bedeutet der Begriff "Propanumsatz" den Anteil an zugeführtem Propan (Summe aus im Roh-Propan und gegebenenfalls rückgeführtem Kreisgas enthaltenem Propan das beim einfachen Durchgang umgesetzt wird). In der Regel beträgt die Selektivität der Propylenbildung 50 bis 98 mol-%, stärker bevorzugt 80 bis 98 mol-%, wobei der Begriff "Selektivität" die Mole an Propylen bezeichnet, die pro Mol umgesetztem Propan erzeugt werden, ausgedrückt als molarer Prozentsatz.

In der Regel enthält das bei der oxidativen Propandehydrierung eingesetzte Ausgangsgemisch 5 bis 95 mol-% Propan (bezogen auf 100 mol-% Ausgangsgemisch). Neben Propan und Sauerstoff kann das Ausgangsgemisch für die heterogen katalysierte Oxidehydrierung auch weitere, insbesondere inerte, Bestandteile wie Kohlendioxid, Kohlenmonoxid, Stickstoff, Edelgase, andere Kohlenwasserstoffe, z.B. im Roh-Propan enthaltene Nebenbestandteile, und/oder Propylen umfassen. Die heterogene Oxidehydrierung kann auch in Anwesenheit von Verdünnungsmitteln, wie zum Beispiel Wasserdampf, durchgeführt werden.

Jede beliebige Reaktorsequenz kann zur Durchführung der homogenen Oxidehydrierung oder der heterogen katalysierten Oxidehydrierung des Propans eingesetzt werden, die dem Fachmann bekannt ist. Zum Beispiel kann die Oxidehydrierung in einem einzigen Reakor oder in einer Kaskade aus zwei oder mehreren Reaktoren, zwischen denen gegebenenfalls Sauerstoff eingeführt wird, durchgeführt werden. Es besteht auch die Möglichkeit, die homogene und die heterogen katalysierte Oxidehydrierung miteinander kombiniert zu praktizieren.

Als mögliche Bestandteile kann das Produktgemisch einer erfindungsgemäßen Propanoxidehydrierung zum Beispiel folgende Komponenten enthalten: Propylen, Propan, Kohlendioxid, Kohlenmonoxid, Wasser, Stickstoff, Sauerstoff, Ethan, Ethen, Methan, Acrolein, Acrylsäure, Ethylenoxid, Butan (z.B. n-Butan oder iso-Butan), Essigsäure, Formaldehyd, Ameisensäure, Propylenoxid und Butene (z.B. Buten-1). In typischer Weise enthält ein bei der erfindungsgemäßen Propanoxidehydrierung erhaltenes Produktgemisch: 5 bis 10 mol-% Propylen, 0,1 bis 2 mol-% Kohlenmonoxid, 1 bis 3 mol-% Kohlendioxid, 4 bis 10 mol.-% Wasser, 0 bis 1 mol.-% Stickstoff, 0,1 bis 0,5 mol-% Acrolein, 0 bis 1 mol.-% Acrylsäure, 0,05 bis 0,2 mol-% Essigsäure, 0,01 bis 0,05 mol-% Formaldehyd, 1 bis 5 mol-% Sauerstoff, 0,1 bis 1,0 mol-% weitere oben genannte Komponenten, sowie als Rest im wesentlichen Propan, jeweils bezogen auf 100 mol-% Produktgemisch.

Generell kann die Propandehydrierung in der ersten Reaktionszone auch als eine heterogen katalysierte Propandehydrierung unter weitgehendem Sauerstoffausschluß wie in der DE-A 3313573, in der WO 01/96270, der DE-A 10131297 oder der DE-A 10211275 beschrieben oder wie folgt durchgeführt werden.

Da die heterogen katalysierte Dehydrierreaktion unter Volumenzunahme abläuft, kann der Umsatz durch Erniedrigung des Partialdrucks der Produkte gesteigert werden. Dies läßt sich in einfacher Weise, zum Beispiel durch Dehydrierung bei vermindertem Druck und/oder durch Zumischen von im wesentlichen inerten Verdünnungsgasen, wie zum Beispiel Wasserdampf, erreichen, der für die Dehydrierreaktion im Normalfall ein Inertgas darstellt. Eine Verdünnung mit Wasserdampf bedingt als weiteren Vorteil in der Regel ein vermindertes Verkoken des verwendeten Katalysators, da der Wasserdampf mit gebildetem Koks nach dem Prinzip der Kohlevergasung reagiert. Außerdem kann Wasserdampf als Verdünnungsgas in der nachfolgenden wenigstens einen Oxidations- und/oder Ammoxidationszone (in dieser Schrift als Kurzbezeichnung auch wenigstens eine Partialzone) mitverwendet werden. Wasserdampf läßt sich aber auch in einfacher Weise teilweise oder vollständig aus dem Produktgemisch der Dehydrierung abtrennen (zum Beispiel durch Kondensieren), was die Möglichkeit eröffnet, bei der Weiterverwendung des dabei erhältlichen modifizierten Produktgemisches in der wenigstens eine Partialzone den Anteil des Verdünnungsgases $N_2$ zu erhöhen. Weitere für die heterogen katalysierte Propandehydrierung geeignete Verdünnungsmittel sind zum Beispiel CO, Methan, Ethan, $CO_2$, Stickstoff und Edelgase wie He, Ne und Ar. Alle genannten Verdünnungsmittel können entweder für sich oder in Form unterschiedlichster Gemische mitverwendet werden. Es ist von Vorteil, dass die genannten Verdünnungsmittel in der Regel auch in der wenigstens einen Partialzone geeignete Verdünnungsmittel sind. Generell sind, wie bereits gesagt, sich in der jeweiligen Reaktionszone inert verhaltende (das heißt zu weniger als 5 mol-%, bevorzugt zu weniger als 3 mol-% und noch besser zu weniger als 1 mol-% sich chemisch verändernde) Verdünnungsmittel bevorzugt. Prinzipiell kommen für die heterogen katalysierte Propandehydrierung alle im Stand der Technik bekannten Dehydrierkatalysatoren in Betracht. Sie lassen sich grob in zwei Gruppen unterteilen. Nämlich in solche, die oxidischer Natur sind (zum Beispiel Chromoxid und/oder Aluminiumoxid) und in solche, die aus wenigstens einem auf einem, in der Regel oxidischen, Träger abgeschiedenen, in der Regel vergleichsweise edlen, Metall (zum Beispiel Platin) bestehen.

Unter anderem können damit alle Dehydrierkatalysatoren eingesetzt werden, die in der WO 01/96270, der EP-A 731077, der DE-A 10211275, der DE-A 10131297, der WO 99/46039, der US-A 4 788 371, der EP-A-0 705 136, der WO 99/29420, der US-A 4 220 091, der US-A 5 430 220, der US-A 5 877 369, der EP-A-0 117 146, der DE-A 199 37 196, der DE-A 199 37 105 sowie der DE-A 199 37 107 empfohlen werden. Im besonderen können sowohl der Katalysator gemäß Beispiel 1, Beispiel 2, Beispiel 3, und Beispiel 4 der DE-A 199 37 107 eingesetzt werden.

Dabei handelt es sich um Dehydrierkatalysatoren, die 10 bis 99,9 Gew.-% Zirkondioxid, 0 bis 60 Gew.-% Aluminiumoxid, Siliciumdioxid und/oder Titandioxid und 0,1 bis 10 Gew.-% mindestens eines Elements der ersten oder zweiten Hauptgruppe, eines Elements der dritten Nebengruppe, eines Elements der achten Nebengruppe des Periodensystems der Elemente, Lanthan und/oder Zinn enthalten, mit der Maßgabe, dass die Summe der Gewichtsprozente 100 Gew.-% ergibt.

Zur Durchführung der heterogen katalysierten Propandehydrierung kommen prinzipiell alle im Stand der Technik bekannten Reaktortypen und Verfahrensvarianten in Betracht. Beschreibungen solcher Verfahrensvarianten enthalten zum Beispiel alle bezüglich der Dehydrierkatalysatoren zitierten Schriften des Standes der Technik.

Eine vergleichsweise ausführliche Beschreibung von erfindungsgemäß geeigneten Dehydrierverfahren enthält auch "Catalytica® Studies Division, Oxidative Dehydrogenation and Alternative Dehydrogenation Processes, Study Number 4192 OD, 1993, 430 Ferguson Drive, Mountain View, California, 94043-5272 U.S.A..

Charakteristisch für die partielle heterogen katalysierte Dehydrierung von Propan ist, dass sie endotherm verläuft. Das heißt, die für die Einstellung der erforderlichen Reaktionstemperatur benötigte Wärme (Energie) muß entweder dem Reaktionsgasausgangsgemisch vorab und/oder im Verlauf der heterogen katalysierten Dehydrierung zugeführt werden.

Ferner ist es insbesondere für heterogen katalysierte Dehydrierungen von Propan aufgrund der hohen benötigten Reaktionstemperaturen typisch, dass in geringen Mengen schwersiedende hochmolekulare organische Verbindungen, bis hin zum Kohlenstoff, gebildet werden, die sich auf der Katalysatoroberfläche abscheiden und selbige dadurch deaktivieren. Um diese nachteilige Begleiterscheinung zu minimieren, kann das zur heterogen katalysierten Dehydrierung bei erhöhter Temperatur über die Katalysatoroberfläche zu leitende Propan haltige Reaktionsgasgemisch mit Wasserdampf verdünnt werden. Sich abscheidender Kohlenstoff wird unter den so gegebenen Bedingungen nach dem Prinzip der Kohlevergasung teilweise oder vollständig eliminiert.

Eine andere Möglichkeit, abgeschiedene Kohlenstoffverbindungen zu beseitigen, besteht darin, den Dehydrierkatalysator von Zeit zu Zeit bei erhöhter Temperatur mit einem Sauerstoff enthaltenden Gas zu durchströmen und damit den abgeschiedenen Kohlenstoff quasi abzubrennen. Eine weitgehende Unterdrückung der Bildung von Kohlenstoffablagerungen ist aber auch dadurch möglich, dass man dem heterogen katalysiert zu dehydrierenden Propan, bevor es bei erhöhter Temperatur über den Dehydrierkatalysator geführt wird, molekularen Wasserstoff zusetzt.

Selbstverständlich besteht auch die Möglichkeit, dem heterogen katalysiert zu dehydrierenden Propan Wasserdampf und molekularen Wasserstoff im Gemisch zuzusetzen. Ein Zusatz von molekularem Wasserstoff zur heterogen katalysierten Dehydrierung von Propan mindert auch die unerwünschte Bildung von Allen (Propadien), Propin und Acetylen als Nebenprodukten.

Eine geeignete Reaktorform für die heterogen katalysierte Propandehydrierung ist der Festbettrohr- beziehungsweise Rohrbündelreaktor. Das heißt, der Dehydrierkatalysator befindet sich in einem oder in einem Bündel von Reaktionsrohren als Festbett. Die Reaktionsrohre werden dadurch beheizt, dass im die Reaktionsrohre umgebenden Raum ein Gas, zum Beispiel ein Kohlenwasserstoff wie Methan, verbrannt wird. Günstig ist es, diese direkte Form der Kontaktrohrerwärmung lediglich auf den ersten etwa 20 bis 30% der Festbettschüttung anzuwenden und die verbleibende Schüttungslänge durch die im Rahmen der Verbrennung freigesetzte Strahlungswärme auf die erforderliche Reaktionstemperatur aufzuwärmen. Auf diesem Weg ist eine annähernd isotherme Reaktionsführung erreichbar. Geeignete Reaktionsrohrinnendurchmesser betragen etwa 10 bis 15 cm. Ein typischer Dehydrierrohrbündelreaktor umfaßt 300 bis 1000 Reaktionsrohre. Die Temperatur im Reaktionsrohrinneren bewegt sich im Bereich von 300 bis 700°C, vorzugsweise im Bereich von 400 bis 700°C. Mit Vorteil wird das Reaktionsgasausgangsgemisch dem Rohrreaktor auf die Reaktionstemperatur vorerwärmt zugeführt. Es ist möglich, dass das Produktgasgemisch das Reaktionsrohr mit einer 50 bis 100°C

tiefergelegenen Temperatur verläßt. Diese Ausgangstemperatur kann aber auch höher oder auf gleichem Niveau liegen. Im Rahmen der vorgenannten Verfahrensweise ist die Verwendung von oxidischen Dehydrierkatalysatoren auf der Grundlage von Chrom- und/oder Aluminiumoxid zweckmäßig. Häufig wird man kein Verdünnungsgas mitverwenden, sondern von im wesentlichen alleinigenm Roh-Propan als Ausgangsreaktionsgas ausgehen. Auch der Dehydrierkatalysator wird meist unverdünnt angewandt.

Großtechnisch kann man mehrere (z.B. drei) solcher Rohrbündelreaktoren parallel betreiben. Dabei können sich erfindungsgemäß gegebenenfalls zwei dieser Reaktoren im Dehydrierbetrieb befinden, während in einem dritten Reaktor die Katalysatorbeschickung regeneriert wird, ohne dass der Betrieb in der wenigstens einen Partialzone leidet.

Eine solche Verfahrensweise ist beispielsweise bei dem in der Literatur bekannten BASF-Linde Propan-Dehydrierverfahren zweckmäßig. Erfindungsgemäß ist es aber von Bedeutung, dass die Verwendung eines solchen Rohrbündelreaktors ausreichend ist.

Eine solche Verfahrensweise ist auch beim sog. "steam active reforming (STAR) process" anwendbar, der von der Phillips Petroleum Co. entwickelt wurde (vergleiche zum Beispiel US-A 4 902 849, US-A 4 996 387 und US-A 5 389 342) . Als Dehydrierkatalysator wird im STAR-Prozess mit Vorteil Promotoren enthaltendes Platin auf Zink (Magnesium) Spinell als Träger angewendet (vergleiche zum Beispiel US-A 5 073 662). Im Unterschied zum BASF-Linde Propan-Dehydrierverfahren wird das zu dehydrierende Propan beim STAR-Prozess mit Wasserdampf verdünnt. Typisch ist ein molares Verhältnis von Wasserdampf zu Propan im Bereich von 4 bis 6. Der Reaktorausgangsdruck liegt häufig bei 303, 975 bis 810, 600 kPa (3 bis 8 atm) und die Reaktionstemperatur wird zweckmäßig zu 480 bis 620°C gewählt. Typische Katalysatorbelastungen mit dem totalen Reaktionsgasgemisch liegen bei 0,5 bis 10 $h^{-1}$ (LHSV) .

Die heterogen katalysierte Propandehydrierung kann auch im Wanderbett gestaltet werden. Beispielsweise kann das Katalysatorwanderbett in einem Radialstromreaktor untergebracht sein. In selbigem bewegt sich der Katalysator langsam von oben nach unten, während das Reaktionsgasgemisch radial fließt. Diese Verfahrensweise wird beispielsweise im sogenannten UOP-Oleflex-Dehydrierverfahren angewandt. Da die Reaktoren bei diesem Verfahren quasi adiabat betrieben werden, ist es zweckmäßig, mehrere Reaktoren als Kaskade hintereinander geschaltet zu betreiben (in typischer Weise bis zu vier) . Dadurch lassen sich zu hohe Unterschiede der Temperaturen des Reaktionsgasgemisches am Reaktoreingang und am Reaktorausgang vermeiden (bei der adiabaten Betriebsweise fungiert das Reaktionsgasausgangsgemisch als Wärmeträger, von dessen Wärmeinhalt der Abfall der Reaktionstemperatur abhängig ist) und trotzdem ansprechende Gesamtumsätze erzielen.

Wenn das Katalysatorbett den Wanderbettreaktor verlassen hat, wird es der Regenerierung zugeführt und anschließend wiederverwendet. Als Dehydrierkatalysator kann für dieses Verfahren zum Beispiel ein kugelförmiger Dehydrierkatalysator eingesetzt werden, der im wesentlichen aus Platin auf kugelförmigem Aluminiumoxidträger besteht. Bei der UOP-Variante wird dem zu dehydrierenden Propan Wasserstoff zugefügt, um eine vorzeitige Katalysatoralterung zu vermeiden. Der Arbeitsdruck liegt typisch bei 202,65 bis 506,625 kPa (2 bis 5 atm). Das Wasserstoff zu Propan-Verhältnis (das molare) beträgt zweckmäßig 0, 1 bis 1. Die Reaktionstemperaturen liegen bevorzugt bei 550 bis 650°C und die Kontaktzeit Belastung des Katalysators mit Reaktionsgasgemisch wird zu etwa 2 bis 6 $h^{-1}$ gewählt.

Bei den beschriebenen Festbettverfahren kann die Katalysatorgeometrie ebenfalls kugelförmig, aber auch zylindrisch (hohl oder voll) oder anderweitig geometrisch gestaltet sein.

Als weitere Verfahrensvariante für die heterogen katalysierte Propandehydrierung beschreibt Proceedings De Witt, Petrochem. Review, Houston, Texas, 1992 a, N1 die Möglichkeit einer heterogen katalysierten Propandehydrierung im Wirbelbett, bei der das Propan nicht verdünnt wird.

Erfindungsgemäß können dabei z.B. zwei Wirbelbetten nebeneinander betrieben werden, von denen sich eines ohne negative Auswirkungen auf den Gesamtprozess zeitweise im Zustand der Regenerierung befinden kann. Als Aktivmasse kommt dabei Chromoxid auf Aluminiumoxid zum Einsatz. Der Arbeitsdruck beträgt typisch 101,325 bis 202,65 kPa (1 bis 2 atm) und die Dehydriertemperatur liegt in der Regel bei 550 bis 600°C. Die für die Dehydrierung erforderliche Wärme wird dadurch in das Reaktionssystem eingebracht, dass der Dehydrierkatalysator auf die Reaktionstemperatur vorerhitzt wird. Die vorstehende Dehydrierweise ist in der Literatur auch als Snamprogetti-Yarsintez Verfahren bekannt.

Alternativ zu den vorstehend beschriebenen Verfahrensweisen kann die heterogen katalysierte Propandehydrierung unter weitgehendem Sauerstoffausschluß auch nach einem von ABB Lummus Crest entwickelten Verfahren realisiert werden (vergleiche Proceedings De Witt, Petrochem. Review, Houston, Texas, 1992, P1).

Den bisher beschriebenen heterogen katalysierten Dehydrierverfahren des Propans unter weitgehendem Sauerstoffausschluß ist gemein, dass sie bei Propanumsätzen von $\geq$ 30 mol-% (in der Regel $\leq$ 60 mol-%) betrieben werden (bezogen auf einmaligen Reaktionszonendurchgang). Erfindungsgemäß von Vorteil ist es, dass es ausreichend ist, einen Propanumsatz von $\geq$ 5 mole bis $\leq$ 30 mol-% oder $\leq$ 25 mol-%, zu erzielen. Das heißt, die heterogen katalysierte Propandehydrierung kann auch bei Propanumsätzen von 10 bis 20 mol-% betrieben werden (die Umsätze beziehen sich auf einmaligen Reaktionszonendurchgang). Dies rührt unter anderem daher, dass die verbliebene Menge an nicht umgesetztem Propan in der nachfolgenden wenigstens einen Partialzone im wesentlichen als inertes Verdünnungsgas fungiert und später weitgehend verlustfrei in die Dehydrierzone und/oder in die wenigstens eine Partialzone rückgeführt

werden kann.

Für die Realisierung der vorgenannten Propanumsätze ist es günstig, die heterogen katalysierte Propandehydrierung bei einem Arbeitsdruck von 30,3975 bis 303,975 kPa (0,3 bis 3 atm) durchzuführen. Ferner ist es günstig, das heterogen katalytisch zu dehydrierende Propan mit Wasserdampf zu verdünnen. So ermöglicht die Wärmekapazität des Wassers einerseits, einen Teil der Auswirkung der Endothermie der Dehydrierung auszugleichen und andererseits reduziert die Verdünnung mit Wasserdampf den Edukt- und Produktpartialdruck, was sich günstig auf die Gleichgewichtslage der Dehydrierung auswirkt. Ferner wirkt sich die Wasserdampfmitverwendung, wie bereits erwähnt, vorteilhaft auf die Standzeit von Edelmetall enthaltenden Dehydrierkatalysatoren aus. Bei Bedarf kann als weiterer Bestandteil auch molekularer Wasserstoff zugegeben werden. Das molare Verhältnis von molekularem Wasserstoff zu Propan ist dabei in der Regel $\leq 5$. Das molare Verhältnis von Wasserdampf zu Propan kann demnach mit vergleichsweise geringem Propanumsatz $\geq 0$ bis 30, zweckmäßig 0,1 bis 2 und günstig 0,5 bis 1 betragen. Als günstig für eine Verfahrensweise mit niederem Propanumsatz erweist es sich auch, dass bei einmaligem Reaktordurchgang des Reaktionsgases lediglich eine vergleichsweise niedrige Wärmemenge verbraucht wird und zur Umsatzerzielung bei einmaligem Reaktordurchgang vergleichsweise niedrige Reaktionstemperaturen ausreichend sind.

Es kann daher zweckmäßig sein, die Propandehydrierung mit vergleichsweise geringem Propanumsatz (quasi) adiabat durchzuführen. Das heißt, man wird das Reaktionsgasausgangsgemisch in der Regel zunächst auf eine Temperatur von 500 bis 700°C (beziehungsweise von 550 bis 650°C) erhitzen (zum Beispiel durch Direktbefeuerung der es umgebenden Wandung). Im Normalfall wird dann ein einziger adiabater Durchgang durch ein Katalysatorbett ausreichend sein, um den gewünschten Umsatz zu erzielen, wobei sich das Reaktionsgasgemisch um etwa 30°C bis 200°C (je nach Umsatz und Verdünnung) abkühlen wird. Ein Beisein von Wasserdampf als Wärmeträger macht sich auch unter dem Gesichtspunkt einer adiabaten Fahrweise vorteilhaft bemerkbar. Die niedrigere Reaktionstemperatur ermöglicht längere Standzeiten des verwendeten Katalysatorbetts.

Prinzipiell ist die heterogen katalysierte Propandehydrierung mit vergleichsweise geringem Propanumsatz, ob adiabat oder isotherm gefahren, sowohl in einem Festbettreaktor als auch in einem Wanderbett- oder Wirbelbettreaktor durchführbar.

Bemerkenswerterweise ist beim erfindungsgemäßen Verfahren zu ihrer Realisierung, insbesondere im adiabatischen Betrieb, ein einzelner Schachtofenreaktor als Festbettreaktor ausreichend, der vom Reaktionsgasgemisch axial und/oder radial durchströmt wird.

Im einfachsten Fall handelt es sich dabei um ein einziges geschlossenes Reaktionsvolumen, zum Beispiel einen Behälter, dessen Innendurchmesser 0,1 bis 10 m, eventuell auch 0,5 bis 5 m beträgt und in welchem das Katalysatorfestbett auf einer Trägervorrichtung (zum Beispiel ein Gitterrost) aufgebracht ist. Das mit Katalysator beschickte Reaktionsvolumen, das im adiabaten Betrieb wärmeisoliert ist, wird dabei mit dem heißen, Propan enthaltenden, Reaktionsgas axial durchströmt. Die Katalysatorgeometrie kann dabei sowohl kugelförmig als auch ringförmig oder strangförmig sein. Da in diesem Fall das Reaktionsvolumen durch einen sehr kostengünstigen Apparat zu realisieren ist, sind alle Katalysatorgeometrien, die einen besonders niedrigen Druckverlust aufweisen, zu bevorzugen. Das sind vor allem Katalysatorgeometrien, die zu einem großen Hohlraumvolumen führen oder strukturiert aufgebaut sind, wie zum Beispiel Monolithe bzw. Wabenkörper. Zur Verwirklichung einer radialen Strömung des Propan enthaltenden Reaktionsgases kann der Reaktor zum Beispiel aus zwei in einer Mantelhülle befindlichen, konzentrisch ineinander gestellten, zylindrischen Gitterrosten bestehen und die Katalysatorschüttung in deren Ringspalt angeordnet sein. Im adiabaten Fall wäre die Mantelhülle gegebenenfalls wiederum thermisch isoliert.

Als Katalysatorbeschickung für eine heterogen katalysierte Propandehydrierung mit vergleichsweise geringem Propanumsatz bei einmaligem Durchgang eignen sich insbesondere die in der DE-A 199 37 107 offenbarten, vor allem alle beispielhaft offenbarten, Katalysatoren.

Nach längerer Betriebsdauer sind vorgenannte Katalysatoren zum Beispiel in einfacher Weise dadurch regenerierbar, dass man bei einer Eintrittstemperatur von 300 bis 600°C, häufig bei 400 bis 550°C, zunächst in ersten Regenerierungsstufen mit Stickstoff und/ oder Wasserdampf (bevorzugt) verdünnte Luft über das Katalysatorbett leitet. Die Katalysatorbelastung mit Regeneriergas kann dabei z.B. 50 bis 10000 $h^{-1}$ und der Sauerstoffgehalt des Regeneriergases 0,5 bis 20 Vol.-% betragen.

In nachfolgenden weiteren Regenerierungsstufen kann unter ansonsten gleichen Regenerierbedingungen als Regeneriergas Luft verwendet werden. Anwendungstechnisch zweckmäßig empfiehlt es sich, den Katalysator vor seiner Regenerierung mit Inertgas (zum Beispiel $N_2$) zu spülen.

Anschließend ist es in der Regel empfehlenswert, noch mit reinem molekularen Wasserstoff oder mit durch Inertgas (vorzusgweise Wasserdampf) verdünntem molekularem Wasserstoff (der Wasserstoffgehalt sollte $\geq 1$ Vol.-% betragen) im ansonsten gleichen Bedingungsraster zu regenerieren.

Die heterogen katalysierte Propandehydrierung mit vergleichsweise geringem Propanumsatz ($\leq$ **3**0 mol-%) kann in allen Fällen bei den gleichen Katalysatorbelastungen (sowohl das Reaktionsgas insgesamt, als auch das in selbigem enthaltende Propan betreffend) betrieben werden wie die Varianten mit hohem Propanumsatz (> 30 mol-%). Diese Belastung mit Reaktionsgas kann zum Beispiel 100 bis 10000 $h^{-1}$, häufig 300 bis 5000 $h^{-1}$, das heißt vielfach

etwa 500 bis 3000 $h^{-1}$ betragen.

In besonders eleganter Weise läßt sich die heterogen katalysierte Propandehydrierung mit vergleichsweise geringem Propanumsatz in einem Hordenreaktor verwirklichen.

Dieser enthält räumlich aufeinanderfolgend mehr als ein die Dehydrierung katalysierendes Katalysatorbett. Die Katalysatorbettanzahl kann 1 bis 20, zweckmäßig 2 bis 8, aber auch 3 bis 6 betragen. Die Katalysatorbetten sind vorzugsweise radial oder axial hintereinander angeordnet. Anwendungstechnisch zweckmäßig wird in einem solchen Hordenreaktor der Katalysatorfestbetttyp angewendet.

Im einfachsten Fall sind die Katalysatorfestbetten in einem Schachtofenreaktor axial oder in den Ringspalten von zentrisch ineinandergestellten zylindrischen Gitterrosten angeordnet. Es ist jedoch auch möglich, die Ringspalte in Segmenten übereinander anzuordnen und das Gas nach radialem Durchtritt in einem Segment in das nächste darüber- oder darunterliegende Segment zu führen.

In zweckmäßiger Weise wird das Reaktionsgasgemisch auf seinem Weg von einem Katalysatorbett zum nächsten Katalysatorbett, zum Beispiel durch Überleiten mit heißen Gasen erhitzte Wärmetauscherrippen oder durch Leiten durch mit heißen Brenngasen erhitzte Rohre, im Hordenreaktor einer Zwischenerhitzung unterworfen.

Wird der Hordenreaktor im übrigen adiabat betrieben, ist es für die gewünschten Propanumsätze ($\leq$30 mol-%) insbesondere bei Verwendung der in der DE-A 199 37 107 beschriebenen Katalysatoren, insbesondere der beispielhaften Ausführungsformen, ausreichend, das Reaktionsgasgemisch auf eine Temperatur von 450 bis 550°C vorerhitzt in den Dehydrierreaktor zu führen und innerhalb des Hordenreaktors in diesem Temperaturbereich zu halten. Das heißt, die gesamte Propandehydrierung ist so bei äußerst niederen Temperaturen zu verwirklichen, was sich für die Standzeit der Katalysatorfestbetten zwischen zwei Regenerierungen als besonders günstig erweist.

Noch geschickter ist es, die katalytische Dehydrierung autotherm, d.h., z.B. die vorstehend geschilderte Zwischenerhitzung auf direktem Weg durchzuführen (autotherme Fahrweise). Dazu wird dem Reaktionsgasgemisch entweder bereits vor Durchströmung des ersten Katalysatorbettes und/oder zwischen den nachfolgenden Katalysatorbetten in begrenztem Umfang molekularer Sauerstoff zugesetzt. Je nach verwendetem Dehydrierkatalysator wird so eine begrenzte Verbrennung der im Reaktionsgasgemisch enthaltenen Kohlenwasserstoffe, gegebenenfalls bereits auf der Katalysatoroberfläche abgeschiedener Kohle beziehungsweise kohleähnlicher Verbindungen und/oder von im Verlauf der heterogen katalysierten Propandehydrierung gebildetem und/oder dem Reaktionsgasgemisch zugesetztem Wasserstoff bewirkt (es kann auch anwendungstechnisch zweckmäßig sein, im Hordenreaktor Katalysatorbetten einzufügen, die mit Katalysator beschickt sind, der spezifisch (selektiv) die Verbrennung von Wasserstoff (und/oder von Kohlenwasserstoff) katalysiert (als solche Katalysatoren kommen zum Beispiel jene der Schriften US-A 4 788 371, US-A 4 886 928, US-A 5 430 209, US-A 5 530 171, US-A 5 527 979 und US-A 5 563 314 in Betracht; beispielsweise können solche Katalysatorbetten in alternierender Weise zu den Dehydrierkatalysator enthaltenden Betten im Hordenreaktor untergebracht sein). Die dabei freigesetzte Reaktionswärme ermöglicht so auf quasi autotherme Weise eine nahezu isotherme Betriebsweise der heterogen katalysierten Propandehydrierung. Mit zunehmender gewählter Verweilzeit des Reaktionsgases im Katalysatorbett ist so eine Propandehydrierung bei abnehmender oder im wesentlichen konstanter Temperatur möglich, was besonders lange Standzeiten zwischen zwei Regenerierungen ermöglicht.

In der Regel sollte eine wie vorstehend beschriebene Sauerstoffeinspeisung so vorgenommen werden, dass der Sauerstoffgehalt des Reaktionsgasgemisches, bezogen auf die darin enthaltene Menge an Propan und Propylen, 0,5 bis 30 Vol.-% beträgt. Als Sauerstoffquelle kommen dabei sowohl reiner molekularer Sauerstoff oder mit Inertgas, zum Beispiel CO, $CO_2$, $N_2$, Edelgase, verdünnter Sauerstoff, insbesondere aber auch Luft, in Betracht. Die resultierenden Verbrennungsgase wirken in der Regel zusätzlich verdünnend und fördern dadurch die heterogen katalysierte Propandehydrierung.

Die Isothermie der heterogen katalysierten Propandehydrierung läßt sich dadurch weiter verbessern, dass man im Hordenreaktor in den Räumen zwischen den Katalysatorbetten geschlossene, vor ihrer Füllung günstigerweise aber nicht notwendigerweise evakuierte, Einbauten (zum Beispiel rohrförmige), anbringt. Derartige Einbauten können auch ins jeweilige Katalysatorbett gestellt werden. Diese Einbauten enthalten geeignete Feststoffe oder Flüssigkeiten, die oberhalb einer bestimmten Temperatur verdampfen oder schmelzen und dabei Wärme verbrauchen und dort, wo diese Temperatur unterschritten wird, wieder kondensieren und dabei Wärme freisetzen.

Eine Möglichkeit, das Reaktionsgasausgangsgemisch für die heterogen katalysierte Propandehydrierung auf die benötigte Reaktionstemperatur zu erwärmen, besteht auch darin, einen Teil des darin enthaltenen Propans und/oder $H_2$ mittels molekularem Sauerstoff zu verbrennen (zum Beispiel an geeigneten spezifisch wirkenden Verbrennungskatalysatoren, zum Beispiel durch einfaches Überleiten und/oder Durchleiten) und mittels der so freigesetzten Verbrennungswärme die Erwärmung auf die gewünschte Reaktionstemperatur zu bewirken. Die resultierenden Verbrennungsprodukte, wie $CO_2$, $H_2O$ sowie das den für die Verbrennung benötigten molekularen Sauerstoff gegebenenfalls begleitende $N_2$ bilden vorteilhaft inerte Verdünnungsgase.

Besonders elegant läßt sich die vorgenannte Wasserstoffverbrennung wie in der DE-A 10211275 beschrieben realisieren. D.h., in einem Verfahren der kontinuierlichen heterogen katalysierten partiellen Dehydrierung von Propan in der Gasphase, bei dem

- einer Reaktionszone ein das zu dehydrierende Propan enthaltendes Reaktionsgas kontinuierlich zugeführt wird,
- das Reaktionsgas in der Reaktionszone über wenigstens ein Katalysatorfestbett geführt wird, an welchem durch katalytische Dehydrierung molekularer Wasserstoff und wenigstens partiell Propylen gebildet werden,
- dem Reaktionsgas vor und/oder nach Eintritt in die Reaktionszone wenigstens ein molekularen Sauerstoff enthaltendes Gas zugesetzt wird,
- der molekulare Sauerstoff in der Reaktionszone im Reaktionsgas enthaltenen molekularen Wasserstoff teilweise zu Wasserdampf oxidiert und
- der Reaktionszone ein Produktgas entnommen wird, das molekularen Wasserstoff, Wasserdampf, Propylen und Propan enthält,

das dadurch gekennzeichnet ist, daß das der Reaktionszone entnommene Produktgas in zwei Teilmengen identischer Zusammensetzung aufgeteilt und eine der beiden Teilmengen als Kreisgas in die Dehydrierreaktionszone zurückgeführt und die andere Teilmenge erfindungsgemäß als Gasgemisch 1 weiterverwendet wird.

Diese Verfahrensvariante ist insbesondere dann zu bevorzugen, wenn man in die Dehydrierzone als weitere Propanquelle neben Roh-Propan aus der wenigstens einen Partialzone stammendes, Propan und gegebenenfalls Propylen enthaltendes Kreisgas (das gegebenenfalls einer Nebenkomponentenabtrennung (z.B. $C_4$-Kohlenwasserstoffe wie Buten-1) unterworfen wurde) führt. Dies gilt vor allem dann, wenn das Kreisgas die einzige Sauerstoffquelle für die Wasserstoffverbrennung bei dieser Verfahrensvariante bildet.

Das im Rahmen der heterogen katalysierten Propandehydrierung gebildete Produktgasgemisch enthält beim erfindungsgemäßen Verfahren in der Regel Propan, Propen, molekularen Wasserstoff, $N_2$, $H_2O$, Methan, Ethan, Ethylen, Buten-1, sonstige Butene und andere $C_4$-Kohlenwasser-stoffe (n-Butan, iso-Butan, Butadien etc.), CO und $CO_2$. Es wird sich in der Regel bei einem Druck von 30,3975 bis 1013,25 kPa (0,3 bis 10 atm) befinden und häufig eine Temperatur von 400 bis 500°C, in günstigen Fällen von 450 bis 500°C aufweisen.

Während die EP-A 117 146, die DE-A 3 313 573 und die US-A 3 161 670 empfehlen, das in der heterogen katalysierten Propandehydrierung gebildete Produktgasgemisch (Gasgemisch 1) als solches zur Beschickung der wenigstens einen Partialzone zu verwenden, ist es erfindungsgemäß meist zweckmäßig aus dem Produktgasgemisch (dem Gasgemisch 1) der Oxidehydrierung und/oder Dehydrierung vor seiner Weiterverwendung zur Beschickung der wenigstens einen Partialzone wenigstens eine Teilmenge der gegebenenfalls darin enthaltenen $C_4$-Kohlenwasserstoffe (z.b. Buten-1, n-Butan, iso-Butan, andere Butene, Butadiene etc.) abzutrennen. Enthält das Gasgemisch 1 Wasserstoff, kann vorgenannte Abtrennung mit wenigstens einer Teilabtrennung des Wasserstoffs einhergehen oder eine solche Wasserstoffabtrennung vorab durchgeführt werden.

Letzteres kann z.B. dadurch erfolgen, dass man das Gasgemisch 1, gegebenenfalls nachdem man es zuvor in einem indirekten Wärmetauscher abgekühlt hat (zweckmäßigerweise wird die dabei entnommene Wärme zum Erhitzen eines für das erfindungsgemäße Verfahren benötigten feed-Gases verwendet) über eine, in der Regel zu einem Rohr gestaltete, Membran leitet, die lediglich für den molekularen Wasserstoff durchlässig ist. Der so abgetrennte molekulare Wasserstoff kann bei Bedarf teilweise in die heterogen katalysierte Dehydrierung von Propan rückgeführt oder einer sonstigen Verwertung zugeführt werden. Beispielsweise kann er in Brennstoffzellen verbrannt werden.

Alternativ kann eine partielle oder vollständige Wasserstoffabtrennung auch durch partielle Kondensation, Adsorption und/oder Rektifikation (vorzugsweise unter Druck) vorgenommen werden. Die Teil- oder Vollabtrennung des molekularen Wasserstoff aus dem Produktgasgemisch (Gasgemisch 1) kann beim erfindungsgemäßen Verfahren auch durch selektive (z.B. heterogen katalysierte) Verbrennung desselben mit molekularem Sauerstoff vorgenommen werden. Das sich dabei bildende Reaktionswasser kann entweder teilweise oder vollständig abgetrennt oder im Gasgemisch belassen werden, da es in der wenigstens einen Partialzone als inertes Verdünnungsgas zu fungieren vermag. Diesbezüglich geeignete Katalysatoren offenbaren beispielsweise die US-A 4 788 371, US-A 4 886 928, US-A 5 430 209, US-A 5 5530171, US-A 5 527979 und US-A 5 563314.

Die Selektivverbrennung des molekularen Wasserstoff kann auch bereits während der heterogen katalysierten Dehydrierung quasi in situ erfolgen, z.B. durch Oxidation mittels eines dem Dehydrierungskatalysator zusätzlich zugesetzten reduzierbaren Metalloxids, wie es z.B. die EP-A 832056 beschreibt.

Erfindungsgemäß zweckmäßig wird man wenigstens 10 mol-%, oder wenigstens 25 mol-%, häufig wenigstens 35 mol-%, oder wenigstens 50 mol-%, vielfach wenigstens 75 mol-% und oft die Gesamtmenge des im Rahmen der heterogen katalysierten Dehydrierung gebildeten molekularen Wasserstoff vorab- und/oder co- abtrennen, bevor das verbleibende Produktgasgemisch (Gasgemisch 1') zur Beschickung der wenigstens einen Partialozone verwendet wird. Bei Bedarf kann aus dem Gasgemisch 1 vor seiner Weiterverarbeitung in der wenigstens einen Partialzone auch eventuell enthaltenes Wasser abgetrennt (z.B. Auskondensieren) werden. Selbstredend kann bei Bedarf im Rahmen der Abtrennung von molekularem Wasserstoff und/oder $C_4$-Kohlenwasser-stoffen wie Buten-1 etc. auch eine Abtrennung anderer, von Propan und Propylen verschiedener, Bestandteile des Produktgasgemisches (Gasgemisches 1) vorgenommen werden.

Eine einfache Möglichkeit dazu besteht z.B. darin, das vorzugsweise abgekühlte (vorzugsweise auf Temperaturen von 10 bis 70°C), Gasgemisch 1, z.B. bei einem Druck von 10,1325 bis 5066,25 kPa (0,1 bis 50 atm) und einer Temperatur von 0 bis 100°C, mit einem (vorzugsweise hochsiedenen) organischen Lösungsmittel (vorzugsweise ein hydrophobes), in welchem Propan und Propen bevorzugt absorbiert werden, in Kontakt zu bringen (z.B. durch einfaches Durchleiten). Durch nachfolgende Desorption, Rektifikation und/oder Strippung mit einem bezüglich der wenigstens einen Partialzone sich inert verhaltenden und/oder in dieser Reaktionszone als Reaktand benötigten Gas (z.B. Luft) werden das Propan und Propen im Gemisch in gereinigter Form rückgewonnen und zur Beschickung der wenigstens einen Partialzone verwendet (wie bereits erwähnt, kann im Fall der Strippung mit Luft das dabei erzeugte Gasgemisch 1' mit dem Gasgemisch 2 identisch sein, d.h., als solches unmittelbar zur Beschickung der Beschickung der wenigstens einen Partialoxidation verwendet werden). Das gegebenenfalls molekularen Wasserstoff enthaltende Abgas der Absorption kann man z.B. wieder einer Membrantrennung unterwerfen und dann, bei Bedarf, den abgetrennten Wasserstoff zur heterogen katalysierten Propandehydrierung mitverwenden.

Allerdings ist der $C_3$-Kohlenwasserstoffe/$C_4$-Kohlenwasserstoffe Trennfaktor beim vorstehenden Trennverfahren vergleichsweise begrenzt und für die erfindungsgemäßen Bedürfnisse häufig nicht ausreichend.

Als Alternative für den beschriebenen Trennschritt via Absorption ist für die erfindungsgemäßen Zwecke daher häufig eine Druckwechseladsorption oder eine Druckrektifikation bevorzugt.

Als Absorptionsmittel für die vorstehend beschriebene absorptive Abtrennung eignen sich grundsätzlich alle Absorptionsmittel, die in der Lage sind, Propan und Propen zu absorbieren. Bei dem Absorptionsmittel handelt es sich vorzugsweise um ein organisches Lösungsmittel, das vorzugsweise hydrophob und/oder hochsiedend ist. Vorteilhafterweise hat dieses Lösungsmittel einen Siedepunkt (bei einem Normaldruck von 101, 325 kPa (1 atm)) von mindestens 120°C, bevorzugt von mindestens 180 °C, vorzugsweise von 200 bis 350°C, insbesondere von 250 bis 300°C, stärker bevorzugt von 260 bis 290°C. Zweckmäßigerweise liegt der Flammpunkt (bei einem Normaldruck von 101,325 kPa (1 atm)) über 110°C. Allgemein eignen sich als Absorptionsmittel relativ unpolare organische Lösungsmittel, wie zum Beispiel aliphatische Kohlenwasserstoffe, die vorzugsweise keine nach außen wirkende polare Gruppe enthalten, aber auch aromatische Kohlenwasserstoffe. Allgemein ist es erwünscht, dass das Absorptionsmittel einen möglichst hohen Siedepunkt bei gleichzeitig möglichst hoher Löslichkeit für Propan und Propen hat. Beispielhaft als Absorptionsmittel genannt seien aliphatische Kohlenwasserstoffe, zum Beispiel $C_8$-$C_{20}$-Alkane oder -Alkene, oder aromatische Kohlenwasserstoffe, zum Beispiel Mittelölfraktionen aus der Paraffindestillation oder Ether mit sperrigen (sterisch anspruchvollen) Gruppen am O-Atom, oder Gemische davon, wobei diesen ein polares Lösungsmittel, wie zum Beispiel das in der DE-A 43 08 087 offenbarte 1,2-Dimethylphthalat zugesetzt sein kann. Weiterhin eignen sich Ester der Benzoesäure und Phthalsäure mit geradkettigen, 1 bis 8 Kohlenstoffatome enthaltenden Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Diphenyl, Diphenylether und Gemische aus Diphenyl und Diphenylether oder deren Chlorderivate und Triarylalkene, zum Beispiel 4-Methyl-4'-benzyl-diphenylmethan und dessen Isomere 2-Methyl-2'-benzyldiphenyl-methan, 2-Methyl-4'-benzyldiphenylmethan und 4-Methyl-2'-benzyl-diphenylmethan und Gemische solcher Isomerer. Ein geeignetes Absorptionsmittel ist ein Lösungsmittelgemisch aus Diphenyl und Diphenylether, bevorzugt in der azeotropen Zusammensetzung, insbesondere aus etwa 25 Gew.-% Diphenyl (Biphenyl) und etwa 75 Gew.-% Diphenylether, beispielsweise das im Handel erhältliche Diphyl® (z.B von der Bayer Aktiengesellschaft bezogenes). Häufig enthält dieses Lösungsmittelgemisch ein Lösungsmittel wie Dimethylphthalat in einer Menge von 0,1 bis 25 Gew.-%, bezogen auf das gesamte Lösungsmittelgemisch, zugesetzt. Besonders geeignete Absorptionsmittel sind auch Octane, Nonane, Decane, Undecane, Dodecane, Tridecane, Tetradecane, Pentadecane, Hexadecane, Heptadecane und Octadecane, wobei sich insbesondere Tetradecane als besonders geeignet erwiesen haben. Günstig ist es, wenn das verwendete Absorptionsmittel einerseits den oben genannten Siedepunkt erfüllt, andererseits gleichzeitig aber ein nicht zu hohes Molekulargewicht aufweist. Mit Vorteil beträgt das Molekulargewicht des Absorptionsmittels $\leq$ 300 g/mol. Geeignet sind auch die in DE-A 33 13 573 beschriebenen Paraffinöle mit 8 bis 6 Kohlenstoffatomen. Beispiele für geeignete Handelsprodukte sind von der Firma Haltermann vertriebene Produkte wie Halpasole i, wie Halpasol 250/340 i und Halpasol 250/275 i, sowie Druckfarbenöle unter den Bezeichnungen PKWF und Printosol. Bevorzugt sind an Aromaten freie Handelsprodukte, z.B. solche des Typs PKWFaf.

Die Durchführung der Absorption unterliegt keinen besonderen Beschränkungen. Es können alle dem Fachmann gängigen Verfahren und Bedingungen eingesetzt werden. Vorzugsweise wird das Gasgemisch bei einem Druck von 100 bis 5000 kPa (1 bis 50 bar), bevorzugt 200 bis 2000 kPa (2 bis 20 bar), stärker bevorzugt 500 bis 1000 kPa (5 bis 10 bar), und einer Temperatur von 0 bis 100°C, insbesondere von 30 bis 50°C, mit dem Absorptionsmittel in Kontakt gebracht. Die Absorption kann sowohl in Kolonnen als auch in Quenchapparaten vorgenommen werden. Dabei kann im Gleichstrom oder im Gegenstrom gearbeitet werden. Geeignete Absorptionskolonnen sind zum Beispiel Bodenkolonnen (mit Glocken- und/oder Siebböden), Kolonnen mit strukturierten Packungen (zum Beispiel Blechpackungen mit

einer spezifischen Oberfläche von 100 bis 1000, oder bis 750 $m^2/m^3$, zum Beispiel Mellapak® 250 Y) und Füllkörperkolonnen (zum Beispiel mit Raschig-Füllkörpern gefüllte). Es können aber auch Riesel- und Sprühtürme, Graphitblockabsorber, Oberflächenabsorber wie Dickschicht- und Dünnschichtabsorber sowie Tellerwäscher, Kreuzschleierwäscher und Rotationswäscher verwendet werden. Zudem kann es günstig sein, die Absorption in einer Blasensäule mit und ohne Einbauten stattfinden zu lassen.

Die Abtrennung des Propans und Propens von dem Absorptionsmittel kann durch Strippung, Entspannungsverdampfung (Flashen) und/oder Destillation erfolgen.

Die Abtrennung des Propans und Propens von dem Absorptionsmittel erfolgt vorzugsweise durch Strippen und/oder Desorption. Die Desorption kann in üblicher Weise über eine Druck- und/oder Temperaturänderung durchgeführt werden, vorzugsweise bei einem Druck von 10 bis 1000 kPa (0,1 bis 10 bar), insbesondere 100 bis 500 kPa (1 bis 5 bar), stärker bevorzugt 100 bis 300 kPa (1 bis 3 bar), und einer Temperatur von 0 bis 200°C, insbesondere 20 bis 100°C, stärker bevorzugt 30 bis 70°C, besonders bevorzugt 40 bis 60°C. Ein für die Strippung geeignetes Gas ist beispielsweise Wasserdampf, bevorzugt sind jedoch insbesondere Sauerstoff-/Stickstoff-Mischungen, beispielsweise Luft. Bei der Verwendung von Luft beziehungsweise Sauerstoff-/Stickstoff-Mischungen, bei denen der Sauerstoffgehalt über 10 Vol-% liegt, kann es sinnvoll sein, vor oder während des Stripprozesses ein Gas zuzusetzen, das den Explosionsbereich reduziert. Besonders geeignet dafür sind Gase mit einer spezifischen Wärmekapazität >□29 J/mol·K bei 20°C, wie zum Beispiel Methan, Ethan, Propan, Propen, Benzol, Methanol, Ethanol, sowie Ammoniak, Kohlendioxid, und Wasser. $C_4$-Kohlenwasserstoffe sind erfindungsgemäß als solche Zusätze jedoch zu vermeiden. Besonders geeignet für die Strippung sind auch Blasensäulen mit und ohne Einbauten.

Die Abtrennung des Propans und Propens von dem Absorptionsmittel kann auch über eine Destillation bzw. Rektifikation erfolgen, wobei die dem Fachmann geläufigen Kolonnen mit Packungen, Füllkörpern oder entsprechenden Einbauten verwendet werden können. Bevorzugte Bedingungen bei der Destillation bzw. Rektifikation sind ein Druck von 1 bis 500 kPa (0,01 bis 5 bar), insbesondere 10 bis 400 kPa (0,1 bis 4 bar), stärker bevorzugt 100 bis 300 kPa (1 bis 3 bar), und eine Temperatur (im Sumpf) von 50 bis 300°C, insbesondere 150 bis 250°C.

Ein durch Strippen aus dem Absorptionsmittel gewonnenes Gasgemisch 1' kann vor seiner Verwendung zur Beschickung der wenigstens einen Partialzone noch einer weiteren Verfahrensstufe zugeführt werden, um z.B. die Verluste an mitgestripptem Absorptionsmittel zu reduzieren (z.B. Abscheidung in Demistern und/oder Tiefenfiltern) und die wenigstens eine Partialzone so gleichzeitig vor Absorptionsmittel zu schützen oder um die Trennwirkung zwischen $C_3$-/$C_4$-Kohlenwasserstoffen weiter zu verbessern. Eine solche Abtrennung des Absorptionsmittels kann nach allen dem Fachmann bekannten Verfahrensschritten erfolgen. Eine im Rahmen des erfindungsgemäßen Verfahrens bevorzugte Ausführungsform einer solchen Abtrennung ist z.B. das Quenchen des Ausgangsstromes aus der Strippvorrichtung mit Wasser. In diesem Fall wird das Absorptionsmittel aus diesem beladenen Ausgangsstrom mit Wasser herausgewaschen und der Ausgangsstrom gleichzeitig mit Wasser beladen. Diese Wäsche beziehungsweise das Quenchen kann z.B. am Kopf einer Desorptionskolonne über einem Flüssigkeits-Fangboden durch Gegensprühen von Wasser oder in einem eigenen Apparat erfolgen.

Zur Unterstützung des Trenneffektes können im Quenchraum die Quenchoberfläche vergößernde Einbauten installiert werden, wie sie dem Fachmann von Rektifikationen, Absorptionen und Desorptionen her bekannt sind.

Wasser ist insofern ein bevorzugtes Waschmittel, da es in der nachfolgenden wenigstens einen Partialzone normalerweise nicht stört. Nachdem das Wasser das Absorptionsmittel aus dem mit Propan und Propen beladenen Ausgangstrom herausgewaschen hat, kann das Wasser/Absorptionsmittel-Gemisch einer Phasentrennung zugeführt und der behandelte Ausgangstrom als ein Gasgemisch 1' der Partialzone zugeführt werden.

Sowohl das $C_3$-frei gestrippte Absorptionsmittel als auch das in der Phasentrennung rückgewonnene Absorptionsmittel können für den Absorptionszweck wiederverwendet werden.

Das Gasgemisch 1 und/oder das aus selbigem erzeugte Gasgemisch 1' kann nun in an sich bekannter Weise in wenigstens einer weiteren Reaktionszone zur Beschickung einer heterogen katalysierten Gasphasenoxidation und/oder -ammoxidation von Propen zu Acrolein und/oder Acrylsäure und/oder Acrylnitril mit einem BeschickungsGasgemisch 2 eingesetzt werden. Als Oxidationsmittel kann dabei reiner molekularer Sauerstoff, Luft, mit Sauerstoff angereicherte Luft oder jedes sonstige Gemisch aus Sauerstoff und Inertgas zugesetzt werden. Handelt es sich bei der Partialoxidation um die Umsetzung von Propylen zu Propylenoxid, kann z.B. wie in der EP-A 372972 beschrieben verfahren werden.

Handelt es sich um eine partielle Ammoxidation zu Acrylnitril, kann z.B. gemäß der DE-A 2351151 verfahren werden. Im Fall einer partiellen Oxidation des Propylens zu Acrolein und/oder Acrylsäure wird man beim erfindungsgemäßen Verfahren die Zusammensetzung des Gasgemisches 2 unter Mitverwendung des Gasgemisches 1 und/ oder 1' (es können auch Gemische beider verwendet werden; d.h. aus einem Teil wird abgetrennt aus einem andern nicht) so einstellen, dass es die nachfolgenden molaren Verhältnisse erfüllt:

Propan : Propen : $N_2$ : $O_2$ : $H_2O$ : sonstige = 0,5 bis 20 : 1 : 0,1 bis 40 : 0,1 bis 10 : 0 bis 20 : 0 bis 1.

Mit Vorteil betragen die vorgenannten molaren Verhältnisse erfindungsgemäß
= 2 bis 10 : 1 : 0,5 bis 20 : 0,5 bis 5 : 0,01 bis 10 : 0 bis 1.

Günstig ist es auch, wenn die vorgenannten molaren Verhältnisse erfindungsgemäß
= 3 bis 6 : 1 : 1 bis 10 : 1 bis 3 : 0,1 bis 2 : 0 bis 0,5 betragen.

Wie bereits erwähnt, läuft die heterogen katalysierte Gasphasen-Partialoxidation von Propylen zu Acrylsäure mit molekularem Sauerstoff in zwei längs der Reaktionskoordinate aufeinanderfolgenden Schritten ab, von denen der erste zum Acrolein und der zweite vom Acrolein zur Acrylsäure führt.

Dieser Reaktionsablauf in zwei zeitlich aufeinanderfolgenden Schritten eröffnet in an sich bekannter Weise die Möglichkeit, die wenigstens eine Partialzone des erfindungsgemäßen Verfahrens in diesem Fall in zwei hintereinander angeordneten Oxidationszonen auszuführen, wobei in jeder der beiden Oxidationszonen der zu verwendende oxidische Katalysator in optimierender Weise angepaßt werden kann. So wird für die erste Oxidationszone (Propylen $\rightarrow$ Acrolein) in der Regel ein Katalysator auf der Basis von die Elementkombination Mo-Bi-Fe enthaltenden Multimetalloxiden bevorzugt, während für die zweite Oxidationszone (Acrolein $\rightarrow$ Acrylsäure) normalerweise Katalysatoren auf der Basis von die Elementkombination Mo-V enthaltenden Multimetalloxiden bevorzugt werden.

Entsprechende Multimetalloxidkatalysatoren für die beiden Oxidationszonen sind vielfach vorbeschrieben und dem Fachmann wohlbekannt. Beispielsweise verweist die EP-A 253 409 auf Seite 5 auf entsprechende US-Patente.

Günstige Katalysatoren für die beiden Oxidationszonen offenbaren auch die DE-A 4 431 957 und die DE-A 4431949. Dieses gilt insbesondere für jene der allgemeinen Formel I in den beiden vorgenannten Schriften.

Für den ersten Schritt der Partialoxidation , die heterogen katalysierte Gasphasen-Partialoxidation von Propylen zu Acrolein, kommen, wie bereits gesagt, prinzipiell alle Mo, Bi und Fe enthaltenden Multimetalloxidmassen in Betracht.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für diesen Oxidationsschritt die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1c aus der EP-A 15565 sowie ein in entsprechender Weise herzustellender Katalysator, dessen Aktivmasse jedoch die Zusammensetzung $Mo_{12}Ni_{6,5}Zn_2Fe_2Bi_1P_{0,0065}K_{0,06}O_x \cdot 10SiO_2$ aufweist. Ferner sind hervorzuheben das Beispiel mit der laufenden Nr. 3 aus der DE-A 19855913 (Stöchiometrie: $Mo_{12}Co_7Fe_3Bi_{0,6}K_{0,08}Si_{1,6}O_x$) als Hohlzylindervollkatalysator der Geometrie 5 mm x 3 mm x 2 mm (Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese Hohlzylinder eine Geometrie 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen.

Eine Vielzahl der für den Schritt vom Propylen zum Acrolein geeigneten Multimetalloxidaktivmassen läßt sich unter der allgemeinen Formel IV

$$Mo_{12}Bi_aFe_bX^1_cX^2_dX^3_eX^4_fO_n \qquad (IV),$$

in der die Variablen nachfolgende Bedeutung aufweisen:

- $X^1$ = Nickel und/oder Kobalt,
- $X^2$ = Thallium, ein Alkalimetall und/oder ein Erdalkalimetall,
- $X^3$ = Zink, Phosphor, Arsen, Bor, Antimon, Zinn, Cer, Blei und/ oder Wolfram,
- $X^4$ = Silicium, Aluminium, Titan und/oder Zirkonium,
- a = 0,5 bis 5,
- b = 0,01 bis 5, vorzugsweise 2 bis 4,

c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in IV bestimmt wird,

subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel IV in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, $NH_3$, CO und/oder $H_2$) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, Ammonium-Salze und/oder Hydroxide in Betracht (Verbindungen wie $NH_4OH$, $(NH_4)_2CO_3$, $NH_4NO_3$, $NH_4CHO_2$, $CH_3COOH$, $NH_4CH_3CO_2$ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die Multimetalloxidaktivmassen der allgemeinen Formel IV können für den Schritt "Propen $\rightarrow$ Acrolein" sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/ oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 $\mu$m, bevorzugt im Bereich 50 bis 500 $\mu$m und besonders bevorzugt im Bereich 150 bis 250 $\mu$m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren unterliegenden Zielreaktion in der Regel im wesentlichen inert. Die Trägerkörper können regelmäßig oder unregelmäßig geformt sein, wobei regelmäßig geformte Trägerkörper mit

deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Für den Schritt vom Propylen zum Acrolein zu verwendende Multimetalloxidaktivmassen sind ferner Massen der allgemeinen Formel V

$$[Y^1_{a'}Y^2_{b'}O_{x'}]_p[Y^3_{c'}Y^4_{d'}Y^5_{e'}Y^6_{f'}Y^7_{g'}Y^2_{h'}O_{y'}]_q \qquad (V),$$

in der die Variablen folgende Bedeutung haben:

- $Y^1$ = nur Wismut oder Wismut und wenigstens eines der Elemente Tellur, Antimon, Zinn und Kupfer,
- $Y^2$ = Molybdän oder Molybdän und Wolfram,
- $Y^3$ = ein Alkalimetall, Thallium und/oder Samarium,
- $Y^4$ = ein Erdalkalimetall, Nickel, Kobalt, Kupfer, Mangan, Zink, Zinn, Cadmium und/oder Quecksilber,
- $Y^5$ = Eisen oder Eisen und wenigstens eines der Elemente Chrom und Cer,
- $Y^6$ = Phosphor, Arsen, Bor und/oder Antimon,
- $Y^7$ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,
- a' = 0,01 bis 8,
- b' = 0,1 bis 30,
- c' = 0 bis 4,
- d' = 0 bis 20,
- e' > 0 bis 20,
- f' = 0 bis 6,
- g' = 0 bis 15,
- h' = 8 bis 16,
- x',y'= Zahlen, die durch die Wertigkeit und Häufigkeit der von Sauerstoff verschiedenen Elemente in V bestimmt werden und
- p,q = Zahlen, deren Verhältnis p/q 0,1 bis 10 beträgt, enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung $Y^1_{a'}Y^2_{b'}O_{x'}$, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 $\mu$m, häufig 10 nm bis 500 nm oder 1 $\mu$m bis 50 bzw. 25 $\mu$m, beträgt.

Besonders vorteilhafte erfindungsgemäße Multimetalloxidmassen V sind solche, in denen $Y^1$ nur Wismut ist.

Unter diesen werden wiederum jene bevorzugt, die der allgemeinen Formel VI

$$[Bi_{a''}Z^2_{b''}O_{x''}]_{p''}[Z^2_{12}Z^3_{c''}Z^4_{d''}Fe_{e''}Z^5_{f''}Z^6_{g''}Z^7_{h''}O_{y''}]_{q''} \qquad (VI)$$

in der die Varianten folgende Bedeutung haben:

- $Z^2$ = Molybdän oder Molybdän und Wolfram,
- $Z^3$ = Nickel und/oder Kobalt,
- $Z^4$ = Thallium, ein Alkalimetall und/oder ein Erdalkalimetall,
- $Z^5$ = Phosphor, Arsen, Bor, Antimon, Zinn, Cer und/oder Blei,
- $Z^6$ = Silicium, Aluminium, Titan und/oder Zirkonium,
- $Z^7$ = Kupfer, Silber und/oder Gold,

- a" = 0,1 bis 1,
- b" = 0,2 bis 2,
- c" = 3 bis 10,

d" = 0, 02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in VI bestimmt werden,
p",q"= Zahlen, deren Verhältnis p"/q" 0,1 bis 5, vorzugsweise 0,5 bis 2 beträgt,

entsprechen, wobei diejenigen Massen VI ganz besonders bevorzugt werden, in denen $Z^2_{b''}$ = (Wolfram)$_{b''}$ und $Z^2_{12}$ = (Molybdän)$_{12}$ ist.

Ferner ist es von Vorteil, wenn wenigstens 25 mol-% (bevorzugt wenigstens 50 mol-% und besonders bevorzugt wenigstens 100 mol-%) des gesamten Anteils $[Y^1_{a'}Y^z_{b'}O_{x'}]_p([Bi_{a''}Z^2_{b''}O_{x''}]_{p''})$ der erfindungsgemäß geeigneten Multimetalloxidmassen V (Multimetalloxidmassen VI) in den erfindungsgemäß geeigneten Multimetalloxidmassen V (Multimetalloxidmassen VI) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung $Y^1_{a'}Y^2_{b'}O_{x'}[Bi_{a''}Z^2_{b''}O_{x''}]$ vorliegen, deren Größtdurchmesser im Bereich 1 nm bis 100 µm liegt.

Hinsichtlich der Formgebung gilt bezüglich Multimetalloxidmassen V-Katalysatoren das bei den Multimetalloxidmassen IV-Katalysatoren Gesagte.

Die Herstellung von Multimetalloxidmassen V-Aktivmassen ist z.B. in der EP-A 575897 sowie in der DE-A 19855913 beschrieben.

Die vorstehend empfohlenen inerten Trägermaterialien kommen u.a. auch als inerte Materialien zur Verdünnung und/oder Abgrenzung der entsprechenden Katalysatorfestbetten, bzw. als deren schützende und/oder das Gasgemisch aufheizende Vorschüttung in Betracht.

An dieser Stelle sei erwähnt, daß sich alle Katalysatoren und Multimetalloxidmassen, die für den Schritt vom Propylen zum Acrolein als geeignet empfohlen wurden, sich grundsätzlich auch für die Partialammoxidation von Propylen zu Acrylnitril eignen.

Für den zweiten Schritt, die heterogen katalysierte Gasphasen-Partialoxidation von Acrolein zu Acrylsäure, kommen, wie bereits gesagt, prinzipiell alle Mo und V enthaltenden Multimetalloxidmassen als Aktivmassen in Betracht, z.B. jene der DE-A 10046928.

Eine Vielzahl derselben, z.B. diejenigen der DE-A 19815281, läßt sich unter der allgemeinen Formel VII

$$Mo_{12}V_aX^1_bX^2_cX^3_dX^4_eX^5_fX^6_gO_n \quad \text{(VII)},$$

in der die Variablen folgende Bedeutung haben:

$X^1$ = W, Nb, Ta, Cr und/oder Ce,
$X^2$ = Cu, Ni, Co, Fe, Mn und/oder Zn,
$X^3$ = Sb und/oder Bi,
$X^4$ = eines oder mehrere Alkalimetalle,
$X^5$ = eines oder mehrere Erdalkalimetalle,
$X^6$ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in VII bestimmt wird, subsummieren.

Erfindungsgemäß bevorzugte Ausführungsformen innerhalb der aktiven Multimetalloxide VII sind jene, die von den nachfolgenden Bedeutungen der Variablen der allgemeinen Formel VII erfaßt werden:

$X^1$ = W, Nb, und/oder Cr,
$X^2$ = Cu, Ni, Co, und/oder Fe,
$X^3$ = Sb,

$X^4$ = Na und/oder K,
$X^5$ = Ca, Sr und/oder Ba,
$X^6$ = Si, Al, und/oder Ti,
a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in VII bestimmt wird.

Erfindungsgemäß ganz besonders bevorzugte Multimetalloxide VII sind jedoch jene der allgemeinen Formel VIII

$$Mo_{12}V_{a'}Y^1_{b'}Y^2_{c'}Y^5_{f'}Y^6_{g'}O_{n'} \qquad (VIII)$$

mit

$Y^1$ = W und/oder Nb,
$Y^2$ = Cu und/oder Ni,
$Y^5$ = Ca und/oder Sr,
$Y^6$ = Si und/oder Al,
a' = 2 bis 4,
b' = 1 bis 1,5,
c' = 1 bis 3,
f' = 0 bis 0,5
g' = 0 bis 8 und
n' = eine Zahl, die durch die Wertigkeit und Häufigkeit der von Sauerstoff verschiedenen Elementen in VIII bestimmt wird.

Die erfindungsgemäß geeigneten Multimetalloxidaktivmassen (VII) sind in an sich bekannter, z.B. in der DE-A 4335973 oder in der EP-A 714700 offenbarter, Weise erhältlich.

Prinzipiell können für den Schritt "Acrolein → Acrylsäure" geeignete Multimetalloxidaktivmassen, insbesondere solche der allgemeinen Formel VII, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen VII kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen VII kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die resultierenden Multimetalloxidmassen, insbesondere jene der allgemeinen Formel VII, können für die Acroleinoxidation sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/ oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl.. EP-A 714 700).

Günstige für den Schritt "Acrolein → Acrylsäure" zu verwendende Multimetalloxidaktivmassen sind ferner Massen der allgemeinen Formel IX,

$$[D]_p[E]_q \qquad (IX),$$

in der die Variablen folgende Bedeutung haben:

D = $Mo_{12}V_{a''}Z^1_{b''}Z^2_{c''}Z^3_{d''}Z^4_{e''}Z^5_{f''}Z^6_{g''}O_{x''}$,
E: = $Z^7_{12}Cu_{h''}H_{i''}O_{y''}$,
$Z^1$ = W, Nb, Ta, Cr und/oder Ce,
$Z^2$ = Cu, Ni, Co, Fe, Mn und/oder Zn,
$Z^3$ = Sb und/oder Bi,
$Z^4$ = Li, Na, K, Rb, Cs und/oder H
$Z^5$ = Mg, Ca, Sr und/oder Ba,
$Z^6$ = Si, Al, Ti und/oder Zr,
$Z^7$ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in IX bestimmt werden und
p,q = von Null verschiedene Zahlen, deren Verhältnis p/q 160:1 bis 1:1 beträgt,
und die dadurch erhältlich sind, daß man eine Multimetalloxidmasse E

$Z^7_{12}Cu_{h''}H_{i''}O_{y''}$ (E),

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$Mo_{12}V_{a''}Z^1_{b''}Z^2_{c''}Z^3_{d''}Z^4_{e''}Z^5_{f''}Z^6_{g''}$ (D),

enthält (Ausgangsmasse 2), im gewünschten Mengenverhältnis p:q einarbeitet, die dabei gegebenenfalls resultierende wäßrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen IX, bei denen die Einarbeitung der vorgebildeten festen Ausgangsmasse 1 in eine wäßrige Ausgangsmasse 2 bei einer Temperatur $\leq$ 70°C erfolgt. Eine detaillierte Beschreibung der Herstellung von Multimetalloxidmassen IX-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen IX-Katalysatoren das bei den Multimetalloxidmassen VII-Katalysatoren Gesagte.

Für den Schritt "Acrolein $\rightarrow$ Acrylsäure" in hervorragender Weise geeignete Multimetalloxidkatalysatoren sind auch jene der DE-A 19815281, insbesondere mit Multimetaloxidaktivmassen der allgemeinen Formel I dieser Schrift.

Vorteilhaft werden für den Schritt vom Propylen zum Acrolein Vollkatalysatorringe und für den Schritt vom Acrolein zur Acrylsäure Schalenkatalysatorringe verwendet.

Die Durchführung des ersten Schrittes der Partialoxidation, vom Propylen zum Acrolein, kann mit den beschriebenen Katalysatoren z.B. in einem Einzonen-Vielkontaktrohr-Festbettreaktor durchgeführt werden, wie ihn die DE-A 4431957 beschreibt.

Als Oxidationsmittel wird Sauerstoff verwendet. Wird als inertes Verdünnungsgas $N_2$ gewählt, erweist sich die Verwendung von Luft als Sauerstoffquelle besonders vorteilhaft.

In der Regel wird bei einem Propylen : Sauerstoff : indifferente Gase (einschließlich Wasserdampf) Volumen (N1)-Verhältnis von 1:(1,0 bis 3,0):(5 bis 25), vorzugsweise 1:(1,7 bis 2,3):(10 bis 15) gearbeitet. Der Reaktionsdruck liegt üblicherweise im Bereich von 1 bis 3 bar und die Gesamtraumbelastung beträgt vorzugsweise 1500 bis 4000 N1/(1·h). Die Propylenlast beträgt typisch 90 bis 200 N1/(1·h).

Bevorzugt wird der Einzonen-Vielkontaktrohr-Festbettreaktor mit dem Beschickungsgasgemisch von oben angeströmt. Als Wärmeaustauschmittel wird zweckmäßig eine Salzschmelze, bevorzugt bestehend aus 60 Gew.-% Kaliumnitrat ($KNO_3$) und 40 Gew.-% Natriumnitrit ($NaNO_2$), oder aus 53 Gew.-% Kaliumnitrat ($KNO_3$), 40 Gew.-% Natriumnitrit ($NaNO_2$) und 7 Gew.-% Natriumnitrat ($NaNO_3$), eingesetzt.

Über den Reaktor betrachtet können Salzschmelze und Reaktionsgasgemisch sowohl im Gleichstrom als auch im Gegenstrom geführt werden. Die Salzschmelze selbst wird bevorzugt mäanderförmig um die Kontaktrohre geführt.

Strömt man die Kontaktrohre von oben nach unten an, ist es zweckmäßig, die Kontaktrohre von unten nach oben wie folgt zu beschikken (für die Anströmung von unten nach oben wird die Beschikkungsabfolge in zweckmäßiger Weise umgekehrt):

- zunächst auf einer Länge von 40 bis 60 % der Kontaktrohrlänge entweder nur Katalysator, oder ein Gemisch aus Katalysator und Inertmaterial, wobei letzteres, bezogen auf die Mischung, ein Gewichtsanteil von bis zu 20 Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und

- abschließend auf einer Länge von 10 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Vorgenannte Beschickungsvariante ist insbesondere dann zweckmäßig, wenn als Katalysatoren solche gemäß Beispiel 1 der DE-A 10046957 oder gemäß Beispiel 3 der DE-A 10046957 und als Inertmaterial Ringe aus Steatit mit der Geometrie 7 mm x 7 mm x 4 mm (Außendurchmesser x Höhe x Innendurchmesser) verwendet werden. Hinsichtlich der Salzbadtemperatur gilt das in der DE-A 4431957 Gesagte.

Die Durchführung des ersten Schrittes der Partialoxidation, vom Propylen zum Acrolein, kann mit den beschriebenen Katalysatoren aber auch z.B. in einem Zweizonen-Vielkontaktrohr-Festbettreaktor durchgeführt werden, wie es

die DE-A 19910506 beschreibt. In beiden vorstehend beschriebenen Fällen liegt der erzielte Propenumsatz bei einfachem Durchgang normalerweise bei Werten ≥ 90 mol-%, bzw. ≥ 95 mol-%. Die Durchführung des zweiten Schrittes der Partialoxidation, vom Acrolein zur Acrylsäure, kann mit den beschriebenen Katalysatoren z.B. in einem Einzonen-Vielkontaktrohr-Festbettreaktor durchgeführt werden, wie es die DE-A 4431949 beschreibt. In der Regel wird das Produktgemisch der Propylenoxidation zu Acrolein als solches (gegebenenfalls nach erfolgter Zwischenkühlung desselben), d.h., ohne Nebenkomponentenabtrennung, in die Acroleinoxidation zu Acrylsäure geführt.

Der für den zweiten Schritt der Partialoxidation benötigte Sauerstoff wird dabei bevorzugt als Luft zugesetzt und in der Regel Produktgasgemisch der Propylenoxidation unmittelbar zugegeben.

In der Regel ist das Beschickungsgasgemisch einer solchen Acroleinoxidation dann wie folgt zusammengesetzt: Acrolein : Sauerstoff : Wasserdampf : Inertgas-Volumenverhältnis (N1) von 1:(1 bis 3):(0 bis 20):(3 bis 30), vorzugsweise von 1:(1 bis 3):(0,5 bis 10):(7 bis 18).

Der Reaktionsdruck beträgt in der Regel auch hier 1 bis 3 bar und die Gesamtraumbelastung liegt vorzugsweise bei 1000 bis 3800 N1/(1-h). Die Acroleinlast beträgt typisch 80 bis 190 N1/(1·h).

Bevorzugt wird der Einzonen-Vielkontaktrohr-Festbettreaktor mit dem Beschickungsgasgemisch ebenfalls von oben angeströmt. Als Wärmeaustauschmittel wird auch in der zweiten Stufe in zweckmäßiger Weise eine Salzschmelze, bevorzugt aus 60 Gew.-% Kaliumnitrat ($KNO_3$) und 40 Gew.-% Natriumnitrit ($NaNO_2$) oder aus 53 Gew.-% Kaliumnitrat ($KNO_3$), 40 Gew.-% Natriumnitrit ($NaNO_2$) und 7 Gew.-% Natriumnitrat ($NaNO_3$) bestehend, eingesetzt. Über den Reaktor betrachtet können Salzschmelze und Reaktionsgasgemisch sowohl im Gleichstrom als auch im Gegenstrom geführt werden. Die Salzschmelze selbst wird bevorzugt mäanderförmig in die Kontaktrohre geführt.

Strömt man die Kontaktrohre von oben nach unten an, ist es zweckmäßig, die Kontaktrohre von unten nach oben wie folgt zu beschikken:

- zunächst auf einer Länge von 50 bis 70 % der Kontaktrohrlänge entweder nur Katalysator oder ein Gemisch aus Katalysator und Inertmaterial, wobei letzteres, bezogen auf die Mischung, einen Gewichtsanteil von bis zu 20 Gew.-% ausmacht (Abschnitt C);

- daran anschließend auf einer Länge von 20 bis 40 % der Gesamtrohrlänge entweder nur Katalysator, oder ein Gemisch aus Katalysator und Inertmaterial, wobei letzteres, bezogen auf die Mischung, einen Gewichtsanteil von bis zu 40 Gew.-% ausmacht (Abschnitt B); und

- abschließend auf einer Länge von 5 bis 20 % der Gesamtrohrlänge eine Schüttung aus Inertmaterial (Abschnitt A), die vorzugsweise so gewählt wird, dass sie einen möglichst geringen Druckverlust bedingt.

Bevorzugt ist der Abschnitt C unverdünnt.

Für eine Anströmung der Kontaktrohre von unten nach oben, wird die Kontaktrohrbeschickung in zweckmäßigerweise umgekehrt.

Vorgenannte Beschickungsvariante ist insbesondere dann zweckmäßig, wenn als Katalysatoren solche gemäß Herstellungsbeispiel 5 der DE-A 10046928 oder solche gemäß DE-A 19815281 und als Inertmaterial Ringe aus Steatit mit der Geometrie 7 mm x 7 mm x 4 mm oder 7 mm x 7 mm x 3 mm (jeweils Außendurchmesser x Höhe x Innendurchmessser) verwendet werden. Hinsichtlich der Salzbadtemperatur gilt das in der DE-A 44 319 49 Gesagte. Sie wird in der Regel so gewählt, daß der erzielte Acroleinumsatz bei einfachem Durchgang normalerweise ≥ 90 mol-%, bzw. ≥ 95 mol-% beträgt.

Die Durchführung des zweiten Schrittes der Partialoxidation, vom Acrolein zur Acrylsäure, kann mit den beschriebenen Katalysatoren aber auch z.B. in einem Zweizonen-Vielkontaktrohr-Festbettreaktor durchgeführt werden, wie es die DE-19910508 beschreibt. Für den Acroleinumsatz gilt das oben Genannte. Auch im Fall der Durchführung dieses zweiten Schrittes in einem zweizonen-Vielkontaktrohr-Festbettreaktor wird man zur Erzeugung des Beschickungsgasgemisches zweckmäßig unmittelbar das Produktgasgemisch einer auf den ersten Schritt der Partialoxidation gerichteten (gegebenenfalls nach erfolgter Zwischenkühlung desselben) verwenden (wie sie vorstehend beschrieben wurde.). Der für den zweiten Schritt der Partialoxidation benötigte Sauerstoff wird dabei bevorzugt als Luft zugesetzt und im zweiten Fall dem Produktgasgemisch des ersten Schritts der Partialoxidation unmittelbar zugegeben.

Bei einer zweistufigen Fahrweise mit unmittelbarer Weiterverwendung des Produktgasgemisches des ersten Schritts der Partialoxidation zur Beschickung des zweiten Schritts der Partialoxidation, wird man in der Regel zwei Einzonen-Vielkontaktrohr-Festbettreaktoren oder zwei zweizonen-Vielkontaktrohr-Festbettreaktoren hintereinanderschalten. Eine gemischte Hintereinanderschaltung (Einzonen/Zweizonen oder umgekehrt) ist auch möglich.

Zwischen den Reaktoren kann sich ein Zwischenkühler befinden, der gegebenenfalls Inertschüttungen enthalten kann, die eine Filterfunktion ausüben können. Die Salzbadtemperatur von Vielkontaktrohrreaktoren für den ersten Schritt der Partialoxidation von Propylen zu Acrylsäure beträgt in der Regel 300 bis 400°C. Die Salzbadtemperatur von Vielkontaktrohrreaktoren für den zweiten Schritt der Partialoxidation von Propylen zu Acrylsäure, die Partialoxidation

von Acrolein zu Acrylsäure, beträgt meist 200 bis 350°C. Außerdem werden die Wärmeaustauschmittel (bevorzugt Salzschmelzen) normalerweise in solchen Mengen durch die relevanten Vielkontaktrohr-Festbettreaktoren geführt, daß der Unterschied zwischen ihrer Eingangs- und ihrer Ausgangstemperatur in der Regel ≤ 5°C beträgt. Wie bereits erwähnt, können beide Schritte der Partialoxidation von Propylen zu Acrylsäure aber auch wie in der DE-A 10121592 beschrieben in einem Reaktor an einer Beschickung vollzogen werden.

Es sei auch nochmals erwähnt, daß ein Teil des Beschickungsgasgemisches (Gasgemisches 2) für den ersten Schritt ("Propylen → Acrolein") aus der Partialoxidation kommendes Kreisgas sein kann.

Hierbei handelt es sich um Gas, das nach der Zielproduktabtrennung (Acrolein- und/oder Acrylsäureabtrennung) vom Produktgasgemisch der Partialoxidation verbleibt und als inertes Verdünnungsgas in die Beschickung für den ersten und/oder gegebenenfalls zweiten Schritt der Partialoxidation von Propylen zu Acrolein und/oder Acrylsäure rückgeführt werden kann.

Bevorzugt wird solches Propan und gegebenenfalls Propylen enthaltendes Kreisgas jedoch in die Beschickung des ersten Schritts des erfindungsgemäßen Verfahrens rückgeführt.

Es sei auch noch erwähnt, daß eine erfindungsgemäße Partialoxidation und/oder -ammoxidation so durchgeführt werden kann, daß man über die Katalysatorbeschickung zunächst ein Reaktionsgasgemisch über die Katalysatorbeschickung leitet, das keinen Sauerstoff enthält. In diesem Fall wird der für die Partialoxidation benötigte Sauerstoff als Gittersauerstoff zur Verfügung gestellt. In einem nachfolgenden Regenerierschritt mit einem Sauerstoff enthaltenden Gas (z.B. Luft, mit Sauerstoff angereicherte Luft oder an Sauerstoff entreicherte Luft) wird das Katalysatorbett regeneriert, um nachfolgend wiederum für ein an Sauerstoff freies Reaktionsgasgemisch zur Verfügung zu stehen usw..

Zusammenfassend bildet nochmals ein Rohrbündelreaktor innerhalb dessen sich die Katalysatorbeschickung längs der einzelnen Kontaktrohre mit Beendigung des ersten Reaktionsschrittes entsprechend ändert (derartige als Reaktionszone B erfindungsgemäß geeignete Propylenpartialoxidationen lehren z.B. die EP-A 911313, die EP-A 979813, die EP-A 990636 und die DE-A 2830765) die einfachste Realisierungsform zweier Oxidationszonen für die beiden Schritte der Partialoxidation von Propylen zu Acrylsäure. Gegebenenfalls wird dabei die Beschickung der Kontaktrohre mit Katalysator durch eine Inertschüttung unterbrochen.

Vorzugsweise werden die beiden Oxidationszonen jedoch in Form zweier hintereinander geschalteter Rohrbündelsysteme realisiert. Diese können sich in einem Reaktor befinden, wobei der Übergang von einem Rohrbündel zum anderen Rohrbündel von einer nicht im Kontaktrohr untergebrachten (zweckmäßigerweise begehbaren) Schüttung aus Inertmaterial gebildet werden. Während die Kontaktrohre in der Regel von einem Wärmeträger umspült werden, erreicht dieser eine wie vorstehend beschrieben angebrachte Inertschüttung nicht. Mit Vorteil werden daher die beiden Kontaktrohrbündel in räumlich voneinander getrennten Reaktoren untergebracht. In der Regel befindet sich dabei zwischen den beiden Rohrbündelreaktoren ein Zwischenkühler, um eine gegebenenfalls erfolgende Acroleinnachverbrennung im Produktgasgemisch, das die erste Oxidationszone verläßt, zu mindern. Anstelle von Rohrbündelreaktoren können auch Plattenwärmetauscherreaktoren mit Salz- und/oder Siedekühlung, wie sie z.B. die DE-A 19 929 487 und die DE-A 19 952 964 beschreiben, eingesetzt werden.

Die Reaktionstemperatur in der ersten Oxidationszone liegt in der Regel bei 300 bis 450°C, bevorzugt bei 320 bis 390°C. Die Reaktionstemperatur in der zweiten Oxidationszone liegt in der Regel bei 200 bis 300°C, häufig bei 220 bis 290°C. Der Reaktionsdruck beträgt in beiden Oxidationszonen zweckmäßig 50,6625 bis 506,625 kPa (0,5 bis 5 atm), vorteilhaft 101,325 bis 303,975 kPa (1 bis 3 atm). Die Belastung (N1/1·h) der Oxidationskatalysatoren mit Reaktionsgas beträgt in beiden Oxidationszonen häufig 1500 bis 2500 $h^{-1}$ bzw. bis 4000 $h^{-1}$. Die Belastung mit Popylen kann dabei bei 100 bis 200 und mehr N1/1·h liegen.

Prinzipiell können die beiden Oxidationszonen beim erfindungsgemäßen Verfahren so gestaltet werden, wie es z.B. in der DE-A 19 837 517, der DE-A 19 910 506, der DE-A 19 910 508 sowie der DE-A 19 837 519 beschrieben ist. Üblicherweise wird die externe Temperierung in den beiden Oxidationszonen, gegebenenfalls in Mehrzonenreaktorsystemen, in an sich bekannter Weise an die spezielle Reaktionsgasgemischzusammensetzung sowie Katalysatorbeschickung angepaßt.

Der für die erfindungsgemäß erforderliche wenigstens eine Partialzone als Oxidationsmittel insgesamt benötigte molekulare Sauerstoff kann dem Beschickungsgasgemisch der wenigstens einen Partialzone in seiner Gesamtmenge vorab zugegeben werden. Selbstverständlich kann aber auch, z.B. bei der Herstellung von Acrylsäure, nach der ersten Partialzone mit Sauerstoff ergänzt werden. Letzteres ist bei der Acrylsäureherstellung bevorzugt.

Vorzugsweise wird dabei in der ersten Oxidationszone (Propylen → Acrolein) ein molares Verhältnis Propylen : molekularer Sauerstoff von 1 : 1 bis 3, häufig 1 : 1,5 bis 2 eingestellt. Ähnliche numerische Werte eignen sich für das molare Verhältnis Acrolein: molekularer Sauerstoff in der zweiten Oxidationszone (1 : 0,5 bis 1,5 wäre bevorzugt) für die Partialoxidation von Acrolein zu Acrylsäure.

In beiden Oxidationszonen wirkt sich dabei ein Überschuß an molekularem Sauerstoff in der Regel vorteilhaft auf die Kinetik der Gasphasenoxidation aus. Im Unterschied zu den Verhältnissen in der erfindungsgemäß anzuwendenden Dehydrierung werden die thermodynamischen Verhältnisse in der wenigstens einen Partialoxidation durch das molare Reaktandenverhältnis im wesentlichen nicht beeinflußt, da die heterogen katalysierte Gasphasen-Partialoxidation

des Propylens zu Acrylsäure kinetischer Kontrolle unterliegt. Prinzipiell kann daher z.B. in der ersten Oxidationszone auch das Propylen gegenüber dem molekularen Sauerstoff im molaren Überschuß vorgelegt werden. In diesem Fall kommt dem überschüssigen Propylen faktisch die Rolle eines Verdünnungsgases zu.

Prinzipiell ist die heterogen katalysierte Gasphasen-Partialoxidation von Propylen zu Acrylsäure aber auch in einer einzigen Oxidationszone realisierbar. In diesem Fall erfolgen beide Reaktionsschritte in einem Oxidationsreaktor der mit einem Katalysator beschickt ist, der die Umsetzung beider Reaktionsschritte zu katalysieren vermag. Selbstredend kann sich auch die Katalysatorbeschickung innerhalb der Oxidationszone längs der Reaktionskoordinate kontinuierlich oder abrupt ändern. Natürlich kann bei einer Ausführungsform der erfindungsgemäß anzuwendenden wenigstens einen Partialoxidation in Gestalt zweier hintereinandergeschalteter Oxidationszonen aus dem das die erste Oxidationszone verlassenden Produktgasgemisch in selbigem enthaltenes, in der ersten Oxidationszone als Nebenprodukt entstandenes, Kohlenoxid und Wasserdampf bei Bedarf vor der Weiterleitung in die zweite Oxidationszone teilweise oder vollständig abgetrennt werden. Vorzugsweise wird man erfindungsgemäß eine Verfahrensweise wählen, die solch einer Abtrennung nicht bedarf.

Als Quelle für den in der wenigstens einen Partialoxidation und/ oder -ammoxidation benötigten molekularen Sauerstoff, der dem Gasgemisch 1 bzw. 1' vor dessen Verwendung zur Beschickung der Partialzone zugemischt wird, kommen sowohl reiner molekularer Sauerstoff als auch mit Inertgas wie $CO_2$, CO, Edelgasen, $N_2$ und/ oder gesättigten Kohlenwasserstoffen verdünnter molekularer Sauerstoff in Betracht.

In zweckmäßiger Weise wird man wenigstens zur Deckung eines Teilbedarfs an molekularem Sauerstoff Luft als Sauerstoffquelle verwenden.

Durch Zudosieren von kalter Luft zu heißem Gasgemisch 1 bzw. 1' kann im Rahmen des erfindungsgemäßen Verfahrens auch auf direktem Weg eine Abkühlung des Gasgemisches 1 bzw. 1' bewirkt werden.

Das die erfindungsgemäß anzuwendende Partialzone verlassende Produktgasgemisch ist im Fall einer Herstellung von Acrolein und/ oder Acrylsäure in der Regel im wesentlichen zusammengesetzt aus dem Zielprodukt Acrolein oder Acrylsäure oder dessen Gemisch mit Acrolein, nicht umgesetztem molekularem Sauerstoff, Propan, nicht umgesetztem Propylen, molekularem Stickstoff, als Nebenprodukt entstandenem und/oder als Verdünnungsgas mitverwendetem Wasserdampf, als Nebenprodukt und/oder als Verdünnungsgas mitverwendeten Kohlenoxiden, sowie geringen Mengen sonstiger niederer Aldehyde, niederer Alkancarbonsäuren (z.B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure), gegebenenfalls weiteren Kohlenwasserstoffen, wie z.B. $C_4$-Kohlenwasserstoffe (z.B. Buten-1 und eventuell sonstige Butene), und anderer inerter Verdünnungsgase.

Das Zielprodukt kann aus dem Produktgasgemisch in an sich bekannter Weise abgetrennt werden (z.B. durch partielle Kondensation der Acrylsäure oder durch Absorption von Acrylsäure in Wasser oder in einem hochsiedenden hydrophoben organischen Lösungsmittel oder durch Absorption von Acrolein in Wasser oder in wäßrigen Lösungen niederer Carbonsäuren sowie anschließende Aufarbeitung der Absorbate; alternativ kann das Produktgasgemisch auch fraktioniert kondensiert werden; vgl. z.B. EP-A 117146, DE-A 4308087, DE-A 4335172, DE-A 4436243, DE-A 19 924 532 sowie DE-A 19 924 533). Eine Acrylsäureabtrennung kann auch wie in der EP-A 982287, der EP-A 982289, der DE-A 19924532, der DE-A 10115277, der DE-A 19606877, der DE-A 19740252, der DE-A 19627847, der DE-A 10053086 und der EP-A 982288 vorgenommen werden.

Nicht umgesetztes Propylen und/oder Acrolein werden gegebenenfalls gleichfalls abgetrennt und in die Partialzone rückgeführt.

Vorzugsweise wird wie in Fig. 7 der WO/0196271 abgetrennt. Ansonsten können die von Acrylsäure und Acrolein verschiedenen wesentlichen Bestandteile des nach der Zielproduktabtrennung verbleibenden Restgases je nach Bedarf, verwendetem Roh-Propan und verwendetem Dehydrier-/Oxidehydrierkatalysator jeweils für sich abgetrennt und/oder mit dem Propan als Kreisgas (Rückführstrom) in die Beschickung des ersten Schrittes des erfindungsgemäßen Verfahrens rückgeführt werden. Selbstverständlich kann aber auch das nicht umgesetzte Propan im Gemisch mit dem nicht umgesetzten Propylen für sich (als Rückführstrom) in diese Beschickung rückgeführt werden. Bei kontinuierlicher Ausführung des erfindungsgemäßen Verfahrens erfolgt so eine kontinuierliche Umsetzung von Propan zu Acrylsäure und/oder Acrolein.

Die Abtrennung von Propan und Propen aus dem nach der Zielproduktabtrennung verbleibenden Restgas (es enthält in der Regel $O_2$, CO, $CO_2$, $H_2O$, $N_2$, Edelgase sowie sonstige niedere Aldehyde, niederer Alkancarbonsäuren (z.B. Essigsäure, Ameisensäure und Propionsäure) sowie Maleinsäureanhydrid, Benzaldehyd, aromatische Carbonsäuren und aromatische Carbonsäureanhydride (z.B. Phthalsäureanhydrid und Benzoesäure) und Kohlenwasserstoffe, z.B. $C_4$-Kohlenwasserstoffe (z.B. Buten-1 und eventuell sonstige Butene)) kann, wie bereits beschrieben, durch Absorption mit nachfolgender Desorption und/oder Strippung (sowie Absorptionsmittelwiederverwendung) in einem hochsiedenden hydrophoben organischen Lösungsmittel erfolgen. Weitere Trennmöglichkeiten sind Adsorption, Rektifikation, Membranverfahren und partielle Kondensation. Bevorzugt werden die genannten Trennverfahren bei erhöhtem Druck ausgeführt.

Bei Verwendung von Dehydrierkatalysatoren, die gegenüber Sauerstoff oder Sauerstoff enthaltenden Verbindungen empfindlich sind, wird man diese Oxygenate vor einer Rückführung von Kreisgas in die Beschickung des ersten Schrittes des erfindungsgemäßen Verfahrens aus dem Kreisgas abtrennen. Eine solche Sauerstoffabtrennung kann auch sinnvoll sein um eine Totalxidation des Propans in der Dehydrierstufe zu vermeiden. Die Dehydrierkatalysatoren der DE-A 19 937 107 sind nicht empfindlich gegen Oxygenate (insbesondere jene gemäß Beispiel 1 bis 4 der DE-A).

Eine andere Abtrennmöglichkeit bietet, wie gleichfalls bereits erwähnt, die fraktionierte Destillation. Vorzugsweise wird eine fraktionierte Druckdestillation bei tiefen Temperaturen durchgeführt. Der anzuwendende Druck kann z.B. 1013,25 bis 10132,5 kPa (10 bis 100 bar) betragen. Als Rektifikationskolonnen können Füllkörperkolonnen, Bodenkolonnen oder Packungskolonnen eingesetzt werden. Als Bodenkolonnen eignen sich solche mit Dual-Flow-Böden, Glockenböden oder Ventilböden. Das Rücklaufverhältnis kann z.B. 1 bis 10 betragen. Andere Trennmöglichkeiten bilden z.B. Druckextraktion, Druckwechseladsorption, Druckwäsche, partielle Kondensation und Druckextraktion.

Selbstverständlich kann erfindungsgemäß, z.B. dann, wenn nach dem ersten Schritt des erfindungsgemäßen Verfahrens eine Abtrennung von Nebenkomponenten (z.B. $C_4$-Kohlenwasserstoffen (z.B. Buten-1, n-Butan, iso-Butan und eventuell sonstige Butene)) integriert ist oder die störenden $C_4$-Kohlenwasserstoffe sich nicht aufpegeln (z.B. wenn sie in der Partialzone an geeigneten Katalysatoren verbrannt werden), auch die Gesamtmenge an Restgas (als Rückführstrom) in die Beschickung des ersten Schritts des erfindungsgemäßen Verfahrens rückgeführt werden. In diesem Fall kann sich der Auslaß für von Propan, Propylen und molekularem Sauerstoff verschiedene Gasbestandteile ausschließlich zwischen dem Gasgemisch 1 und dem Gasgemisch 1' befinden.

Selbstredend kann ein weiterer Auslaß nach der Zielproduktabtrennung eingerichtet sein. Falls das in eine Propandehydrierung rückgeführte Kreisgas Kohlenmonoxid enthält, kann dieses, bevor mit frischem Roh-Propan ergänzt wird, katalytisch zu $CO_2$ verbrannt werden. Die dabei freigesetzte Reaktionswärme kann zum Aufheizen auf die Dehydriertemperatur Anwendung finden.

Eine katalytische Nachverbrennung von im Restgas enthaltenem CO zu $CO_2$ kann auch dann empfehlenswert sein, wenn eine Abtrennung der Kohlenoxide aus dem Restgas vor dessen Rückführung als Kreisgas in Propandehydrierung und/oder -oxidehydrierung angestrebt wird, läßt sich doch $CO_2$ vergleichsweise einfach abtrennen (z.B. durch Wäsche mit einer basischen Flüssigkeit). Eine solche katalytische CO-Nachverbrennung kann auch in der Dehydrierzone durchgeführt werden, z.B. an den oben beschriebenen Dehydrierkatalysatoren (z.B. jenen der DE-A 19937107, insbesondere die aus Bsp. 1 bis 4 dieser DE-A).

Natürlich kann auch so verfahren werden, daß man einen Teil des Restgases unverändert in die Propandehydrierung und/oder -oxidehydrierung rückführt und nur aus dem verbliebenen Teil Propan und Propylen im Gemisch abtrennt und ebenfalls in die Propandehydrierung und/oder -oxidehydrierung und/oder in die wenigstens eine Partialzone rückführt. Im letzteren Fall vereint man den verbliebenen Teil des Restgases zweckmäßigerweise mit dem Gasgemisch 1 bzw. Gasgemisch 1'.

Im Rahmen einer fraktionierten Destillation des Restgases kann eine Trennlinie z.B. so gelegt werden, daß im Auftriebsteil der Rektifikationskolonne im wesentlichen alle diejenigen Bestandteile abgetrennt werden und am Kopf der Kolonne abgezogen werden können, deren Siedepunkt tiefer als der Siedepunkt von Propylen liegt. Diese Bestandteile werden in erster Linie die Kohlenoxide CO und $CO_2$ sowie nicht umgesetzter Sauerstoff und Ethylen sowie Methan und $N_2$ sein. Im Sumpf können z.B. schwerer siedende $C_4$-Kohlenwasserstoffe abgetrennt werden.

Wird als erster Schritt des erfindungsgemäßen Verfahrens eine heterogen katalysierte Oxidehydrierung des Propans angewendet, können Nebenkomponentenabtrennungen auch immer dann mit vorgenommen werden, wenn in den Schriften DE-A 19837520, DE-A 19837517, DE-A 19837519 und DE-A 19837518 Abtrennungen von molekularem Stickstoff vorgenommen werden.

Beispiele

Heterogen katalysierte Gasphasenpartialoxidation von Propylen in zwei hintereinandergeschalteten Festbettreaktoren mittels verschiedener Propylen und Propan enthaltender Gasgemische 2

A) Beschreibung der allgemeinen Verfahrensbedingungen

1. Erster Festbettreaktor für den Schritt der Partialoxidation des Propylens zu Acrolein

| | |
|---|---|
| Verwendetes Wärmeaustauschmittel: | Salzschmelze, bestehend aus<br>53 Gew.-% Kaliumnitrat,<br>40 Gew.-% Nariumnitrit und<br>7 Gew.-% Natriumnitrat. |

(fortgesetzt)

| | |
|---|---|
| Abmessung des Kontaktrohres: | 4200 mm Gesamtlänge,<br>26 mm Innendurchmesser,<br>30 mm Außendurchmesser,<br>2 mm Wandstärke. |
| Reaktor: | Besteht aus einem doppelwandigen Zylinder aus Edelstahl (zylindrisches Führungsrohr, umgeben von einem zylindrischen Außenbehälter). Die Wanddicken betrugen überall 2 bis 5 mm.<br>Der Innendurchmesser des äußeren Zylinders betrug 168 mm. Der Innendurchmesser des Führungsrohres betrug ca. 60 mm.<br>Oben und unten war der doppelwandige Zylinder durch einen Deckel beziehungsweise Boden abgeschlossen.<br>Das Kontaktrohr war im zylindrischen Behälter so untergebracht, dass es am oberen bzw. unteren Ende desselben (abgedichtet) durch den Deckel bzw. Boden jeweils um 250 mm herausgeführt war.<br>Das Wärmeaustauschmittel war im zylindrischen Behälter eingeschlossen. Um über die gesamte im zylindrischen Behälter befindliche Kontaktrohrlänge (3700 mm) möglichst gleichmäßige thermische Randbedingungen an der Außenwand des Kontaktrohres zu gewährleisten, wurde das Wärmeaustauschmittel durch Einperlen von Stickstoff im zylindrischen Behälter umgewälzt.<br>Mittels des aufsteigenden Stickstoffs wurde das Wärmeaustauschmittel im zylindrischen Führungsrohr von unten nach oben befördert, um dann im Zwischenraum zwischen zylindrischem Führungsrohr und zylindrischem Außenbehälter wieder nach unten zu strömen (eine Umwälzung gleicher Güte kann auch durch Umpumpen (z.B. Propellerpumpen) erreicht werden). Durch eine auf den Außenmantel aufgebrachte elektrische Heizung könnte die Temperatur des Wärmeaustauschmittels auf das gewünschte Niveau geregelt werden. Im übrigen bestand Luftkühlung. |
| Reaktorbeschickung: | Über den Reaktor betrachtet wurden Salzschmelze und Reaktionsgasgemisch (das jeweilige Gasgemisch 2) im Gegenstrom geführt. Das Reaktionsgasgemisch trat oben in den Reaktor ein. Es wurde jeweils mit einer Temperatur von 250°C ins Reaktionsrohr geführt.<br>Die Salzschmelze trat unten mit einer Temperatur $T^{ein}$ in das zylindrische Führungsrohr ein und oben mit einer Temperatur $T^{aus}$ aus dem zylindrischen Führungsrohr aus. Der Unterschied zwischen $T^{ein}$ und $T^{aus}$ betrug etwa 2°C. $T^{mittel} = (T^{ein} + T^{aus})/2$. |
| Kontaktrohrbeschickung: (von oben nach unten) | Abschnitt A: 50 cm Länge Vorschüttung aus Steatit-Ringen der Geometrie 7 mm x 7 mm x 4 mm (Außendurchmesser x Länge x Innendurchmesser).<br>Abschnitt B: 100 cm Länge Kontaktrohrbeschickung mit einem homogenen Gemisch aus 30 Gew.-% an SteatitRingen der Geometrie 5 mm x 3 mm x 2 mm (Außendurchmesser x Länge x Innendurchmessser) und 70 Gew.-% Vollkatalysator aus Abschnitt C.<br>Abschnitt C: 170 cm Länge Katalysatorbeschickung mit ringförmigen (5 mm x 3 mm x 2 mm = Außendurchmesser x Länge x Innendurchmesser) Vollkatalysator gemäß Beispiel 1 der DE-A 10046957.<br>Abschnitt D: 50 cm Länge Nachschüttung aus Steatit-Ringen der Geometrie 7 mm x 7 mm x 4 mm (Außendurchmesser x Länge x Innendurchmesser). |

Belastung des Reaktors
mit Reaktionsgasausgangsgemisch: in allen Fällen 3860 g/h an Gasgemisch 2.
Propylenbelastung der Katalysatorbeschickung: 100 NLiter/1·h.
2. Beschreibung der Zwischenkühlung und Sauerstoffzwischeneinspeisung
Das den ersten Festbettreaktor verlassende Produktgasgemisch wurde zum Zweck der Zwischenkühlung (indirekt mittels Luft) durch ein Verbindungsrohr (Länge = 400 mm, Innendurchmesser = 26 mm, Wanddicke = 2 mm, Material = Edelstahl) geführt, das auf einer Länge von 200 mm zentriert untergebracht, mit einer Inertschüttung aus Staatitkugeln des Durchmessers 6 mm beschickt und unmittelbar an das Kontraktrohr des ersten Festbettreaktors angeflanscht war.
Das Gasgemisch trat in allen Fällen mit einer Temperatur von mehr als 310°C in das Verbindungsrohr ein und verließ es mit einer Temperatur von etwa 140°C. Anschließend wurden dem Gasgemisch als Sauerstoffquelle 290 NLiter/h an komprimierter Luft zugemischt.
Das dabei resultierende Beschickungsgasgemisch wurde mit einer Temperatur von 220°C dem Festbettreaktor für den Schritt der Partialoxidation des Acroleins zu Acrylsäure zugeführt.
3. Zweiter Festbettreaktor für den Schritt der Partialoxidation des Acroleins zu Acrylsäure
Es wurde ein Festbettreaktor verwendet, der mit jenem für den ersten Schritt baugleich war. Salzschmelze und Reaktionsgasgemisch wurden über den Reaktor betrachtet im Gleichstrom geführt. Die Salzschmelze trat unten ein, das Reaktionsgasgemisch ebenfalls.

Die Kontaktrohrbeschickung (von unten nach oben) war:

Abschnitt A: 20 cm Länge
Vorschüttung aus Steatit-Ringen der Geometrie
7 mm x 7 mm x 4 mm
(Außendurchmesser x Länge x Innendurchmesser).

Abschnitt B: 100 cm Länge
Katalysatorbeschickung mit einem homogenen Gemisch aus
30 Gew.-% an Steatit-Ringen der Geometrie 7 mm x 3 mm x 4 mm
(Außendurchmesser x Länge x Innendurchmesser) und 70 Gew.-% Schalenkatalysator aus Abschnitt C.

Abschnitt C: 200 cm Länge
Katalysatorbeschickung mit ringförmigem
(7 mm x 3 mm x 4 mm = Außerdurchmesser x Länge x Innendurchmesser) Schalenkatalysator gemäß Herstellungsbeispiel 5 der DE-A 10046928.

Abschnitt D: 50 cm Länge
Nachschüttung aus Steatit-Ringen der Geometrie 7 mm x 7 mm x 4 mm
(Außendurchmesser x Länge x Innendurchmesser).

Der zweite Reaktor wurde in allen Fällen mit nominell ca. 4240 g/h an Beschickungsgasgemisch belastet. $T^{mittel}$ ist wie für den ersten Festbettreaktor definiert.

In allen nachfolgenden Beispielen wurde der Propylenumsatz im ersten Reaktor auf 97,7 mol-% und der Acroleinumsatz im zweiten Reaktor auf 99,3 mol-% eingestellt.

Die dabei in Abhängigkeit von der Zusammensetzung des Gasgemisches 2 erforderlichen $T^{mittel}$, sowie die dabei in Abhängigkeit von der Zusammensetzung des Gasgemisches 2 erzielten, auf über beide Reaktoren umgesetztes Propylen bezogenen, Ausbeuten $A^{AA}$ (mol-%) an Acrylsäure und Selektivitäten der Kohlenoxidbildung $S^{COx}$ (mol-%) weisen in den einzelnen Beispielen die nachfolgenden Werte auf.

B) Beispiel 1

Die Zusammensetzung des Gasgemisches 2 war:

6,18 Vol.-% Propylen,
33,1 Vol.-% Propan,
12,3 Vol.-% Sauerstoff,
0,15 Vol.-% $CO_x$,
46,7 Vol.-% $N_2$, und
1,63 Vol.-% $H_2O$.

$A^{AA}$ = 86,1 mol-% $T^{mittel}$ 1. Reaktor = 316°C.
$S^{COx}$ = 9,2 mol-% $T^{mittel}$ 2. Reaktor = 274°C.

C) Beispiel 2

Die Zusammensetzung des Gasgemisches 2 war:

6,04 Vol.-% Propylen,
42,3 Vol.-% Propan,
10,4 Vol.-% Sauerstoff,
0,15 Vol.-% $CO_x$,
39,5 Vol.-% $N_2$, und
1,60 Vol.-% $H_2O$.

$A^{AA}$ = 85,2 mol-% $T^{mittel}$ 1. Reaktor = 322°C.
$S^{COx}$ = 9,9 mol-% $T^{mittel}$ 2. Reaktor = 278°C.

D) Beispiel 3

Die Zusammensetzung des Gasgemisches 2 war:

0,20 Vol.-% Ethan,
6,14 Vol.-% Propylen,
33,0 Vol.-% Propan,
12,2 Vol.-% Sauerstoff,
0,16 Vol.-% $CO_x$,
46,6 Vol.-% $N_2$, und
1,65 Vol.-% $H_2O$.

$A^{AA}$ = 86,1 mol-% $T^{mittel}$ 1. Reaktor = 316°C.
$S^{COx}$ = 9,2 mol-% $T^{mittel}$ 2. Reaktor = 274°C.

E) Beispiel 4

Die Zusammensetzung des Gasgemisches 2 war:

0,22 Vol.-% Ethylen,
6,13 Vol.-% Propylen,
33,0 Vol.-% Propan,
12,2 Vol.-% Sauerstoff,
0,16 Vol.-% $CO_x$,
46,6 Vol.-% N2, und
1,64 Vol.-% $H_2O$.

$A^{AA}$ = 86,1 mol-% $T^{mittel}$ 1. Reaktor = 316°C.
$S^{COx}$ = 9,2 mol-% $T^{mittel}$ 2. Reaktor = 274°C.

F) Beispiel 5

Die Zusammensetzung des Gasgemisches 2 war:

0,20 Vol.-% n-Butan,
6,14 Vol.-% Propylen,
33,0 Vol.-% Propan,
12,2 Vol.-% Sauerstoff,
0,16 Vol.-% $CO_x$,
46,6 Vol.-% $N_2$, und
1,65 Vol.-% $H_2O$.

$A^{AA}$ = 85,2 mol-% $T^{mittel}$ 1. Reaktor = 316, 5°C.
$S^{COx}$ = 9,mol-% $T^{mittel}$ 2. Reaktor = 274°C.

G) Beispiel 6

Die Zusammensetzung des Gasgemisches 2 war:

2,02 Vol.-% n-Butan,
5,98 Vol.-% Propylen,
32,4 Vol.-% Propan,
12,0 Vol.-% Sauerstoff,
0,16 Vol.-% $CO_x$,
45,8 Vol.-% $N_2$, und
1,64 Vol.-% $H_2O$.

Der gewünschte Propylenumsatz war nicht mehr durch eine Erhöhung von $T^{mittel}$ im Rahmen der Katalysatorverträglichkeit aufrechtzuerhalten.

H) Beispiel 7

Die Zusammensetzung des Gasgemisches 2 war:

0,05 Vol.-% Buten-1,
6,16 Vol.-% Propylen,
33,0 Vol.-% Propan,
12,3 Vol.-% Sauerstoff,
0,16 Vol.-% $CO_x$,
46,7 Vol.-% $N_2$, und
1,70 Vol.-% $H_2O$.

$A^{AA}$ = 85,1 mol-% $T^{mittel}$ 1. Reaktor = 318°C.
$S^{COx}$ = 10 mol-% $T^{mittel}$ 2. Reaktor = 281°C.

I) Beispiel 8

Die Zusammensetzung des Gasgemisches 2 war:

0,09 Vol.-% Buten-1,
6,16 Vol.-% Propylen,
32,9 Vol.-% Propan,
12,3 Vol.-% Sauerstoff,
0,15 Vol.-% $CO_x$,
46,8 Vol.-% $N_2$, und
1,68 Vol.-% $H_2O$.

$A^{AA}$ = 85,0 mol-% $T^{mittel}$ 1. Reaktor = 320°C.
$S^{COx}$ = 10, mol-% $T^{mittel}$ 2. Reaktor = 287°C.

J) Beispiel 9

Die Zusammensetzung des Gasgemisches 2 war:

0,20 Vol.-% Buten-1,
6,19 Vol.-% Propylen,
32,7 Vol.-% Propan,
12,3 Vol.-% Sauerstoff,
0,18 Vol.-% $CO_x$,
46,7 Vol.-% $N_2$, und

1,71 Vol.-% $H_2O$.

Der gewünschte Propylenumsatz war nicht mehr durch eine Erhöhung von $T^{mittel}$ im Rahmen der Katalysatorverträglichkeit aufrechtzuerhalten.

## Patentansprüche

1. Verfahren zur Herstellung von wenigstens einem partiellen Oxidations- und/oder Ammoxidationsprodukt des Propylens, bei dem man

   a) in einem ersten Schritt Roh- Propan im Beisein und/oder unter Ausschluß von Sauerstoff einer homogenen und/oder einer heterogen katalysierten Dehydrierung und/oder Oxidhydrierung unterwirft, wobei ein Propan und Propylen enthaltendes Gasgemisch 1 erhalten wird,
   und
   b) aus im ersten Schritt gebildetem Gasgemisch 1, von den darin enthaltenen, von Propan und Propylen verschiedenen, Bestandteilen gegebenenfalls eine Teilmenge abtrennt und/oder in andere Verbindungen wandelt, wobei aus dem Gasgemisch 1 jeweils ein Propan und Propylen sowie auch von Sauerstoff, Propan und Propylen verschiedene Verbindungen enthaltendes Gasgemisch 1' erzeugt wird, und in wenigstens einem weiteren Schritt
   c) Gasgemisch 1 und/oder Gasgemisch 1' als Bestandteil eines Gasgemisches 2 einer heterogen katalysierten Gasphasen-Partialoxidation und/oder partiellen Gasphasen-Ammoxidation von im Gasgemisch 1 und/oder Gasgemisch 1' enthaltenem Propylen unterwirft,

   **dadurch gekennzeichnet, daß**
   der Gehalt des Gasgemisches 2 an Buten-1 ≤ 1 Vol.-% beträgt, wobei das erfindungsgemäße Verfahren in einer Reaktionszone an einer Katalysatorbeschickung durchgeführt wird, deren Aktivmasse wenigstens eine Multimetalloxidmasse ist, die die Elemente Mo, V, wenigstens eines der beiden Elemente Te und Sb, und wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au und In in Kombination enthält.

2. Verfahren nach Anspruch 1, **dadurch gekennzeichnet, daß** der Gehalt des Gasgemisches 2 an Buten-1 > 0,003 Vol.-% beträgt.

3. Verfahren nach Anspruch 1, **dadurch gekennzeichnet, daß** der Gehalt des Gasgemisches 2 an Buten-1 > 0,001 Vol.-% beträgt.

4. Verfahren nach einem der Ansprüche 1 bis 3, **dadurch gekennzeichnet, daß** der Gehalt des Gasgemisches 2 an trans-Buten-2 ≤ 0,05 Vol.-% beträgt.

5. Verfahren nach einem der Ansprüche 1 bis 4, **dadurch gekennzeichnet, daß** der Gehalt des Gasgemisches 2 an cis-Buten-2 ≤ 0,05 Vol.-% beträgt.

6. Verfahren nach einem der Ansprüche 1 bis 5, **dadurch gekennzeichnet, daß** der Gehalt des Gasgemisches 2 an iso-Buten ≤ 0,05 Vol.-% beträgt.

7. Verfahren nach einem der Ansprüche 1 bis 6, **dadurch gekennzeichnet, daß** der Gesamtgehalt des Gasgemisches 2 an Butenen ≤ 1 Vol.-% beträgt.

8. Verfahren nach einem der Ansprüche 1 bis 6, **dadurch gekennzeichnet, daß** der Gesamtgehalt des Gasgemisches 2 an Butenen ≤ 0,05 Vol.-% beträgt.

9. Verfahren nach einem der Ansprüche 1 bis 8, **dadurch gekennzeichnet, daß** der Gesamtgehalt des Gasgemisches 2 an $C_4$-Kohlenwasserstoffen ≤ 3 Vol.-% beträgt.

10. Verfahren nach einem der Ansprüche 1 bis 9, **dadurch gekennzeichnet, daß** der Gesamtgehalt des Gasgemisches 2 an $C_4$-Kohlenwasserstoffen > 0,05 und < 3 Vol.-% beträgt.

11. Verfahren nach einem der Ansprüche 1 bis 10, **dadurch gekennzeichnet, daß** das Gasgemisch 1' ≥ 0,1 Vol.-%

an von Propan und Propylen sowie Sauerstoff verschiedenen Bestandteilen enthält.

**12.** Verfahren nach einem der Ansprüche 1 bis 11, **dadurch gekennzeichnet, daß** das Gasgemisch 2 bis zu 60 Vol.-% an Propan enthält.

**13.** Verfahren nach einem der Ansprüche 1 bis 11, **dadurch gekennzeichnet, daß** das Gasgemisch 2, 20 bis 40 Vol.-% Propan enthält.

**14.** Verfahren nach einem der Ansprüche 1 bis 11, **dadurch gekennzeichnet, daß** das Gasgemisch 2 die nachfolgenden Gehalte aufweist,

7 bis 15 Vol.-% $O_2$,
5 bis 10 Vol.-% Propylen,
15 bis 40 Vol.-% Propan,
25 bis 60 Vol.-% Stickstoff,
1 bis 5 Vol.-% Summe aus CO, $CO_2$ und $H_2O$ und
0 bis 5 Vol.-% sonstige Bestandteile,

wobei gegebenenfalls enthaltener Ammoniak unberücksichtigt ist.

**15.** Verfahren nach einem der Ansprüche 1 bis 11, **dadurch gekennzeichnet, daß** das Gasgemisch 2 die nachfolgenden Gehalte aufweist,

$H_2O \leq 60$ Vol.-%,
$N_2 \leq 80$ Vol.-%,
$O_{22} > 0, \leq 20$ Vol.-%,
$CO \leq 2$ Vol.-%,
$CO_2 \leq 5$ Vol.-%,
Ethan ≤ 10 Vol.-%,
Ethylen ≤ 5 Vol.-%,
Methan ≤ 5 Vol.-%,
Propan > 0, ≤ 50 Vol.-%,
Cyclopropan ≤ 0,1 Vol.-%,
Propin ≤ 0,1 Vol.-%,
Propadien ≤ 0,1 Vol.-%,
Propylen > 0, ≤ 30 Vol.-%,
$H_2 \leq 30$ Vol.-%,
iso-Butan ≤ 3 Vol.-%,
n-Butan ≤ 3 Vol.-%,
trans-Buten-2 ≤ 1 Vol.-%,
cis-Buten-2 ≤ 1 Vol.-%,
Buten-1 ≤ 1 Vol.-%,
iso-Buten ≤ 1 Vol.-%,
Butadien 1,3 ≤ 1 Vol.-%,
Butadien 1,2 ≤ 1 Vol.-%,
1-Butin ≤ 0,5 Vol.-% und
2-Butin ≤ 0,5 Vol.-%,

wobei gegebenenfalls enthaltener Ammoniak unberücksichtigt ist.

**16.** Verfahren nach einem der Ansprüche 1 bis 15, **dadurch gekennzeichnet, daß** das Roh-Propan ≥ 0,25 Vol.-% an von Propan und Propylen verschiedenen Bestandteilen enthält.

**17.** Verfahren nach einem der Ansprüche 1 bis 16, **dadurch gekennzeichnet, daß** das Roh-Propan bis zu 6 Vol.-% an $C_4$-Kohlenwasserstoffen enthält.

**18.** Verfahren nach einem der Ansprüche 1 bis 16, **dadurch gekennzeichnet, daß** das Roh-Propan 0,1 bis 6 Vol.-% an $C_4$-Kohlenwasserstoffen enthält.

**19.** Verfahren nach einem der Ansprüche 1 bis 18, **dadurch gekennzeichnet, daß** das Roh-Propan bis zu 0,5 Vol.-% Buten-1 enthält.

**20.** Verfahren nach einem der Ansprüche 1 bis 18, **dadurch gekennzeichnet, daß** das Roh-Propan 5 Vol.ppm bis 0,5 Vol.-% Buten-1 enthält.

**21.** Verfahren nach einem der Ansprüche 1 bis 20, **dadurch gekennzeichnet, daß** das Roh-Propan insgesamt bis zu 0,5 Vol.-% an Butenen enthält.

**22.** Verfahren nach einem der Ansprüche 1 bis 20, **dadurch gekennzeichnet, daß** das Roh-Propan insgesamt 5 Vol.ppm bis zu 0,5 Vol.-% an Butenen enthält.

**23.** Verfahren nach einem der Ansprüche 1 bis 22, **dadurch gekennzeichnet, daß** das Roh-Propan die folgende Spezifikation erfüllt:

Gehalt an Propan ≥ 90 Vol.-%,
Gesamtgehalt aus Propan und Propylen ≤ 99 Vol.-%,
Gesamtgehalt an $C_4$-Kohlenwasserstoffen ≤ 6 Vol.-%,
Gehalt an Buten-1 ≤ 0,5 Vol.-%,
Gesamtgehalt an Butenen ≤ 0,5 Vol.-%,
Gehalt an Ethan ≤ 10 Vol.-%,
Gehalt an Ethylen ≤ 5 Vol.-%,
Gehalt an Methan ≤ 5 Vol.-%,
Gehalt an Cyclopropan ≤ 0,1 Vol.-%,
Gehalt an Propylen ≤ 10 Vol.-%,
Gesamtgehalt an von Propan und Propylen verschiedenen $C_3$-Kohlenwasserstoffen ≤ 0,3 Vol.-%,
Gesamtgehalt an $C_5$-Kohlenwasserstoffen ≤ 0,3 Vol.-%,
und Gesamtgehalt an $C_6$-bis $C_8$-Kohlenwasserstoffen
≤ 600 Vol.ppm.

**24.** Verfahren nach einem der Ansprüche 1 bis 23, **dadurch gekennzeichnet, daß** der Propanumsatz im ersten Schritt ≥ 5 mol.-% bis ≤ 30 mol.-% beträgt.

**25.** Verfahren nach einem der Ansprüche 1 bis 24, **dadurch gekennzeichnet, dass** man aus dem Produktgasgemisch der Gasphasen- Partialoxidation und/oder partiellen Gasphasen-Ammoxidation von dem wenigstens einen partiellen Oxidations- und/oder Ammoxidationsprodukt des Propylens abtrennt und wenigstens in diesem Produktgasgemisch enthaltenes nicht umgesetztes Propan in den ersten Schritt und/oder in die Gasphasen-Partialoxidation und/oder partielle Gasphasen-Ammoxidation rückführt.

**26.** Verfahren nach Anspruch 1, **dadurch gekennzeichnet, daß** die Aktivmasse wenigstens eine Multimetalloxidmasse ist, die die Elementstöchiometrie I

$$Mo_1V_bM^1_cM^2_d \qquad (I)$$

mit

$M^1$ = Te und/oder Sb,
$M^2$ = wenigstens eines der Elemente aus der Gruppe umfassend Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au und In,
b = 0,01 bis 1,
c = > 0 bis 1 und
d = > 0 bis 1

enthält.

**27.** Verfahren nach einem der Ansprüche 1 oder 26, **dadurch gekennzeichnet, daß** das Röntgendiffraktogramm der

wenigstens einen Multimetalloxidaktivmasse ein Röntgendiffraktogramm aufweist, das Beugungsreflexe h und i zeigt, deren Scheitelpunktlagen bei den Beugungswinkeln 22,2 ± 0,5° (h) und 27,3 ± 0,5° (i) liegen.

**28.** Verfahren nach einem der Ansprüche 1 bis 27, **dadurch gekennzeichnet, daß** das wenigstens eine partielle Oxidations- und/oder Ammoxidationsprodukt des Propylens wenigstens eine Verbindung aus der Gruppe umfassend Propylenoxid, Acrolein, Acrylsäure und Acrylnitril ist.

**29.** Verfahren nach einem der Ansprüche 1 bis 28, **dadurch gekennzeichnet, dass** man in einem ersten Schritt.Roh-Propan im Beisein und/oder unter Ausschluß von Sauerstoff einer heterogen katalysierten Dehydrierung unterwirft und Gasgemisch 1 einer heterogen katalysierten Gasphasen-Ammoxidation von im Gasgemisch 1 enthaltenem Propylen unterwirft.

**30.** Verfahren nach einem der Ansprüche 1 bis 29, **dadurch gekennzeichnet, dass** man in einem ersten Schritt Roh-Propan im Beisein und/oder unter Ausschluß von Sauerstoff sowie im Beisein von Wasserdampf einer heterogen katalysierten Dehydrierung unterwirft und aus im ersten Schritt gebildetem Gasgemisch 1 Wasserdampf durch Kondensieren teilweise oder vollständig abtrennt und das dabei erzeugte Gasgemisch 1' einer heterogen katalysierten Gasphasen-Partialoxidation und/oder partiellen Gasphasen-Ammoxidation von im Gasgemisch 1' enthaltenem Propylen unterwirft.

**31.** Verfahren nach einem der Ansprüche 1 bis 30, **dadurch gekennzeichnet, dass** man in einem ersten Schritt Roh-Propan einer autothermen heterogen katalysierten Dehydrierung unterwirft.

**32.** Verfahren nach einem der Ansprüche 1 bis 31, **dadurch gekennzeichnet, dass** man das Gasgemisch 1 und/oder Gasgemisch 1' als Bestandteil eines Gasgemisches 2 einer heterogen katalysierten partiellen Gasphasen-Ammoxidation von im Gasgemisch 1 und/oder Gasgemisch 1' enthaltenem Propylen unterwirft.

**33.** Verfahren zur Herstellung von wenigstens einem partiellen Oxidations- und/oder Ammoxidationsprodukt des Propylens, bei dem man

a) in einem ersten Schritt Roh- Propan im Beisein und/oder unter Ausschluß von Sauerstoff einer homogenen und/oder einer heterogen katalysierten Dehydrierung und/oder Oxidhydrierung unterwirft, wobei ein Propan und Propylen enthaltendes Gasgemisch 1 erhalten wird,
und
b) aus im ersten Schritt gebildetem Gasgemisch 1, von den darin enthaltenen, von Propan und Propylen verschiedenen, Bestandteilen gegebenenfalls eine Teilmenge abtrennt und/oder in andere Verbindungen wandelt, wobei aus dem Gasgemisch 1 jeweils ein Propan und Propylen sowie auch von Sauerstoff, Propan und Propylen verschiedene Verbindungen enthaltendes Gasgemisch 1' erzeugt wird, und in wenigstens einem weiteren Schritt
c) Gasgemisch 1 und/oder Gasgemisch 1' als Bestandteil eines Gasgemisches 2 einer heterogen katalysierten Gasphasen-Partialoxidation und/oder partiellen Gasphasen-Ammoxidation von im Gasgemisch 1 und/oder Gasgemisch 1' enthaltenem Propylen unterwirft,

**dadurch gekennzeichnet, daß**
der Gehalt des Gasgemisches 2 an Buten-1 ≤ 1 Vol.-% beträgt, wobei der erste Schritt in einer getrennten Reaktionszone durchgeführt wird
und in der heterogen katalysierten Gasphasen-Partialoxidation ein Katalysator mitverwendet wird, dessen Aktivmasse die Elemente Mo und V enthält.

**34.** Verfahren nach Anspruch 33, **dadurch gekennzeichnet, daß** der Gehalt des Gasgemisches 2 an Buten-1 > 0,003 Vol.-% beträgt.

**35.** Verfahren nach Anspruch 33, **dadurch gekennzeichnet, daß** der Gehalt des Gasgemisches 2 an Buten-1 > 0,001 Vol.-% beträgt.

**36.** Verfahren nach einem der Ansprüche 33 bis 35, **dadurch gekennzeichnet, daß** der Gehalt des Gasgemisches 2 an trans-Buten-2 ≤ 0,05 Vol.-% beträgt.

**37.** Verfahren nach einem der Ansprüche 33 bis 36, **dadurch gekennzeichnet, daß** der Gehalt des Gasgemisches 2 an cis-Buten-2 ≤ 0,05 Vol.-% beträgt.

**38.** Verfahren nach einem der Ansprüche 33 bis 37, **dadurch gekennzeichnet, daß** der Gehalt des Gasgemisches 2 an iso-Buten ≤ 0,05 Vol.-% beträgt.

**39.** Verfahren nach einem der Ansprüche 33 bis 38, **dadurch gekennzeichnet, daß** der Gesamtgehalt des Gasgemisches 2 an Butenen ≤ 1 Vol.-% beträgt.

**40.** Verfahren nach einem der Ansprüche 33 bis 38, **dadurch gekennzeichnet, daß** der Gesamtgehalt des Gasgemisches 2 an Butenen ≤ 0,05 Vol.-% beträgt.

**41.** Verfahren nach einem der Ansprüche 33 bis 40, **dadurch gekennzeichnet, daß** der Gesamtgehalt des Gasgemisches 2 an $C_4$-Kohlenwasserstoffen ≤ 3 Vol.-% beträgt.

**42.** Verfahren nach einem der Ansprüche 33 bis 40, **dadurch gekennzeichnet, daß** der Gesamtgehalt des Gasgemisches 2 an $C_4$-Kohlenwasserstoffen >< 0,05 und < 3 Vol.-% beträgt.

**43.** Verfahren nach einem der Ansprüche 33 bis 42, **dadurch gekennzeichnet, daß** das Gasgemisch 1' ≥ 0,1 Vol.-% an von Propan und Propylen sowie Sauerstoff verschiedenen Bestandteilen enthält.

**44.** Verfahren nach einem der Ansprüche 33 bis 43, **dadurch gekennzeichnet, daß** das Gasgemisch 2 bis zu 60 Vol.-% an Propan enthält.

**45.** Verfahren nach einem der Ansprüche 33 bis 43, **dadurch gekennzeichnet, daß** das Gasgemisch 2, 20 bis 40 Vol.-% Propan enthält.

**46.** Verfahren nach einem der Ansprüche 33 bis 43, **dadurch gekennzeichnet, daß** das Gasgemisch 2 die nachfolgenden Gehalte aufweist,

7 bis 15 Vol.-% $O_2$,
5 bis 10 Vol.-% Propylen,
15 bis 40 Vol.-% Propan,
25 bis 60 Vol.-% Stickstoff,
1 bis 5 Vol.-% Summe aus CO, $CO_2$ und $H_2O$ und
0 bis 5 Vol.-% sonstige Bestandteile,

wobei gegebenenfalls enthaltener Ammoniak unberücksichtigt ist.

**47.** Verfahren nach einem der Ansprüche 33 bis 43, **dadurch gekennzeichnet, daß** das Gasgemisch 2 die nachfolgenden Gehalte aufweist,

$H_2O$ ≤ 60 Vol.-%,
$N_2$ ≤ 80 Vol.-%,
$O_{22}$ > 0, ≤ 20 Vol.-%,
CO ≤ 2 Vol.-%,
$CO_2$ ≤ 5 Vol.-%,
Ethan ≤ 10 Vol.-%,
Ethylen ≤ 5 Vol.-%,
Methan ≤ 5 Vol.-%,
Propan > 0, ≤ 50 Vol.-%,
Cyclopropan ≤ 0,1 Vol.-%,
Propin ≤ 0,1 Vol.-%,
Propadien ≤ 0,1 Vol.-%,
Propylen > 0, ≤ 30 Vol.-%,
$H_2$ ≤ 30 Vol.-%,
iso-Butan ≤ 3 Vol.-%,
n-Butan ≤ 3 Vol.-%,
trans-Buten-2 ≤ 1 Vol.-%,
cis-Buten-2 ≤ 1 Vol.-%,
Buten-1 ≤ 1 Vol.-%,

iso-Buten ≤ 1 Vol.-%,
Butadien 1,3 ≤ 1 Vol.-%,
Butadien 1,2 ≤ 1 Vol.-%,
1-Butin ≤ 0,5 Vol.-% und
2-Butin ≤ 0,5 Vol.-%,

wobei gegebenenfalls enthaltener Ammoniak unberücksichtigt ist.

**48.** Verfahren nach einem der Ansprüche 33 bis 47, **dadurch gekennzeichnet, daß** das Roh-Propan ≥ 0,25 Vol.-% an von Propan und Propylen verschiedenen Bestandteilen enthält.

**49.** Verfahren nach einem der Ansprüche 33 bis 48, **dadurch gekennzeichnet, daß** das Roh-Propan bis zu 6 Vol.-% an $C_4$-Kohlenwasserstoffen enthält.

**50.** Verfahren nach einem der Ansprüche 33 bis 48, **dadurch gekennzeichnet, daß** das Roh-Propan 0,1 bis 6 Vol.-% an $C_4$-Kohlenwasserstoffen enthält.

**51.** Verfahren nach einem der Ansprüche 33 bis 50, **dadurch gekennzeichnet, daß** das Roh-Propan bis zu 0,5 Vol.-% Buten-1 enthält.

**52.** Verfahren nach einem der Ansprüche 33 bis 50, **dadurch gekennzeichnet, daß** das Roh-Propan 5 Vol.ppm bis 0,5 Vol.-% Buten-1 enthält.

**53.** Verfahren nach einem der Ansprüche 33 bis 52, **dadurch gekennzeichnet, daß** das Roh-Propan insgesamt bis zu 0,5 Vol.-% an Butenen enthält.

**54.** Verfahren nach einem der Ansprüche 33 bis 52, **dadurch gekennzeichnet, daß** das Roh-Propan insgesamt 5 Vol.ppm bis zu 0,5 Vol.-% an Butenen enthält.

**55.** Verfahren nach einem der Ansprüche 33 bis 54, **dadurch gekennzeichnet, daß** das Roh-Propan die folgende Spezifikation erfüllt:

Gehalt an Propan ≥ 90 Vol.-%,
Gesamtgehalt aus Propan und Propylen ≤ 99 Vol.-%,
Gesamtgehalt an $C_4$-Kohlenwasserstoffen ≤ 6 Vol.-%,
Gehalt an Buten-1 ≤ 0,5 Vol.-%,
Gesamtgehalt an Butenen ≤ 0,5 Vol.-%,
Gehalt an Ethan ≤ 10 Vol.-%,
Gehalt an Ethylen ≤ 5 Vol.-%,
Gehalt an Methan ≤ 5 Vol.-%,
Gehalt an Cyclopropan ≤ 0,1 Vol.-%,
Gehalt an Propylen ≤ 10 Vol.-%,
Gesamtgehalt an von Propan und Propylen verschiedenen $C_3$-Kohlenwasserstoffen ≤ 0,3 Vol.-%,
Gesamtgehalt an $C_5$-Kohlenwasserstoffen ≤ 0,3 Vol.-%,
und Gesamtgehalt an $C_6$-bis $C_8$-Kohlenwasserstoffen
≤ 600 Vol.ppm.

**56.** Verfahren nach einem der Ansprüche 33 bis 54, **dadurch gekennzeichnet, daß** der Propanumsatz im ersten Schritt ≥ 5 mol.-% bis ≤ 30 mol.-% beträgt.

**57.** Verfahren nach einem der Ansprüche 33 bis 56, **dadurch gekennzeichnet, dass** man aus dem Produktgasgemisch der Gasphasen- Partialoxidation und/oder partiellen Gasphasen-Ammoxidation von dem wenigstens einen partiellen Oxidations- und/oder Ammoxidationsprodukt des Propylens abtrennt und wenigstens in diesem Produktgasgemisch enthaltenes nicht umgesetztes Propan in den ersten Schritt und/oder in die Gasphasen-Partialoxidation und/oder partielle Gasphasen-Ammoxidation rückführt.

**58.** Verfahren nach Anspruch 33, **dadurch gekennzeichnet, daß** der erste Schritt eine heterogen katalysierte Dehydrierung ist.

**59.** Verfahren nach Anspruch 33 oder 58, **dadurch gekennzeichnet, daß** aus dem Gasgemisch 1 von den darin enthaltenen, von Propan und Propylen verschiedenen, Bestandteilen eine Teilmenge abgetrennt wird, die wenigstens einen $C_4$-Kohlenwasserstoff umfaßt.

**60.** Verfahren nach einem der Ansprüche 33 , 58 oder 59, **dadurch gekennzeichnet, daß** aus dem Gasgemisch 1 von den darin enthaltenen, von Propan und Propylen verschiedenen, Bestandteilen eine Teilmenge abgetrennt wird, die wenigstens ein Buten umfaßt.

**61.** Verfahren nach einem der Ansprüche 33 oder 58 bis 60, **dadurch gekennzeichnet, daß** aus dem Gasgemisch 1 von den darin enthaltenen, von Propan und Propylen verschiedenen, Bestandteilen eine Teilmenge abgetrennt wird, die wenigstens Buten-1 umfaßt.

**62.** Verfahren nach einem der Ansprüche 33 oder 58 bis 61, **dadurch gekennzeichnet, daß** in der heterogen katalysierten Gasphasen-Partialoxidation und/oder partiellen Gasphasen-Ammoxidation ein Katalysator mitverwendet wird, dessen Aktivmasse die Elemente Mo, Bi und Fe enthält.

**63.** Verfahren nach einem der Ansprüche 33 oder 58 bis 62, **dadurch gekennzeichnet, daß** in der heterogen katalysierten Gasphasen-Partialoxidation und/oder partiellen Gasphasen-Ammoxidation ein Katalysator mitverwendet wird, dessen Aktivmasse ein Multimetalloxid der allgemeinen Formel IV

$$Mo_{12}Bi_aFe_bX^1_cX^2_dX^3_eX^4_fO_n \qquad (IV),$$

in der die Variablen nachfolgende Bedeutung aufweisen:

$X^1$= Nickel und/oder Kobalt,
$X^2$= Thallium, ein Alkalimetall und/oder ein Erdalkalimetall,
$X^3$= Zink, Phosphor, Arsen, Bor, Antimon, Zinn, Cer, Blei und/oder Wolfram,
$X^4$= Silicium, Aluminium, Titan und/oder Zirkonium,
a= 0 bis 5,
b= 0,01 bis 5,
C= 0 bis 10,
d= 0 bis 2,
e= 0 bis 8,
f= 0 bis 10 und
n= eine Zahl, die durch die Wertigkeit und Häufigkeit der von Sauerstoff verschiedenen Elemente in (IV) bestimmt wird,

ist.

**64.** Verfahren nach einem der Ansprüche 33 oder 58 bis 63, **dadurch gekennzeichnet, daß** in der heterogen katalysierten Gasphasen-Partialoxidation ein Katalysator mitverwendet wird, dessen Aktivmasse ein Multimetalloxid der allgemeinen Formel VII

$$Mo_{12}V_aX^1_bX^2_cX^3_dX^4_eX^5_fX^6_gO_n \qquad (VII),$$

in der die Variablen folgende Bedeutung haben:

$X^1$= W, Nb, Ta, Cr und/oder Ce
$X^2$= Cu, Ni, Co, Fe, Mn und/oder Zn,
$X^3$= Sb und/oder Bi,
$X^4$= eines oder mehrere Alkalimetalle,
$X^5$= eines oder mehrere Erdalkalimetalle,
$X^6$= Si, Al, Ti und/oder Zr,

a= 1 bis 6
b= 0,2 bis 4,
c= 0,5 bis 18,
d= 0 bis 40,
e= 0 bis 2,
f= 0 bis 4,
g= 0 bis 40 und
n= eine Zahl, die durch die Wertigkeit und Häufigkeit der von Sauerstoff verschiedenen Elemente in VII bestimmt wird,

ist.

**65.** Verfahren nach einem der Ansprüche 33 bis 64, **dadurch gekennzeichnet, daß** das wenigstens eine partielle Oxidationsund/oder Ammoxidationsprodukt des Propylens wenigstens eine Verbindung aus der Gruppe umfassend Propylenoxid, Acrolein, Acrylsäure und Acrylnitril ist.

**66.** Verfahren nach einem der Ansprüche 33 bis 65, **dadurch gekennzeichnet, dass** man in einem ersten Schritt Roh-Propan im Beisein und/oder unter Ausschluß von Sauerstoff einer heterogen katalysierten Dehydrierung unterwirft und Gasgemisch 1 einer heterogen katalysierten Gasphasen-Ammoxidation von im Gasgemisch 1 enthaltenem Propylen unterwirft.

**67.** Verfahren nach einem der Ansprüche 33 bis 66, **dadurch gekennzeichnet, dass** man in einem ersten Schritt Roh-Propan im Beisein und/oder unter Ausschluß von Sauerstoff sowie im Beisein von Wasserdampf einer heterogen katalysierten Dehydrierung unterwirft und aus im ersten Schritt gebildetem Gasgemisch 1 Wasserdampf durch Kondensieren teilweise oder vollständig abtrennt und das dabei erzeugte Gasgemisch 1' einer heterogen katalysierten Gasphasen-Partialoxidation und/oder partiellen Gasphasen-Ammoxidation von im Gasgemisch 1' enthaltenem Propylen unterwirft.

**68.** Verfahren nach einem der Ansprüche 33 bis 67, **dadurch gekennzeichnet, dass** man in einem ersten Schritt Roh-Propan einer autothermen heterogen katalysierten Dehydrierung unterwirft.

**69.** Verfahren nach einem der Ansprüche 33 bis 68, **dadurch gekennzeichnet, dass** man das Gasgemisch 1 und/oder Gasgemisch 1' als Bestandteil eines Gasgemisches 2 einer heterogen katalysierten partiellen Gasphasen-Ammoxidation von im Gasgemisch 1 und/oder Gasgemisch 1' enthaltenem Propylen unterwirft.

## Claims

**1.** A process for preparing at least one partial oxidation and/or ammoxidation product of propylene, in which

a) crude propane is subjected to a homogeneously and/or heterogeneously catalyzed dehydrogenation and/or oxydehydrogenation in the presence and/or absence of oxygen in a first step to give a gas mixture 1 comprising propane and propylene,
and
b) part of the constituents other than propane and propylene present in the gas mixture 1 formed in the first step is optionally separated off from the gas mixture 1 and/or converted into other compounds so as to produce a gas mixture 1' comprising propane and propylene and also compounds other than oxygen, propane and propylene from the gas mixture 1 and, in at least one further step,
c) gas mixture 1 and/or gas mixture 1' are/is subjected as constituent of a gas mixture 2 to a heterogeneously catalyzed gas-phase partial oxidation and/or partial gas-phase ammoxidation of propylene present in gas mixture 1 and/or gas mixture 1',

wherein the 1-butene content of the gas mixture 2 is $\leq$ 1% by volume, where the process of the present invention is carried out in a reaction zone over a catalyst charge whose active composition consists of at least one multimetal oxide composition comprising the elements Mo, V, at least one of the two elements Te and Sb and at least one element selected from the group consisting of Nb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au and In in combination.

2. The process according to claim 1, wherein the 1-butene content of the gas mixture 2 is > 0.003% by volume.

3. The process according to claim 1, wherein the 1-butene content of the gas mixture 2 is > 0.001% by volume.

4. The process according to any of claims 1 to 3, wherein the trans-2-butene content of the gas mixture 2 is ≤ 0.05% by volume.

5. The process according to any of claims 1 to 4, wherein the cis-2-butene content of the gas mixture 2 is ≤ 0.05% by volume.

6. The process according to any of claims 1 to 5, wherein the isobutene content of the gas mixture 2 is ≤ 0.05% by volume.

7. The process according to any of claims 1 to 6, wherein the total content of butenes in the gas mixture 2 is ≤ 1% by volume.

8. The process according to any of claims 1 to 6, wherein the total content of butenes in the gas mixture 2 is ≤ 0.05% by volume.

9. The process according to any of claims 1 to 8, wherein the total content of $C_4$-hydrocarbons in the gas mixture 2 is ≤ 3% by volume.

10. The process according to any of claims 1 to 9, wherein the total content of $C_4$-hydrocarbons in the gas mixture 2 is > 0.05 and < 3% by volume.

11. The process according to any of claims 1 to 10, wherein the gas mixture 1' contains ≥ 0.1% by volume of constituents other than propane and propylene and oxygen.

12. The process according to any of claims 1 to 11, wherein the gas mixture 2 contains up to 60% by volume of propane.

13. The process according to any of claims 1 to 11, wherein the gas mixture 2 contains from 20 to 40% by volume of propane.

14. The process according to any of claims 1 to 11, wherein the gas mixture 2 comprises

from 7 to 15% by volume of $O_2$,
from 5 to 10% by volume of propylene,
from 15 to 40% by volume of propane,
from 25 to 60% by volume of nitrogen,
from 1 to 5% by volume of the total of CO, $CO_2$ and $H_2O$ and
from 0 to 5% by volume of other constituents,

with any ammonia present being disregarded.

15. The process according to any of claims 1 to 11, wherein the gas mixture 2 comprises

$H_2O$ ≤ 60% by volume,
$N_2$ ≤ 80% by volume,
$O_{22}$ > 0, ≤ 20% by volume,
CO ≤ 2% by volume,
$CO_2$ ≤ 5% by volume,
ethane ≤ 10% by volume,
ethylene ≤ 5% by volume,
methane ≤ 5% by volume,
propane > 0, ≤ 50% by volume,
cyclopropane ≤ 0.1% by volume,
propane ≤ 0.1% by volume,
propadiene ≤ 0.1% by volume,
propylene > 0, ≤ 30% by volume,

$H_2$ ≤ 30% by volume,
isobutane ≤ 3% by volume,
n-butane ≤ 3% by volume,
trans-2-butene ≤ 1% by volume,
cis-2-butene ≤ 1% by volume,
1-butene ≤ 1% by volume,
isobutene ≤ 1% by volume,
1,3-butadiene ≤ 1% by volume,
1,2-butadiene ≤ 1% by volume,
1-butyne ≤ 0.5% by volume and
2-butyne ≤ 0.5% by volume,

with any ammonia present being disregarded.

**16.** The process according to any of claims 1 to 15, wherein the crude propane contains ≥ 0.25% by volume of constituents other than propane and propylene.

**17.** The process according to any of claims 1 to 16, wherein the crude propane contains up to 6% by volume of $C_4$-hydrocarbons.

**18.** The process according to any of claims 1 to 16, wherein the crude propane contains from 0.1 to 6% by volume of $C_4$-hydrocarbons.

**19.** The process according to any of claims 1 to 18, wherein the crude propane contains up to 0.5% by volume of 1-butene.

**20.** The process according to any of claims 1 to 18, wherein the crude propane contains from 5 ppm by volume to 0.5% by volume of 1-butene.

**21.** The process according to any of claims 1 to 20, wherein the crude propane contains a total of up to 0.5% by volume of butenes.

**22.** The process according to any of claims 1 to 20, wherein the crude propane contains a total of from 5 ppm by volume to 0.5% by volume of butenes.

**23.** The process according to any of claims 1 to 22, wherein the crude propane meets the following specification:

propane content ≥ 90% by volume,
total content of propane and propylene ≤ 99% by volume,
total content of $C_4$-hydrocarbons ≤ 6% by volume,
1-butene content ≤ 0.5% by volume,
total content of butenes ≤ 0.5% by volume,
ethane content ≤ 10% by volume,
ethylene content ≤ 5% by volume,
methane content ≤ 5% by volume,
cyclopropane content ≤ 0.1% by volume,
propylene content ≤ 10% by volume,
total content of $C_3$-hydrocarbons other than propane and propylene ≤ 0.3% by volume,
total content of $C_5$-hydrocarbons ≤ 0.3% by volume, and total content of $C_6$-$C_8$-hydrocarbons ≤ 600 ppm by volume.

**24.** The process according to any of claims 1 to 23, wherein the propane conversion in the first step is from ≥ 5 mol% to ≤ 30 mol%.

**25.** The process according to any of claims 1 to 24, wherein of the at least one partial oxidation and/or ammoxidation product or products of propylene is separated off from the product gas mixture from the gas-phase partial oxidation and/or partial gas-phase ammoxidation and at least the unreacted propane present in this product gas mixture is recirculated to the first step and/or to the gas-phase partial oxidation and/or partial gas-phase ammoxidation.

**26.** The process according to claim 1, wherein the active composition consists of at least one multimetal oxide composition which has the element stoichiometry I

$$Mo_1V_bM^1_cM^2_d \qquad (I),$$

where

$M^1$ = Te and/or Sb,
$M^2$ = at least one element from the group consisting of Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au and In,
b = 0.01 to 1,
c = > 0 to 1 and
d = > 0 to 1.

**27.** The process according to either of claims 1 and 26, wherein the X-ray diffraction pattern of the active multimetal oxide composition or compositions displays reflections h and i whose maxima are at the diffraction angles 22.2 ±0.5° (h) and 27.3 ±0.5° (i).

**28.** The process according to any of claims 1 to 27, wherein the partial oxidation and/or ammoxidation product or products of propylene comprise(s) at least one compound from the group consisting of propylene oxide, acrolein, acrylic acid and acrylonitrile.

**29.** The process according to any of claims 1 to 28, wherein a first step comprises subjecting crude propane to a heterogeneously catalyzed dehydrogenation in the presence and/or absence of oxygen and subjecting gas mixture 1 to a heterogeneously catalyzed gas phase ammoxidation of propylene present in the gas mixture 1.

**30.** The process according to any of claims 1 to 29, wherein a first step comprises subjecting crude propane to a heterogeneously catalyzed dehydrogenation in the presence and/or absence of oxygen and also in the presence of water vapor and condensing all or some of the water vapor out of the gas mixture 1 formed in the first step to obtain a gas mixture 1' which is subjected to a heterogeneously catalyzed gas phase partial oxidation and/or partial gas phase ammoxidation of propylene present in the gas mixture 1'.

**31.** The process according to any of claims 1 to 30, wherein a first step comprises subjecting crude propane to a heterogeneously catalyzed dehydrogenation which is autothermal.

**32.** The process according to any of claims 1 to 31, wherein the gas mixture 1 and/or gas mixture 1' are subjected as a constituent of a gas mixture 2 to a heterogeneously catalyzed partial gas phase ammoxidation of propylene present in the gas mixture 1 and/or gas mixture 1'.

**33.** A process for preparing at least one partial oxidation and/or ammoxidation product of propylene, in which

a) crude propane is subjected to a homogeneously and/or heterogeneously catalyzed dehydrogenation and/or oxydehydrogenation in the presence and/or absence of oxygen in a first step to give a gas mixture 1 comprising propane and propylene,
and
b) part of the constituents other than propane and propylene present in the gas mixture 1 formed in the first step is optionally separated off from the gas mixture 1 and/or converted into other compounds so as to produce a gas mixture 1' comprising propane and propylene and also compounds other than oxygen, propane and propylene from the gas mixture 1 and, in at least one further step,
c) gas mixture 1 and/or gas mixture 1' are/is subjected as constituent of a gas mixture 2 to a heterogeneously catalyzed gas-phase partial oxidation and/or partial gas-phase ammoxidation of propylene present in gas mixture 1 and/or gas mixture 1',

wherein the 1-butene content of the gas mixture 2 is ≤ 1% by volume, where the first step is carried out in a separate reaction zone and, in the heterogeneously catalyzed gas-phase partial oxidation, a catalyst whose active composition contains the elements Mo and V is co-used.

**34.** The process according to claim 33, wherein the 1-butene content of the gas mixture 2 is > 0.003% by volume.

**35.** The process according to claim 33, wherein the 1-butene content of the gas mixture 2 is > 0.001% by volume.

**36.** The process according to any of claims 33 to 35, wherein the trans-2-butene content of the gas mixture 2 is ≤ 0.05% by volume.

**37.** The process according to any of claims 33 to 36, wherein the cis-2-butene content of the gas mixture 2 is ≤ 0.05% by volume.

**38.** The process according to any of claims 33 to 37, wherein the isobutene content of the gas mixture 2 is ≤ 0.05% by volume.

**39.** The process according to any of claims 33 to 38, wherein the total content of butenes in the gas mixture 2 is ≤ 1% by volume.

**40.** The process according to any of claims 33 to 38, wherein the total content of butenes in the gas mixture 2 is ≤ 0.05% by volume.

**41.** The process according to any of claims 33 to 40, wherein the total content of $C_4$-hydrocarbons in the gas mixture 2 is ≤ 3% by volume.

**42.** The process according to any of claims 33 to 40, wherein the total content of $C_4$-hydrocarbons in the gas mixture 2 is >< 0.05 and < 3% by volume.

**43.** The process according to any of claims 33 to 42, wherein the gas mixture 1' contains ≥ 0.1% by volume of constituents other than propane and propylene and oxygen.

**44.** The process according to any of claims 33 to 43, wherein the gas mixture 2 contains up to 60% by volume of propane.

**45.** The process according to any of claims 33 to 43, wherein the gas mixture 2 contains from 20 to 40% by volume of propane.

**46.** The process according to any of claims 33 to 43, wherein the gas mixture 2 comprises

from 7 to 15% by volume of $O_2$,
from 5 to 10% by volume of propylene,
from 15 to 40% by volume of propane,
from 25 to 60% by volume of nitrogen,
from 1 to 5% by volume of the total of CO, $CO_2$ and $H_2O$ and
from 0 to 5% by volume of other constituents,

with any ammonia present being disregarded.

**47.** The process according to any of claims 33 to 43, wherein the gas mixture 2 comprises

$H_2O$ ≤ 60% by volume,
$N_2$ ≤ 80% by volume,
$O_{22}$ > 0, ≤ 20% by volume,
CO ≤ 2% by volume,
$CO_2$ ≤ 5% by volume,
ethane ≤ 10% by volume,
ethylene ≤ 5% by volume,
methane ≤ 5% by volume,
propane > 0, ≤ 50% by volume,
cyclopropane ≤ 0.1% by volume,
propane ≤ 0.1% by volume,
propadiene ≤ 0.1% by volume,

propylene > 0, ≤ 30% by volume,
$H_2$ ≤ 30% by volume,
isobutane ≤ 3% by volume,
n-butane ≤ 3% by volume,
trans-2-butene ≤ 1% by volume,
cis-2-butene ≤ 1% by volume,
1-butene ≤ 1% by volume,
isobutene ≤ 1% by volume,
1,3-butadiene ≤ 1% by volume,
1,2-butadiene ≤ 1% by volume,
1-butyne ≤ 0.5% by volume and
2-butyne ≤ 0.5% by volume,

with any ammonia present being disregarded.

**48.** The process according to any of claims 33 to 47, wherein the crude propane contains ≥ 0.25% by volume of constituents other than propane and propylene.

**49.** The process according to any of claims 33 to 48, wherein the crude propane contains up to 6% by volume of $C_4$-hydrocarbons.

**50.** The process according to any of claims 33 to 48, wherein the crude propane contains from 0.1 to 6% by volume of $C_4$-hydrocarbons.

**51.** The process according to any of claims 33 to 50, wherein the crude propane contains up to 0.5% by volume of 1-butene.

**52.** The process according to any of claims 33 to 50, wherein the crude propane contains from 5 ppm by volume to 0.5% by volume of 1-butene.

**53.** The process according to any of claims 33 to 52, wherein the crude propane contains a total of up to 0.5% by volume of butenes.

**54.** The process according to any of claims 33 to 52, wherein the crude propane contains a total of from 5 ppm by volume to 0.5% by volume of butenes.

**55.** The process according to any of claims 33 to 54, wherein the crude propane meets the following specification:

propane content ≥ 90% by volume,
total content of propane and propylene ≤ 99% by volume,
total content of $C_4$-hydrocarbons ≤ 6% by volume,
1-butene content ≤ 0.5% by volume,
total content of butenes ≤ 0.5% by volume,
ethane content ≤ 10% by volume,
ethylene content ≤ 5% by volume,
methane content ≤ 5% by volume,
cyclopropane content ≤ 0.1% by volume,
propylene content ≤ 10% by volume,
total content of $C_3$-hydrocarbons other than propane and propylene ≤ 0.3% by volume,
total content of $C_5$-hydrocarbons ≤ 0.3% by volume, and total content of $C_6$-$C_8$-hydrocarbons ≤ 600 ppm by volume.

**56.** The process according to any of claims 33 to 54, wherein the propane conversion in the first step is from ≥ 5 mol% to ≤ 30 mol%.

**57.** The process according to any of claims 33 to 56, wherein of the at least one partial oxidation and/or ammoxidation product or products of propylene is separated off from the product gas mixture from the gas-phase partial oxidation and/or partial gas-phase ammoxidation and at least the unreacted propane present in this product gas mixture is

recirculated to the first step and/or to the gas-phase partial oxidation and/or partial gas-phase ammoxidation.

**58.** The process according to claim 33, wherein the first step is a heterogeneously catalyzed dehydrogenation.

**59.** The process according to claim 33 or 58, wherein a part comprising at least one $C_4$-hydrocarbon of the constituents other than propane and propylene present in the gas mixture 1 is separated off from the gas mixture 1.

**60.** The process according to any of claims 33, 58 and 59, wherein a part comprising at least one butene of the constituents other than propane and propylene present in the gas mixture 1 is separated off from the gas mixture 1.

**61.** The process according to any of claims 33 and 58 to 60, wherein a part comprising at least 1-butene of the constituents other than propane and propylene present in the gas mixture 1 is separated off from the gas mixture 1.

**62.** The process according to any of claims 33 and 58 to 61, wherein the heterogeneously catalyzed gas-phase partial oxidation and/or partial gas-phase ammoxidation is carried out using a catalyst whose active composition comprises the elements Mo, Bi and Fe.

**63.** The process according to any of claims 33 and 58 to 62, wherein the heterogeneously catalyzed gas-phase partial oxidation and/or partial gas-phase ammoxidation is carried out using a catalyst whose active composition is a multimetal oxide of the formula IV

$$Mo_{12}Bi_aFe_bX^1_cX^2_dX^3_eX^4_fO_n \quad (IV),$$

where the variables have the following meanings:

$X^1$=nickel and/or cobalt,
$X^2$=thallium, an alkali metal or an alkaline earth metal,
$X^3$=zinc, phosphorus, arsenic, boron, antimony, tin, cerium, lead and/or tungsten,
$X^4$=silicon, aluminum, titanium and/or zirconium,
a= 0 to 5,
b= 0.01 to 5,
c= 0 to 10,
d= 0 to 2,
e= 0 to 8,
f= 0 to 10 and
n= a number determined by the valence and abundance of the elements other than oxygen in (IV).

**64.** The process according to any of claims 33 and 58 to 63, wherein the heterogeneously catalyzed gas-phase partial oxidation is carried out using a catalyst whose active composition is a multimetal oxide of the formula VII

$$Mo_{12}V_aX^1_bX^2_cX^3_dX^4_eX^5_fX^6_gO_n \quad (VII),$$

where the variables have the following meanings:

$X^1$=W, Nb, Ta, Cr and/or Ce,
$X^2$=Cu, Ni, Co, Fe, Mn and/or Zn,
$X^3$=Sb and/or Bi,
$X^4$=one or more alkali metals,
$X^5$=one or more alkaline earth metals,
$X^6$=Si, Al, Ti and/or Zr,
a= 1 to 6,
b= 0.2 to 4,
c= 0.5 to 18,

d= 0 to 40,
e= 0 to 2,
f= 0 to 4,
g= 0 to 40 and
n= a number determined by the valence and abundance of the elements other than oxygen in VII.

**65.** The process according to any of claims 33 to 64, wherein the partial oxidation and/or ammoxidation product or products of propylene comprise(s) at least one compound from the group consisting of propylene oxide, acrolein, acrylic acid and acrylonitrile.

**66.** The process according to any of claims 33 to 65, wherein a first step comprises subjecting crude propane to a heterogeneously catalyzed dehydrogenation in the presence and/or absence of oxygen and subjecting gas mixture 1 to a heterogeneously catalyzed gas phase ammoxidation of propylene present in the gas mixture 1.

**67.** The process according to any of claims 33 to 66, wherein a first step comprises subjecting crude propane to a heterogeneously catalyzed dehydrogenation in the presence and/or absence of oxygen and also in the presence of water vapor and condensing all or some of the water vapor out of the gas mixture 1 formed in the first step to obtain a gas mixture 1' which is subjected to a heterogeneously catalyzed gas phase partial oxidation and/or partial gas phase ammoxidation of propylene present in the gas mixture 1'.

**68.** The process according to any of claims 33 to 67, wherein a first step comprises subjecting crude propane to a heterogeneously catalyzed dehydrogenation which is autothermal.

**69.** The process according to any of claims 33 to 68, wherein the gas mixture 1 and/or gas mixture 1' are subjected as a constituent of a gas mixture 2 to a heterogeneously catalyzed partial gas phase ammoxidation of propylene present in the gas mixture 1 and/or gas mixture 1'.

**Revendications**

**1.** Procédé pour la préparation d'au moins un produit d'oxydation et/ou d'ammoxydation partielle du propylène, dans lequel

a) dans une première étape, on soumet du propane brut à une déshydrogénation et/ou à une oxydéshydrogénation catalysée(s) par voie homogène et/ou hétérogène en présence d'oxygène et/ou en excluant l'oxygène, un mélange gazeux 1 contenant du propane et du propylène étant obtenu, et
b) on sépare, à partir du mélange gazeux 1 formé dans la première étape, le cas échéant une partie des constituants différents du propane et du propylène contenus dans celui-ci et/ou on les transforme en d'autres composés, un mélange gazeux 1', contenant du propane et du propylène ainsi qu'également des composés différents de l'oxygène, du propane et du propylène, étant à chaque fois formé à partir du mélange gazeux 1, et, dans au moins une autre étape
c) on soumet le mélange gazeux 1 et/ou le mélange gazeux 1', comme constituant d'un mélange gazeux 2, à une oxydation partielle en phase gazeuse et/ou à une ammoxydation partielle en phase gazeuse, catalysée(s) par voie hétérogène du propylène contenu dans le mélange gazeux 1 et/ou le mélange gazeux 1',

**caractérisé en ce que** la teneur du mélange gazeux 2 en butène-1 est ≤ 1% en volume, le procédé selon l'invention étant réalisé dans une zone de réaction sur une charge catalytique dont la masse active est au moins une masse d'oxyde multimétallique qui contient les éléments Mo, V, au moins un des deux éléments Te et Sb et au moins un des éléments du groupe comprenant Nb, Ta, W, Ti, Al, Zr, Cr, Mn, Ga, Fe, Ru, Co, Rh, Ni, Pd, Pt, La, Bi, B, Ce, Sn, Zn, Si, Na, Li, K, Mg, Ag, Au et In en combinaison.

**2.** Procédé selon la revendication 1, **caractérisé en ce que** la teneur du mélange gazeux 2 en butène-1 est > 0,003% en volume.

**3.** Procédé selon la revendication 1, **caractérisé en ce que** la teneur du mélange gazeux 2 en butène-1 est > 0,001% en volume.

**4.** Procédé selon l'une quelconque des revendications 1 à 3, **caractérisé en ce que** la teneur du mélange gazeux 2

en trans-butène-2 est ≤ 0,05% en volume.

5. Procédé selon l'une quelconque des revendications 1 à 4, **caractérisé en ce que** la teneur du mélange gazeux 2 en cis-butène-2 est ≤ 0,05% en volume.

6. Procédé selon l'une quelconque des revendications 1 à 5, **caractérisé en ce que** la teneur du mélange gazeux 2 en isobutène est ≤ 0,05% en volume.

7. Procédé selon l'une quelconque des revendications 1 à 6, **caractérisé en ce que** la teneur totale du mélange gazeux 2 en butènes est ≤ 1% en volume.

8. Procédé selon l'une quelconque des revendications 1 à 6, **caractérisé en ce que** la teneur totale du mélange gazeux 2 en butènes est ≤ 0,05% en volume.

9. Procédé selon l'une quelconque des revendications 1 à 8, **caractérisé en ce que** la teneur totale du mélange gazeux 2 en hydrocarbures en $C_4$ est ≤ 3% en volume.

10. Procédé selon l'une quelconque des revendications 1 à 9, **caractérisé en ce que** la teneur totale du mélange gazeux 2 en hydrocarbures en $C_4$ est > 0,05 et < 3% en volume.

11. Procédé selon l'une quelconque des revendications 1 à 10, **caractérisé en ce que** le mélange gazeux 1' contient ≥ 0,1% en volume de constituants différents du propane et du propylène ainsi que de l'oxygène.

12. Procédé selon l'une quelconque des revendications 1 à 11, **caractérisé en ce que** le mélange gazeux 2 contient jusqu'à 60% en volume de propane.

13. Procédé selon l'une quelconque des revendications 1 à 11, **caractérisé en ce que** le mélange gazeux 2 contient 20 à 40% en volume de propane.

14. Procédé selon l'une quelconque des revendications 1 à 11, **caractérisé en ce que** le mélange gazeux 2 présente les teneurs suivantes
7 à 15% en volume d'$O_2$,
5 à 10% en volume de propylène,
15 à 40% en volume de propane,
25 à 60% en volume d'azote,
1 à 5% en volume de la somme de CO, $CO_2$ et $H_2O$ et
0 à 5% en volume d'autres constituants,
l'ammoniaque le cas échéant contenu n'étant pas pris en considération.

15. Procédé selon l'une quelconque des revendications 1 à 11, **caractérisé en ce que** le mélange gazeux 2 présente les teneurs suivantes
$H_2O$ ≤ 60% en volume,
$N_2$ ≤ 80% en volume,
$O_{22}$ > 0, ≤ 20% en volume,
CO ≤ 2% en volume,
$CO_2$ ≤ 5% en volume,
éthane ≤ 10% en volume,
éthylène ≤ 5% en volume,
méthane ≤ 5% en volume,
propane > 0, ≤ 50% en volume,
cyclopropane ≤ 0,1% en volume,
propyne ≤ 0,1% en volume,
propadiène ≤ 0,1% en volume,
propylène > 0, ≤ 30% en volume,
$H_2$ ≤ 30% en volume,
isobutane ≤ 3% en volume,
n-butane ≤ 3% en volume,
trans-butène-2 ≤ 1% en volume,

cis-butène-2 ≤ 1% en volume,
butène-1 ≤ 1% en volume,
isobutène ≤ 1% en volume,
butadiène-1,3 ≤ 1% en volume,
butadiène-1,2 ≤ 1% en volume,
1-butyne ≤ 0,5% en volume et
2-butyne ≤ 0,5% en volume,
l'ammoniaque le cas échéant contenu n'étant pas pris en considération.

**16.** Procédé selon l'une quelconque des revendications 1 à 15, **caractérisé en ce que** le propane brut continent ≥ 0,25% en volume de constituants différents du propane et du propylène.

**17.** Procédé selon l'une quelconque des revendications 1 à 16, **caractérisé en ce que** le propane brut contient jusqu'à 6% en volume d'hydrocarbures en $C_4$.

**18.** Procédé selon l'une quelconque des revendications 1 à 16, **caractérisé en ce que** le propane brut contient 0,1 à 6% en volume d'hydrocarbures en $C_4$.

**19.** Procédé selon l'une quelconque des revendications 1 à 18, **caractérisé en ce que** le propane brut contient jusqu'à 0,5% en volume de butène-1.

**20.** Procédé selon l'une quelconque des revendications 1 à 18, **caractérisé en ce que** le propane brut contient 5 ppm en volume à 0,5% de butène-1.

**21.** Procédé selon l'une quelconque des revendications 1 à 20, **caractérisé en ce que** le propane brut contient au total jusqu'à 0,5% en volume de butènes.

**22.** Procédé selon l'une quelconque des revendications 1 à 20, **caractérisé en ce que** le propane brut contient au total 5 ppm en volume à 0,5% en volume de butènes.

**23.** Procédé selon l'une quelconque des revendications 1 à 22, **caractérisé en ce que** le propane brut répond à la spécification suivante :

teneur en propane ≥ 90% en volume,
teneur totale en propane et en propylène ≤ 99% en volume,
teneur totale en hydrocarbures en $C_4$ ≤ 6% en volume,
teneur en butène-1 ≤ 0,5% en volume,
teneur totale en butènes ≤ 0,5% en volume,
teneur en éthane ≤ 10% en volume,
teneur en éthylène ≤ 5% en volume,
teneur en méthane ≤ 5% en volume,
teneur en cyclopropane ≤ 0,1% en volume,
teneur en propylène ≤ 10% en volume,
teneur totale en hydrocarbures en $C_3$ différents du propane et du propylène ≤ 0,3% en volume,
teneur totale en hydrocarbures en $C_5$ ≤ 0,3% en volume,
et teneur totale en hydrocarbures en $C_6$ à $C_8$ ≤ 600 ppm en volume.

**24.** Procédé selon l'une quelconque des revendications 1 à 23, **caractérisé en ce que** la conversion du propane dans la première étape est ≥ 5% en mole, jusqu'à ≤ 30% en mole.

**25.** Procédé selon l'une quelconque des revendications 1 à 24, **caractérisé en ce qu'**on sépare, à partir du mélange gazeux de produits de l'oxydation partielle en phase gazeuse et/ou de l'ammoxydation partielle en phase gazeuse, du propylène dudit au moins un produit d'oxydation partielle et/ou d'ammoxydation partielle et on recycle au moins le propane non transformé contenu dans ce mélange gazeux de produits dans la première étape et/ou dans l'oxydation partielle en phase gazeuse et/ou dans l'ammoxydation partielle en phase gazeuse.

**26.** Procédé selon la revendication 1, **caractérisé en ce que** la masse active est au moins une masse d'oxyde multimétallique qui contient la stoéchiométrie élémentaire I

$$Mo_lV_bM_c^1M_d^2 \quad (I)$$

dans laquelle

$M^1$ = Te et/ou Sb,
$M^2$ = au moins un des éléments du groupe comprenant Nb, Ta, W, Ti, Al, Zr, Cr, Mn, Ga, Fe, Ru, Co, Rh, Ni, Pd, Pt, La, Bi, Ce, Sn, Zn, Si, Na, Li, K, Mg, Ag, Au et In,
b = 0, 01 à 1,
c = > 0 à 1 et
d = > 0 à 1.

**27.** Procédé selon l'une quelconque des revendications 1 ou 26, **caractérisé en ce que** le diffractogramme des rayons X de ladite au moins une masse active d'oxyde multimétallique présente un diffractogramme de rayons X qui présente des réflexions de diffraction h et i dont les positions des sommets se situent aux angles de diffraction 22,2 $\pm$ 0,5° (h) et 27, 3 $\pm$ 0, 5° (i).

**28.** Procédé selon l'une quelconque des revendications 1 à 27, **caractérisé en ce que** ledit au moins un produit d'oxydation et/ou d'ammoxydation partielle (s) du propylène est au moins un composé du groupe comprenant l'oxyde de propylène, l'acroléine, l'acide acrylique et l'acrylonitrile.

**29.** Procédé selon l'une quelconque des revendications 1 à 28, **caractérisé en ce que**, dans une première étape, on soumet du propane brut à une déshydrogénation catalysée par voie hétérogène en présence d'oxygène et/ou en excluant l'oxygène et on soumet le mélange gazeux 1 à une ammoxydation en phase gazeuse catalysée par voie hétérogène du propylène contenu dans le mélange gazeux 1.

**30.** Procédé selon l'une quelconque des revendications 1 à 29, **caractérisé en ce que**, dans une première étape, on soumet du propane brut à une déshydrogénation catalysée par voie hétérogène en présence d'oxygène et/ou en excluant l'oxygène ainsi qu'en présence de vapeur d'eau et on sépare partiellement ou complètement, à partir du mélange gazeux 1 formé dans la première étape, la vapeur d'eau par condensation et on soumet le mélange gazeux 1' ainsi obtenu à une oxydation partielle en phase gazeuse et/ou à une ammoxydation partielle en phase gazeuse, catalysée(s) par voie hétérogène, du propylène contenu dans le mélange gazeux 1'.

**31.** Procédé selon l'une quelconque des revendications 1 à 30, **caractérisé en ce qu'**on soumet, dans une première étape, du propane brut à une déshydrogénation autothermique catalysée par voie hétérogène.

**32.** Procédé selon l'une quelconque des revendications 1 à 31, **caractérisé en ce qu'**on soumet le mélange gazeux 1 et/ou le mélange gazeux 1', comme constituant d'un mélange gazeux 2, à une ammoxydation partielle en phase gazeuse, catalysée par voie hétérogène, du propylène contenu dans le mélange gazeux 1 et/ou dans le mélange gazeux 1'.

**33.** Procédé pour la préparation d'au moins un produit d'oxydation et/ou d'ammoxydation partielle du propylène, dans lequel

a) dans une première étape, on soumet du propane brut à une déshydrogénation et/ou à une oxydéshydrogénation catalysée(s) par voie homogène et/ou hétérogène en présence d'oxygène et/ou en excluant l'oxygène, un mélange gazeux 1 contenant du propane et du propylène étant obtenu, et
b) on sépare, à partir du mélange gazeux 1 formé dans la première étape, le cas échéant une partie des constituants différents du propane et du propylène contenus dans celui-ci et/ou on les transforme en d'autres composés, un mélange gazeux 1', contenant du propane et du propylène ainsi qu'également des composés différents de l'oxygène, du propane et du propylène, étant à chaque fois formé à partir du mélange gazeux 1, et, dans au moins une autre étape
c) on soumet le mélange gazeux 1 et/ou le mélange gazeux 1', comme constituant d'un mélange gazeux 2, à une oxydation partielle en phase gazeuse et/ou à une ammoxydation partielle en phase gazeuse, catalysée(s) par voie hétérogène du propylène contenu dans le mélange gazeux 1 et/ou dans le mélange gazeux 1',

**caractérisé en ce que** la teneur du mélange gazeux 2 en butène-1 est $\leq$ 1% en volume,
la première étape étant réalisée dans une zone de réaction séparée

et un catalyseur dont la masse active contient les éléments Mo et V étant utilisé conjointement dans l'oxydation partielle en phase gazeuse catalysée par voie hétérogène.

**34.** Procédé selon la revendication 33, **caractérisé en ce que** la teneur du mélange gazeux 2 en butène-1 est > 0,003% en volume.

**35.** Procédé selon la revendication 33, **caractérisé en ce que** la teneur du mélange gazeux 2 en butène-1 est > 0,001% en volume.

**36.** Procédé selon l'une quelconque des revendications 33 à 35, **caractérisé en ce que** la teneur du mélange gazeux 2 en trans-butène-2 est ≤ 0,05% en volume.

**37.** Procédé selon l'une quelconque des revendications 33 à 36, **caractérisé en ce que** la teneur du mélange gazeux 2 en cis-butène-2 est ≤ 0,05% en volume.

**38.** Procédé selon l'une quelconque des revendications 33 à 37, **caractérisé en ce que** la teneur du mélange gazeux 2 en isobutène est ≤ 0,05% en volume.

**39.** Procédé selon l'une quelconque des revendications 33 à 38, **caractérisé en ce que** la teneur totale du mélange gazeux 2 en butènes est ≤ 1% en volume.

**40.** Procédé selon l'une quelconque des revendications 33 à 38, **caractérisé en ce que** la teneur totale du mélange gazeux 2 en butènes est ≤ 0,05% en volume.

**41.** Procédé selon l'une quelconque des revendications 33 à 40, **caractérisé en ce que** la teneur totale du mélange gazeux 2 en hydrocarbures en $C_4$ est ≤ 3% en volume.

**42.** Procédé selon l'une quelconque des revendications 33 à 40, **caractérisé en ce que** la teneur totale du mélange gazeux 2 en hydrocarbures en $C_4$ est >< 0,05 et < 3% en volume.

**43.** Procédé selon l'une quelconque des revendications 33 à 42, **caractérisé en ce que** le mélange gazeux 1' contient ≥ 0,1% en volume de constituants différents du propane et du propylène ainsi que de l'oxygène.

**44.** Procédé selon l'une quelconque des revendications 33 à 43, **caractérisé en ce que** le mélange gazeux 2 contient jusqu'à 60% en volume de propane.

**45.** Procédé selon l'une quelconque des revendications 33 à 43, **caractérisé en ce que** le mélange gazeux 2 contient 20 à 40% en volume de propane.

**46.** Procédé selon l'une quelconque des revendications 33 à 43, **caractérisé en ce que** le mélange gazeux 2 présente les teneurs suivantes 7 à 15% en volume d'$O_2$,
5 à 10% en volume de propylène,
15 à 40% en volume de propane,
25 à 60% en volume d'azote,
1 à 5% en volume de la somme de CO, $CO_2$ et $H_2O$ et
0 à 5% en volume d'autres constituants,
l'ammoniaque le cas échéant contenu n'étant pas pris en considération.

**47.** Procédé selon l'une quelconque des revendications 33 à 43, **caractérisé en ce que** le mélange gazeux 2 présente les teneurs suivantes $H_2O$ ≤ 60% en volume,
$N_2$ ≤ 80% en volume,
$O_{22}$ > 0, ≤ 20% en volume,
CO ≤ 2% en volume,
$CO_2$ ≤ 5% en volume,
éthane ≤ 10% en volume,
éthylène ≤ 5% en volume,
méthane ≤ 5% en volume,
propane > 0, ≤ 50% en volume,

cyclopropane ≤ 0,1% en volume,
propyne ≤ 0,1% en volume,
propadiène ≤ 0,1% en volume,
propylène > 0, ≤ 30% en volume,
$H_2$ ≤ 30% en volume,
isobutene 3% en volume,
n-butane ≤ 3% en volume,
trans-butène-2 ≤ 1% en volume,
cis-butène-2 ≤ 1% en volume,
butène-1 ≤ 1% en volume,
isobutène ≤ 1% en volume,
butadiène 1,3 ≤ 1% en volume,
butadiène 1,2 ≤ 1% en volume,
1-butyne ≤ 0,5% en volume et
2-butyne ≤ 0,5% en volume,
l'ammoniaque le cas échéant contenu n'étant pas pris en considération.

**48.** Procédé selon l'une quelconque des revendications 33 à 47, **caractérisé en ce que** le propane brut contient ≥ 0,25% en volume de constituants différents du propane et du propylène.

**49.** Procédé selon l'une quelconque des revendications 33 à 48, **caractérisé en ce que** le propane brut contient jusqu'à 6% en volume d'hydrocarbures en $C_4$.

**50.** Procédé selon l'une quelconque des revendications 33 à 48, **caractérisé en ce que** le propane brut contient 0,1 à 6% en volume d'hydrocarbures en $C_4$.

**51.** Procédé selon l'une quelconque des revendications 33 à 50, **caractérisé en ce que** le propane brut contient jusqu'à 0,5% en volume de butène-1.

**52.** Procédé selon l'une quelconque des revendications 33 à 50, **caractérisé en ce que** le propane brut contient 5 ppm en volume à 0,5% de butène-1.

**53.** Procédé selon l'une quelconque des revendications 33 à 52, **caractérisé en ce que** le propane brut contient au total jusqu'à 0,5% en volume de butènes.

**54.** Procédé selon l'une quelconque des revendications 33 à 52, **caractérisé en ce que** le propane brut contient au total 5 ppm en volume à 0,5% en volume de butènes.

**55.** Procédé selon l'une quelconque des revendications 33 à 54, **caractérisé en ce que** le propane brut répond à la spécification suivante :

teneur en propane ≥ 90% en volume,
teneur totale en propane et en propylène ≤ 99% en volume,
teneur totale en hydrocarbures en $C_4$ ≤ 6% en volume,
teneur en butène-1 ≤ 0,5% en volume,
teneur totale en butènes ≤ 0,5% en volume,
teneur en éthane ≤ 10% en volume,
teneur en éthylène ≤ 5% en volume,
teneur en méthane ≤ 5% en volume,
teneur en cyclopropane ≤ 0,1% en volume,
teneur en propylène ≤ 10% en volume,
teneur totale en hydrocarbures en $C_3$ différents du propane et du propylène ≤ 0,3% en volume,
teneur totale en hydrocarbures en $C_5$ ≤ 0,3% en volume et
teneur totale en hydrocarbures en $C_6$ à $C_8$ ≤ 600 ppm en volume.

**56.** Procédé selon l'une quelconque des revendications 33 à 54, **caractérisé en ce que** la conversion du propane dans la première étape est ≥ 5% en mole, jusqu'à ≤ 30% en mole.

**57.** Procédé selon l'une quelconque des revendications 33 à 56, **caractérisé en ce qu'**on sépare, à partir du mélange gazeux de produits de l'oxydation partielle en phase gazeuse et/ou de l'ammoxydation partielle en phase gazeuse, du propylène dudit au moins un produit d'oxydation partielle et/ou d'ammoxydation partielle et on recycle au moins le propane non transformé contenu dans ce mélange gazeux de produits dans la première étape et/ou dans l'oxydation partielle en phase gazeuse et/ou dans l'ammoxydation partielle en phase gazeuse.

**58.** Procédé selon la revendication 33, **caractérisé en ce que** la première étape est une déshydrogénation catalysée par voie hétérogène.

**59.** Procédé selon la revendication 33 ou 58, **caractérisé en ce qu'**une quantité partielle, qui contient au moins un hydrocarbure en $C_4$, est séparée à partir du mélange gazeux 1 des constituants différents du propane et du propylène, qui y sont contenus.

**60.** Procédé selon l'une quelconque des revendications 33, 58 ou 59, **caractérisé en ce qu'**une quantité partielle, qui contient au moins un butène, est séparée à partir du mélange gazeux 1 des constituants différents du propane et du propylène, qui y sont contenus.

**61.** Procédé selon l'une quelconque des revendications 33 ou 58 à 60, **caractérisé en ce qu'**une quantité partielle, qui contient au moins du butène-1, est séparée à partir du mélange gazeux 1 des constituants différents du propane et du propylène, qui y sont contenus.

**62.** Procédé selon l'une quelconque des revendications 33 ou 58 à 61, **caractérisé en ce qu'**un catalyseur, dont la masse active contient les éléments Mo, Bi et Fe, est utilisé conjointement dans l'oxydation partielle en phase gazeuse et/ou dans l'ammoxydation partielle en phase gazeuse, catalysée(s) par voie hétérogène.

**63.** Procédé selon l'une quelconque des revendications 33 ou 58 à 62, **caractérisé en ce qu'**un catalyseur, dont la masse active est un oxyde multimétallique de formule générale IV, est utilisé conjointement dans l'oxydation partielle en phase gazeuse et/ou dans l'ammoxydation partielle en phase gazeuse, catalysée(s) par voie hétérogène:

$$Mo_{12}Bi_aFe_bX^1_cX^2_dX^3_eX^4_fO_n \quad (IV),$$

dans laquelle les variables ont la signification suivante :

$X^1$ = nickel et/ou cobalt,
$X^2$ = thallium, un métal alcalin et/ou un métal alcalino-terreux,
$X^3$ = zinc, phosphore, arsenic, bore, antimoine, étain, cérium, plomb et/ou tungstène,
$X^4$ = silicium, aluminium, titane et/ou zirconium,
a = 0 à 5,
b = 0, 01 à 5,
c = 0 à 10,
d = 0 à 2,
e = 0 à 8,
f = 0 à 10 et
n = un nombre qui est déterminé par la valence et la fréquence des éléments différents de l'oxygène dans (IV).

**64.** Procédé selon l'une quelconque des revendications 33 ou 58 à 63, **caractérisé en ce qu'**un catalyseur, dont la masse active est un oxyde multimétallique de formule générale VII, est utilisé conjointement dans l'oxydation partielle en phase gazeuse, catalysée par voie hétérogène :

$$Mo_{12}V_aX^1_bX^2_cX^3_dX^4_eX^5_fX^6_gO_n \quad (VII),$$

dans laquelle les variables ont la signification suivante :

$X^1$ = W, Nb, Ta, Cr et/ou Ce,
$X^2$ = Cu, Ni, Co, Fe, Mn et/ou Zn,
$X^3$ = Sb et/ou Bi,
$X^4$ = un ou plusieurs métaux alcalins,
$X^5$ = un ou plusieurs métaux alcalino-terreux,

$X^6$ = Si, Al, Ti et/ou Zr,
a = 1 à 6
b = 0,2 à 4,
c = 0,5 à 18,
d = 0 à 40,
e = 0 à 2,
f = 0 à 4,
g = 0 à 40 et
n = un nombre qui est déterminé par la valence et la fréquence des éléments différents de l'oxygène dans VII.

**65.** Procédé selon l'une quelconque des revendications 33 à 64, **caractérisé en ce que** ledit au moins un produit d'oxydation et/ou d'ammoxydation partielle(s) du propylène est au moins un composé du groupe comprenant l'oxyde de propylène, l'acroléine, l'acide acrylique et l'acrylonitrile.

**66.** Procédé selon l'une quelconque des revendications 33 à 65, **caractérisé en ce que**, dans une première étape, on soumet du propane brut à une déshydrogénation catalysée par voie hétérogène en présence d'oxygène et/ou en excluant l'oxygène et on soumet le mélange gazeux 1 à une ammoxydation en phase gazeuse catalysée par voie hétérogène du propylène contenu dans le mélange gazeux 1.

**67.** Procédé selon l'une quelconque des revendications 33 à 66, **caractérisé en ce que**, dans une première étape, on soumet du propane brut à une déshydrogénation catalysée par voie hétérogène en présence d'oxygène et/ou en excluant l'oxygène ainsi qu'en présence de vapeur d'eau et on sépare partiellement ou complètement, à partir du mélange gazeux 1 formé dans la première étape, la vapeur d'eau par condensation et on soumet le mélange gazeux 1' ainsi obtenu à une oxydation partielle en phase gazeuse et/ou à une ammoxydation partielle en phase gazeuse, catalysée(s) par voie hétérogène, du propylène contenu dans le mélange gazeux 1'.

**68.** Procédé selon l'une quelconque des revendications 33 à 67, **caractérisé en ce qu'**on soumet, dans une première étape, du propane brut à une déshydrogénation autothermique catalysée par voie hétérogène.

**69.** Procédé selon l'une quelconque des revendications 33 à 68, **caractérisé en ce qu'**on soumet le mélange gazeux 1 et/ou le mélange gazeux 1', comme constituant d'un mélange gazeux 2, à une ammoxydation partielle en phase gazeuse, catalysée par voie hétérogène, du propylène contenu dans le mélange gazeux 1 et/ou dans le mélange gazeux 1'.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 3798283 A
- DE 2058054 A
- DE 19530494 A
- EP 731077 A
- WO 0196270 A
- DE 2351151 A
- EP 372972 A
- DE 1924431 A
- EP 293224 A
- DE AS2251364 B
- DE 10131297 A
- DE 3521458 A
- EP 938463 B
- EP 117146 A
- EP 731077 B
- EP 1192987 A
- DE 10122027 A
- EP 608838 A
- EP 529853 A
- DE 10051419 A
- DE 10119933 A
- WO 0196271 A
- DE 10211275 A
- EP 938463 A
- DE 19835247 A
- EP 895809 A
- JP 7232071 A
- JP 11169716 A
- JP 10057813 A
- JP 2000037623 A
- JP 10036311 A
- WO 0029105 A
- EP 767164 A
- DE 10029338 A
- JP 8057319 A
- JP 10028862 A
- JP 11043314 A
- JP 11574719 A
- WO 0029106 A
- JP 10330343 A
- JP 11285637 A
- JP 310539 A
- JP 11042434 A
- JP 11343261 A
- JP 3423262 A
- WO 9903825 A
- JP 7053448 A
- JP 2000051693 A
- JP 11263745 A
- DE 10046672 A
- DE 10118814 A
- JP 2000143244 A
- EP 318295 A
- EP 603836 A
- DE 19832033 A
- DE 19836359 A
- EP 962253 A
- DE 10033121 A
- DE 10145958 A
- EP 1193240 A
- EP 700714 A
- EP 700893 A
- EP 929853 A
- DE 10053086 A
- DE 19627847 A
- DE 19740253 A
- DE 19740252 A
- DE 19606877 A
- DE 10059122 A
- JP 3170445 A
- DE 3313573 A
- GB 2118939 A
- US 3161670 A
- DE 19837520 A
- DE 19837517 A
- DE 19837519 A
- DE 19837518 A
- DE 19948241 A
- DE 19927624 A
- DE 19910508 A
- DE 19910506 A
- DE 19948248 A
- CN 1105352 A
- DE 19622331 A
- US 4788371 A
- CN 1073893 A
- WO 9736849 A
- DE 19753817 A
- US 3862256 A
- US 3887631 A
- DE 19530454 A
- US 4341664 A
- US 5086032 A
- US 4255284 A

- EP 167109 A
- DE 19838312 A
- EP 0700893 A
- EP 0700714 A
- WO 9946039 A
- EP 0705136 A
- WO 9929420 A
- US 4220091 A
- US 5430220 A
- US 5877369 A
- EP 0117146 A
- DE 19937196 A
- DE 19937105 A
- DE 19937107 A
- US 4902849 A
- US 4996387 A
- US 5389342 A
- US 5073662 A
- US 4886928 A
- US 5430209 A
- US 5530171 A
- US 5527979 A
- US 5563314 A
- US 55530171 A
- EP 832056 A
- DE 4308087 A
- EP 253409 A
- DE 4431957 A
- DE 4431949 A
- DE 19955176 A
- DE 19948523 A
- DE 10101695 A
- DE 19955168 A
- DE 10046957 A
- DE 10063162 A
- DE 3338380 C
- DE 19902562 A
- EP 15565 A
- DE 2380765 C
- EP 807465 A
- EP 279374 A
- DE 3300044 A
- EP 575897 A
- US 4438217 A
- DE 19855913 A
- WO 9824746 A
- DE 19746210 A
- JP 3294239 A
- DE 4023239 A
- DE 2909671 A
- EP 293859 A
- EP 714700 A
- DE 10046928 A
- DE 19815281 A
- DE 4335973 A
- EP 668104 A
- DE 19736105 A
- DE 19740493 A
- DE 19528646 A
- DE 19910508
- DE 10121592 A
- EP 911313 A
- EP 979813 A
- EP 990636 A
- DE 2830765 A
- DE 19929487 A
- DE 19952964 A
- DE 4335172 A
- DE 4436243 A
- DE 19924532 A
- DE 19924533 A
- EP 982287 A
- EP 982289 A
- DE 10115277 A
- EP 982288 A

**In der Beschreibung aufgeführte Nicht-Patentliteratur**

- **RESASCO, DANIEL E. ; HALLER, GARY L.** Catalysis. University Oklahoma, 1994, vol. 11, 379-411
- *Applied Catalysis,* 1991, vol. 70 (2), 175-187
- *Catalysis Today,* 1992, vol. 13, 673-678
- *Catalysis Letters,* 1994, vol. 23, 103-106
- **W. ZHANG.** *Gaodeng Xuexiao Huaxue Xuebao,* 1993, vol. 14, 566
- **Z. HUANG.** *Shiyou Huagong,* 1992, vol. 21, 592
- *J. of Catalysis,* 1997, vol. 167, 560-569
- *J. of Catalysis,* 1997, vol. 167, 550-559
- *Topics in Catalysis,* 1996, vol. 3, 265-275
- *Catalysis Letters,* 1991, vol. 10, 181-192
- *Ind. Eng. Chem. Res.,* 1996, vol. 35, 14-18
- *Applied Catalysis A: General,* 1993, vol. 100, 111-130
- *J. of Catalysis,* 1994, vol. 148, 56-67
- New Developments in Selective Oxidation. Elsevier Science B.V, 1994, vol. II, 305-313
- 3rd World Congress on Oxidation Catalysis. Elsevier Science B.V, 1997, 375ff